(19) Europäisches Patentamt / European Patent Office / Office européen des brevets

(11) EP 2 464 973 B1

(12) **FASCICULE DE BREVET EUROPEEN**

(45) Date de publication et mention de la délivrance du brevet:
**05.10.2016 Bulletin 2016/40**

(21) Numéro de dépôt: **10761046.1**

(22) Date de dépôt: **12.08.2010**

(51) Int Cl.:
***G01N 33/74*** *(2006.01)* ***G01N 33/542*** *(2006.01)*

(86) Numéro de dépôt international:
**PCT/FR2010/051700**

(87) Numéro de publication internationale:
**WO 2011/018586 (17.02.2011 Gazette 2011/07)**

(54) **METHODE DE DETERMINATION DE LA LIAISON D'UN COMPOSE DONNE A UN RECEPTEUR MEMBRANAIRE.**

VERFAHREN ZUR VORBESTIMMUNG DER BINDUNG EINER BESTIMMTEN VERBINDUNG AN EINEN MEMBRANREZEPTOR

METHOD FOR PREDETERMINING THE BINDING OF A GIVEN COMPOUND TO A MEMBRANE RECEPTOR

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **13.08.2009 FR 0955667**

(43) Date de publication de la demande:
**20.06.2012 Bulletin 2012/25**

(73) Titulaires:
- **CISBIO BIOASSAYS**
  **30200 Codolet (FR)**
- **Centre National de la Recherche Scientifique**
  **75016 Paris (FR)**

(72) Inventeurs:
- **TRINQUET, Eric**
  **F-30130 Pont-saint-esprit (FR)**
- **ZWIER, Jurriaan**
  **F-30650 Rochefort Du Gard (FR)**
- **MATHIS, Gérard**
  **F-30200 Bagnols Sur Ceze (FR)**
- **PIN, Jean-Philippe**
  **F-34000 Montpellier (FR)**
- **DURROUX, Thierry**
  **F-34980 Saint Gely-du-fesc (FR)**

(74) Mandataire: **Nevant, Marc**
**Cabinet Beau de Loménie**
**158, rue de l'Université**
**75340 Paris Cedex 07 (FR)**

(56) Documents cités:
**WO-A1-2007/116069 WO-A2-2008/001361**
**FR-A1- 2 890 174**

- **MAUREL D ET AL: "Cell surface detection of membrane protein interaction with homogeneous time-resolved fluorescence resonance energy transfer technology", ANALYTICAL BIOCHEMISTRY, ACADEMIC PRESS INC, NEW YORK, vol. 329, no. 2, 15 juin 2004 (2004-06-15) , pages 253-262, XP004509821, ISSN: 0003-2697**
- **MAUREL D ET AL: "Cell-surface protein-protein interaction analysis with time-resolved FRET and snap-tag technologies: Application to GPCR oligomerization", NATURE METHODS, NATURE PUBLISHING GROUP, GB, vol. 5, no. 6, 1 juin 2008 (2008-06-01), pages 561-567, XP002556321, ISSN: 1548-7091 [extrait le 2008-05-18]**
- **MILLIGAN G: "Applications of bioluminescence- and fluorescence resonance energy transfer to drug discovery at G protein-coupled receptors", EUROPEAN JOURNAL OF PHARMACEUTICAL SCIENCES, ELSEVIER, AMSTERDAM, NL, vol. 21, no. 4, 1 mars 2004 (2004-03-01), pages 397-405, XP002350686, ISSN: 0928-0987, DOI: DOI:10.1016/J.EJPS.2003.11.010**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 464 973 B1

- ILIEN B ET AL: "Fluorescence resonance energy transfer to probe human M1 muscarinic receptor structure and drug binding properties", JOURNAL OF NEUROCHEMISTRY, WILEY INTERSCIENCE, NEW YORK, NY, US, vol. 85, no. 3, 1 mai 2003 (2003-05-01), pages 768-778, XP002265260, ISSN: 0022-3042

**Description**

ETAT DE LA TECHNIQUE

L'invention relève du domaine du criblage de composés capables de se lier à des récepteurs membranaires.

La liaison de ligands extracellulaires à leurs récepteurs membranaires constitue la première étape de la transduction de signaux biochimiques de l'extérieur vers l'intérieur des cellules vivantes et est donc un élément essentiel dans la régulation de ces cellules. Les récepteurs membranaires constituent donc une cible de choix pour le criblage de nouvelles molécules capables de réguler les processus biologiques, en particulier pour le criblage de nouveaux médicaments.

Il est établi que les récepteurs membranaires peuvent fonctionner non seulement seuls, mais également sous forme d'homodimères ou d'hétérodimères (S. R. George et al, Nature drug discovery 1, 808-820 (2002) et G. Milligan, Biochimica et Biophysica Acta 1768 , 825-835). Certains récepteurs membranaires ne sont fonctionnels que sous forme de dimère, c'est le cas par exemple du récepteur de l'acide gamma aminobutyrique (GABAB), qui est constitué d'une sous unité GABAB1 et GABAB2. La plupart des récepteurs métabotropiques au glutamate fonctionnent sous forme d'homodimères.

De nombreuses méthodes existent pour déterminer si un composé donné se lie ou non à un récepteur membranaire. La méthode la plus classique consiste à marquer le composé testé par un atome radioactif (tritium, Iode 125), à le mettre en contact avec les cellules exprimant le récepteur d'intérêt, et à compter la radioactivité fixée à la cellule après lavage. Il est également courant de marquer un ligand connu d'un récepteur, et de mesurer la capacité d'un composé à tester à rentrer en compétition avec ce ligand marqué pour le site de liaison au récepteur. Ces méthodes présentent plusieurs inconvénients : elles sont basées sur l'utilisation de composés radioactifs ce qui présente des problème de sécurité et de traitement des déchets, et elles peuvent difficilement être mises en oeuvre sur des plateformes à haut débit, c'est-à-dire lorsque l'on souhaite rapidement tester des milliers de composés. Elles ne permettent pas non plus d'obtenir d'information quant au récepteur auquel se lient les composés testés, en particulier il est impossible de déterminer si le composé testé se lie à un type de récepteur ou à plusieurs, à un récepteur monomérique ou à un récepteur dimérique.

D'autres méthodes ont été décrites, notamment des méthodes basées sur l'utilisation de composés fluorescents.

La spectroscopie de corrélation de fluorescence (« FCS ») permet par exemple de mesurer les fluctuations de fluorescence des molécules en diffusion dans un milieu exposé à un laser confocal. Le temps de diffusion de ces molécules étant dépendant du coefficient de diffusion, lui-même corrélé à la taille de ces molécules, les données recueillies par FCS permettent de différencier les molécules à diffusion rapide de celles à diffusion lente, et par conséquent les ligands liés à leur récepteur de ceux qui ne le sont pas. Cette technique a été appliquée par exemple à l'étude des récepteurs de la galanine, en utilisant de la galanine marquée par de la rhodamine (Pramanik, A. et al., 2001. Fluorescence correlation spectroscopy detects galanin receptor diversity on insulinoma cells. Biochemistry, 40(36), 10839-10845).

Handl, H.L. et al., (2004. Lanthanide-based time-resolved fluorescence of in cyto ligand-receptor interactions. Analytical Biochemistry, 330(2), 242-250) ont décrit une alternative aux études de liaison par compétition avec des ligands radioactifs, basée sur la technique DELFIA (acronyme de l'expression anglaise « Diffusion enhanced lanthanide fluoroimmunoassay »). Selon cette technique, un ligand marqué par un chélate de lanthanide faiblement fluorescent est mis en contact avec une cellule exprimant les récepteurs étudiés. Après une étape de lavage destinée à éliminer le ligand non-lié, une solution destinée à amplifier la fluorescence du lanthanide est ajoutée au milieu de mesure. Cette approche a été utilisée par les auteurs dans des expériences de liaison d'un dérivé de l'$\alpha$-MSH conjuguée à un chélate d'Europium, avec le récepteur de la mélanocortine MC4.

La polarisation de fluorescence (FP) est une autre technique qui a été utilisée comme alternative à l'utilisation de ligands radiomarqués : Gagne, A et al par exemple ont décrit la préparation de ligands de récepteurs couplés aux protéines G (GPCR) marqués par un fluorophore connu sous le nom commercial de Bodypy™. En particulier, des ligands des récepteurs de l'hormone de mélano-concentration, de la bradykine et de la mélanocortine ont été marqués par ce fluorophore et leur liaison pour ces récepteurs a été étudiée en mesurant les variations de polarisation de la fluorescence émise par le milieu de mesure (2002. Use of fluorescence polarization détection for the measurement of fluopeptide binding to G protein-coupled receptors. Journal of Receptor and Signal Transduction Research, 22(1-4), 333-343), Ilien, B. et al., (2003. Fluorescence resonnance energy transfer to probe human M1 muscarinic receptor structure and drug binding properties. Journal of Neurochemistry, 85, 768-778) ont

une méthode visant à détecter des composés entrant en compétition pour la liaison d'un ligand du récepteur muscarinique M1 marqué par un fluorophore accepteur, avec ce récepteur, lui-même marqué par un fluorophore donneur.

L'invention se propose de fournir une nouvelle méthode de criblage de composés capables de se lier à des récepteurs membranaires, en particulier pour déterminer si un composé se lie préférentiellement à des dimères de récepteurs, notamment des hétérodimères de GPCR.

## RESUME DE L'INVENTION

L'invention concerne une méthode permettant de déterminer si un composé à tester se lie préférentiellement à un récepteur membranaire R1 ou à un récepteur membranaire R2, ces récepteurs étant connus pour être exprimés à la surface des cellules sous forme monomérique ou homodimérique, c'est-à-dire qu'ils sont présents à la surface des cellules sous forme de monomères R1 ou R2, ou encore sous forme d'homodimères R1R1 ou R2R2. Cette méthode permet également de déterminer si un composé à tester se lie indifféremment à ces récepteurs quel que soit leur état de dimérisation.

Cette méthode peut également être mise en oeuvre avec des récepteurs formant des oligomères, en particulier avec des récepteurs formant des trimères, des tétramères ou des pentamères.

La méthode selon l'invention permet également de déterminer si un composé à tester se lie aux hétérodimères R1R2, R1 et R2 représentant des récepteurs connus pour être exprimés à la surface des cellules sous forme monomérique, homodimérique ou hétérodimérique, c'est-à-dire qu'ils sont présents à la surface des cellules sous forme de monomères R1 ou R2, sous forme d'homodimères R1R1 ou R2R2, ou encore sous forme d'hétérodimères R1R2.

Dans cet aspect de l'invention, il est possible non seulement de déterminer si le composé à tester se lie aux hétérodimères R1R2, mais également de préciser si le composé à tester se lie également aux monomères R2 et aux homodimères R2R2, ou encore s'il se lie spécifiquement aux monomères R2 ou aux homodimères R2R2 et pas aux hétérodimères R1R2.

La méthode selon l'invention est basée sur l'utilisation de couples de partenaires de FRET dont l'un des membres est conjugué à un ligand de l'un ou des deux récepteurs étudiés, et l'autre est conjugué à un des récepteurs étudiés. Cette méthode met en oeuvre un ou deux couples de partenaires de FRET.

## BREVE DESCRIPTION DES FIGURES

Les Figures 1 et 2 représentent un aspect de l'invention où les récepteurs R1 et R2 sont exprimés à la surface des cellules sous forme monomérique ou homodimérique.

Les Figures 3 et 4 représentent un aspect de l'invention où les récepteurs R1 et R2 sont exprimés à la surface des cellules sous forme monomérique, homodimérique ou hétérodimérique.

La figure 5 représente le plasmide FLAG-ST-V2R.

La figure 6 représente le plasmide FLAG-SNAP-delta Opioïde

La figure 7 représente le plasmide FLAG-Halo-Dopamine D2

La figure 8 représente la détermination de la constante de dissociation de l'antagoniste rouge du récepteur des opioïdes sur le récepteur Delta Opioïde de l'hétérodimère Delta Opioïde / Dopamine D2. Le récepteur dopamine D2 est marqué par du HALO-Lumi4Tb permettant ainsi la visualisation de la fixation du ligand fluorescent sur l'hétérodimère.

La figure 9 représente la détermination de la constante de dissociation de l'antagoniste rouge du récepteur de la dopamine D2 sur le récepteur Dopamine D2 de l'hétérodimère Delta Opioïde / Dopamine D2. Le récepteur Delta Opioïde est marqué par du SNAP-Lumi4Tb permettant ainsi la visualisation de la fixation du ligand fluorescent sur l'hétérodimère

La figure 10 représente le criblage de composés spécifiques du récepteur Delta Opioïde impliqué dans l'hétérodimère avec Dopamine D2. Le récepteur Dopamine D2 est marqué à l'HALO-Lumi4Tb permettant ainsi la visualisation de la compétition spécifiquement sur les hétérodimères.

La figure 11 représente le criblage de composés spécifiques du récepteur Dopamine D2 impliqué dans l'hétérodimère avec Delta Opioïde. Le récepteur Delta Opioïde est marqué au SNAP-Lumi4Tb permettant ainsi la visualisation de la compétition sur les récepteurs Dopamine D2 des hétérodimères.

## DESCRIPTION DETAILLEE

Selon un premier aspect, la méthode de l'invention est mise en oeuvre sur des récepteurs membranaires R1 et R2 exprimés dans les membranes cellulaires, ces récepteurs étant connus pour être présents sous forme monomérique, homodimérique, mais pas hétérodimérique, et permet de déterminer si un composé à tester se lie préférentiellement à l'un ou l'autre de ces récepteurs quel que soit leur état de dimérisation, ou encore de déterminer si ce composé se lie indifféremment à ces récepteurs quel que soit leur état de dimérisation.

Selon un mode de réalisation de ce premier aspect (représenté à la Fig. 1), la méthode selon l'invention est une méthode de détermination de la liaison d'un composé à tester avec des récepteurs R1 et R2 exprimés dans les membranes cellulaires, ces récepteurs étant connus pour être présents sous forme homodimérique mais pas hétérodimérique, méthode qui comprend les étapes suivantes :

(a) Marquage du récepteur R1 de manière covalente ou non-covalente par un premier composé donneur d'énergie D1 et du récepteur R2 de manière covalente ou non-covalente par un second composé donneur d'énergie D2,
(b) Ajout au milieu de mesure d'un ligand connu capable de se lier aux récepteurs R1, R2 et aux homodimères R1R1 et R2R2, ce ligand étant marqué par un composé accepteur d'énergie A, et les couples (D1,A) et (D2,A) formant chacun des couples de partenaires de FRET,
(c) Mesure de la luminescence totale $L_{tot}$ émise à la longueur d'onde d'émission du composé accepteur A,
(d) Ajout d'un composé extincteur du signal de FRET du couple (D1,A),
(e) Mesure de la luminescence $L_2$ émise à la longueur d'onde d'émission du composé accepteur A,
(f) Détermination de la luminescence $L_1$ due au couple (D1,A) par la formule $L_1=L_{tot}-L_2$

la séquence des étapes (c) à (f) étant mise en oeuvre en présence et en l'absence du composé à tester.

Selon ce mode de réalisation :

une disparition de la luminescence L1 et un maintien de la luminescence L2 en présence du composé à tester sont représentatifs de la liaison du composé testé au récepteur R1 et à l'homodimère R1R1,
une disparition de la luminescence L2 et un maintien de la luminescence L1 en présence du composé à tester sont représentatifs de la liaison du composé testé au récepteur R2 et à l'homodimère R2R2,
une disparition de la luminescence L1 et une disparition de la luminescence L2 en présence du composé à tester sont représentatives d'un composé se liant à la fois aux récepteurs R1, R2, et aux homodimères R1R1 ou R2R2.

Les composés extincteurs de FRET nécessaires à la mise en oeuvre de cette méthode sont décrits dans la demande internationale de brevet WO 2007/116069. De manière préférée, le composé donneur D1 est un complexe de lanthanide, en particulier un cryptate ou un chélate de lanthanide (tel qu'un cryptate ou un chélate de terbium ou d'europium) et le composé extincteur de FRET du couple (D1,A) est un composé comportant un domaine de liaison avec D1, en particulier un anticorps spécifique du complexe de lanthanide D1.

Selon un autre mode de réalisation du premier aspect (représenté à la Fig. 2), la méthode selon l'invention peut être mise en oeuvre en marquant les récepteurs R1 et R2 par des composés accepteurs de FRET A1 et A2 et le ligand avec un donneur D1, ce qui correspond au format inverse de celui présenté dans le mode de réalisation précédent. Ainsi, selon ce mode de réalisation, les deux couples de partenaires de FRET utilisés sont (D,A1) et (D, A2).

Ce second mode de réalisation concerne donc une méthode de détermination de la liaison d'un composé à tester avec des récepteurs membranaires R1 et R2 exprimés dans les membranes cellulaires, ces récepteurs étant connus pour être présents sous forme homodimérique mais pas hétérodimérique, méthode qui comprend les étapes suivantes :

(a) Marquage du récepteur R1 de manière covalente ou non-covalente par un premier composé accepteur d'énergie $A_1$ et du récepteur R2 de manière covalente ou non-covalente par un second composé accepteur d'énergie A2, A1 et A2 ayant des longueurs d'onde d'émission différentes,
(b) Ajout au milieu de mesure d'un ligand connu capable de se lier aux récepteurs R1, R2 et aux homodimères R1R1 et R2R2, ce ligand étant marqué par un composé donneur d'énergie D, et les couples (D,A1) et (D,A2) formant chacun des couples de partenaires de FRET,
(c) Mesure de la luminescence $L_1$ émise à la longueur d'onde d'émission du composé accepteur A1 d'une part, et de la luminescence $L_2$ émise à la longueur d'onde d'émission du composé accepteur A2 d'autre part, chacune en présence et en l'absence du composé à tester.

Selon ce second mode de réalisation :

une disparition de la luminescence L1 et un maintien de la luminescence L2 en présence du composé à tester sont représentatifs de la liaison du composé testé au récepteur R1 et à l'homodimère R1R1,
une disparition de la luminescence L2 et un maintien de la luminescence L1 en présence du composé à tester sont représentatifs de la liaison du composé testé au récepteur R2 et à l'homodimère R2R2,
une disparition de la luminescence L1 et une disparition de la luminescence L2 en présence du composé à tester sont représentatives d'un composé se liant à la fois aux récepteurs R1, R2, et aux homodimères R1R1 ou R2R2.

Selon un second aspect, la méthode de l'invention est mise en oeuvre sur des récepteurs membranaires R1 et R2 exprimés dans les membranes cellulaires, ces récepteurs étant connus pour être présents sous forme hétérodimérique, et éventuellement monomérique et /ou homodimérique, et permet de déterminer (i) si un composé à tester se

lie aux hétérodimères R1R2, et (ii) si un composé à tester se lie spécifiquement aux hétérodimères R1R2, ou bien s'il se lie spécifiquement au récepteur R2 et à l'homodimère R2R2, ou encore s'il se lie à la fois à au récepteur R2, a l'homodimère R2R2 et à l'hétérodimère R1R2.

Selon un mode de réalisation de ce second aspect (représenté à la Fig. 3), la méthode selon l'invention est une méthode de détermination de la liaison d'un composé à tester avec des récepteurs membranaires R1 et R2 exprimés dans les membranes cellulaires, ces récepteurs étant connus pour être présents sous forme hétérodimérique (éventuellement également sous forme monomérique ou homodimérique), méthode qui comprend les étapes suivantes :

(a) Marquage du récepteur R1 de manière covalente ou non-covalente par le premier membre d'un couple de partenaires de FRET (D,A1), dans lequel D est le composé donneur et A1 est le composé accepteur d'énergie,
(b) Ajout au milieu de mesure d'un ligand connu de R2 capable de se lier à l'hétérodimère R1R2 mais ni au récepteur R1 ni à l'homodimère R1R1, ledit ligand étant marqué par le second membre dudit couple de partenaires de FRET,
(c) Mesure de la luminescence $L_1$ émise à la longueur d'onde d'émission du composé accepteur A1, en présence et en l'absence du composé à tester.

Selon ce mode de réalisation, une disparition de la luminescence L1 en présence du composé à tester est représentative de la liaison de ce composé à l'hétérodimère R1R2.

Le premier membre du couple de partenaires de FRET, qui marque le récepteur R1, peut être un composé donneur d'énergie D et le second membre, qui est lié au ligand, peut être un composé accepteur A1. Le format inverse est également utilisable, à savoir, le premier membre du couple de partenaire de FRET, qui marque R1, peut être un composé accepteur d'énergie A1 et le second membre de ce couple, lié au ligand, peut être un composé donneur d'énergie D.

Selon un autre mode de réalisation du second aspect (représenté à la Fig. 4), le ligand est conjugué avec un composé donneur D, le récepteur R1 est marqué par un composé accepteur A1, et le récepteur R2 est marqué par un composé accepteur d'énergie A2. En l'absence de composé à tester, on peut mesurer la luminescence L1 ou L2 émise à la longueur d'onde d'émission du composé accepteur A1 ou A2, respectivement. Dans ce mode de réalisation, le signal L1 provient du FRET émis par le couple (D,A1) qui marque l'hétérodimère R1R2, et le signal L2 provient des couples (D,A2) qui marquent le récepteur R2, l'homodimère R2R2, et l'hétérodimère R1R2. En fonction de la spécificité de liaison du composé testé, le signal L2 va donc être maintenu, supprimé ou encore diminué.

Cet autre mode de réalisation permet donc avantageusement de préciser si le composé testé est spécifique de l'hétérodimère R1R2 ou bien s'il se lie également au récepteur R2 ou à l'homodimère R2R2, ou encore s'il est spécifique du récepteur R2 et de l'homodimère R2R2, dans l'hypothèse où ces espèces sont présentes dans le milieu de mesure.

Selon cet autre mode de réalisation du second aspect de l'invention, l'étape (a) comprend également le marquage du récepteur R2 de manière covalente ou non-covalente par un second composé accepteur d'énergie A2, A1 et A2 ayant des longueurs d'onde d'émission différentes et (D,A2) formant un couple de partenaires de FRET, et l'étape (c) comprend également la mesure de la luminescence $L_2$ émise à la longueur d'onde d'émission du composé accepteur A2 , en présence et en l'absence du composé à tester. Selon cet autre mode de réalisation :

une diminution de la luminescence L2 et une disparition de la luminescence L1 en présence du composé à tester sont représentatives de la liaison de ce composé à l'hétérodimère R1R2,
une diminution de la luminescence L2 et un maintien de la luminescence L1 en présence du composé à tester sont représentatifs de la liaison de ce composé avec le récepteur R2 et l'homodimère R2R2 mais pas avec l'hétérodimère R1R2, et
une disparition des signaux L1 et L2 en présence du composé à tester est représentative de la liaison de ce composé à la fois avec le récepteur R2, l'homodimère R2R2 et l'hétérodimère R1R2.

**COUPLE DE PARTENAIRES DE FRET**

Selon l'invention, les récepteurs membranaires R1 et/ou R2 ainsi que le ligand sont marqués par un membre d'un couple de partenaires de FRET, à savoir, un composé fluorescent donneur ou un composé fluorescent accepteur d'énergie. Avantageusement, le marquage des récepteurs R1 et R2 par les membres d'un couple de partenaires de FRET est un marquage direct par liaison covalente.

Le FRET est défini comme un transfert d'énergie non radiatif résultant d'une interaction dipôle-dipôle entre un donneur et un accepteur d'énergie. Ce phénomène physique nécessite une compatibilité énergétique entre ces molécules. Cela signifie que le spectre d'émission du donneur doit recouvrir, au moins partiellement, le spectre d'absorption de l'accepteur. En accord avec la théorie de Förster, le FRET est un processus qui dépend de la distance séparant les deux molécules, donneur et accepteur : lorsque ces molécules sont à proximité l'une de l'autre, un signal de FRET sera

émis.

Les composés fluorescents donneurs et accepteurs peuvent être choisis dans le groupe suivant : les allophycocyanines, en particulier celles connues sous la dénomination commerciale XL665 ; les molécules organiques luminescentes, telles que les rhodamines, les cyanines (comme par exemple Cy5), les squaraines, les coumarines, les proflavines, les acridines, les fluorescéines, les dérivés du boron-dipyrrométhène (commercialisés sous la dénomination « Bodipy »), les fluorophores connus sous la dénomination « Atto », les fluorophores connus sous la dénomination « Dy », les composés connus sous la dénomination « Alexa », le nitrobenzoxadiazole, les complexes métalliques fluorescents, tels que les cryptates de terre rare, les chélates de terre rare (en particulier les chélates et cryptates d'europium, de terbium, de samarium, de dysprosium, de neodymium); les particules inorganiques luminescentes comme les nanocristaux («quantum dots» en langue anglaise). Ces composés fluorescents peuvent être utilisés soit comme composés fluorescents donneurs soit comme composés fluorescents accepteurs dans un système de FRET. Avantageusement, les composés fluorescents accepteurs sont choisis parmi les allophycocyanines, les rhodamines, les cyanines, les squaraines, les coumarines, les proflavines, les acridines, les fluorescéines, les dérivés du boron-dipyrrométhène, le nitrobenzoxadiazole.

Les expressions « les cyanines » et « les rhodamines » doivent être respectivement comprises comme « les dérivés de cyanine » et « les dérivés de rhodamine ». L'homme du métier connaît ces différents fluorophores, disponibles dans le commerce.

Les composés « Alexa » sont commercialisés par la société Invitrogen; les composés « Atto » sont commercialisés par la société Attotec ; les composés « Dy » sont commercialisés par la société Dyomics ; les composés « Cy » sont commercialisés par la société Amersham Biosciences ; les autres composés sont commercialisés par divers fournisseurs de réactifs chimiques, tels que les sociétés Sigma, Aldrich ou Acros.

Les composés fluorescents donneurs d'énergie à durée de vie longue (> 0,1 ms, de préférence comprise entre 0,5 et 6 ms), en particulier les chélates ou cryptates de terres rares sont avantageux puisqu'ils permettent d'effectuer des mesures en temps résolu, c'est-à-dire de mesurer des signaux de TR-FRET (en langue anglaise, « Time Resolved FRET ») en s'affranchissant d'une grande partie du bruit de fond émis par le milieu de mesure. Ils sont pour cette raison et de manière générale préférés pour la mise en oeuvre de la méthode selon l'invention. Avantageusement, ces composés sont des complexes de lanthanides. Ces complexes (tels que chélates ou cryptates) sont particulièrement appropriés en tant que membre du couple de FRET donneur d'énergie.

Les complexes de dysprosium (Dy3+), de samarium (Sm3+), de neodymium (Nd3+), d'ytterbium (Yb3+) ou encore d'erbium (Er3+) sont des complexes de terres rares également appropriés aux fins de l'invention, mais les complexes d'europium (Eu3+) et de terbium (Tb3+) sont particulièrement préférés.

De très nombreux complexes de terres rares ont été décrits et plusieurs sont actuellement commercialisés par les sociétés PerkinElmer, Invitrogen et Cisbio Bioassay.

Des exemples de chélates ou cryptates de terres rares appropriés aux fins de l'invention sont :

- Les cryptates de terres rares, comportant un ou plusieurs motifs pyridine. De tels cryptates de terre rare sont décrits par exemple dans les brevets EP 0 180 492, EP 0 321 353, EP 0 601 113 et dans la demande internationale WO 01/96 877. Les cryptates de terbium (Tb3+) et d'europium (Eu3+) sont particulièrement appropriés aux fins de la présente invention. Des cryptates de terres rares sont commercialisés par la société Cisbio Bioassay. On peut citer à titre d'exemple non limitatif les cryptates d'europium de formules ci-dessous (qui peuvent être couplés au composé à marquer via un groupe réactif, ici par exemple un groupe NHS) :

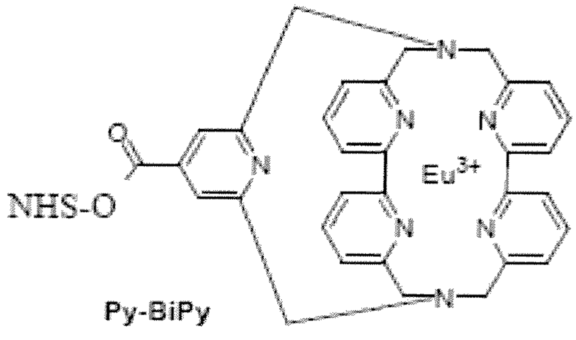

Py-BiPy

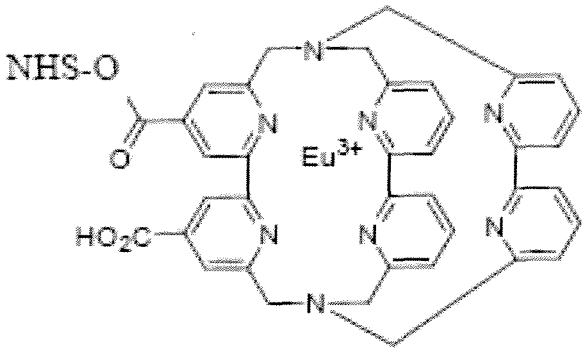

TrisBiPy-Eu

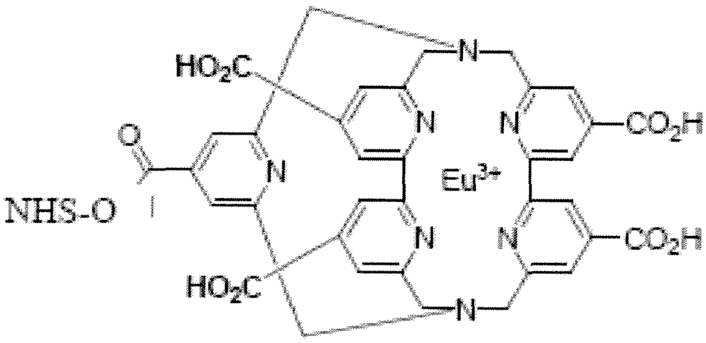

K-Py-BiPy-tetraacide-Eu

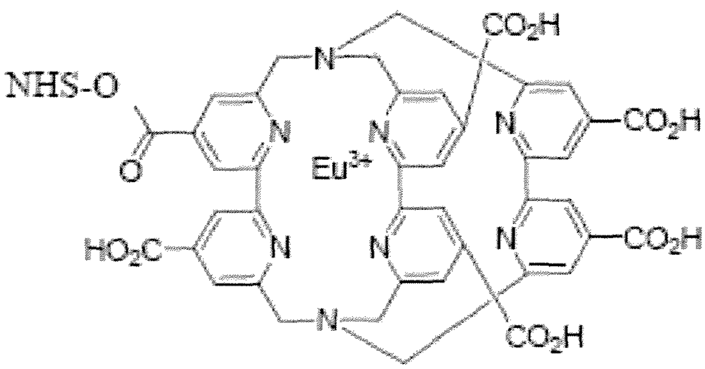

K-TrisBiPy-pentaacide-Eu

Le cryptate d'europium Py-BiPy-tétraacide-Eu est particulièrement adapté à la mise en oeuvre de l'invention en raison de ses propriétés de résistance à l'extinction de fluorescence dans les milieux biologiques.

- Les chélates de terres rares décrits notamment dans les brevets US 4 761 481, US 5 032 677, US 5 055 578, US 5 106 957, US 5 116 989, US 4 761 481, US 4 801 722, US 4 794 191, US 4 637 988, US 4 670 572, US 4 837 169, US 4 859 777. Les brevets EP 0 403 593, US 5 324 825, US 5 202 423, US 5 316 909 décrivent des chélates composés d'un ligand nonadenté tel que la terpyridine. Ces chélates de terres rares sont commercialisés par la société PerkinElmer.

- Des complexes de terres rares constitués d'un agent chélatant tel que le tetraazacyclododécane, substitué par un chromophore comportant des cycles aromatiques, tels que ceux décrits par Poole R. et al. dans Biomol. Chem, 2005, 3, 1013-1024 "Synthesis and characterisation of highly emissive and kinetically stable lanthanide complexes

suitable for usage in celluloll, peuvent également être utilisés. On peut aussi utiliser les complexes décrits dans la demande WO2009/10580.

- Le cryptate de terbium Tb(KR) de formule ci-dessous (qui peut être couplé au composé à marquer via un groupe réactif, ici par exemple un groupe NHS) :

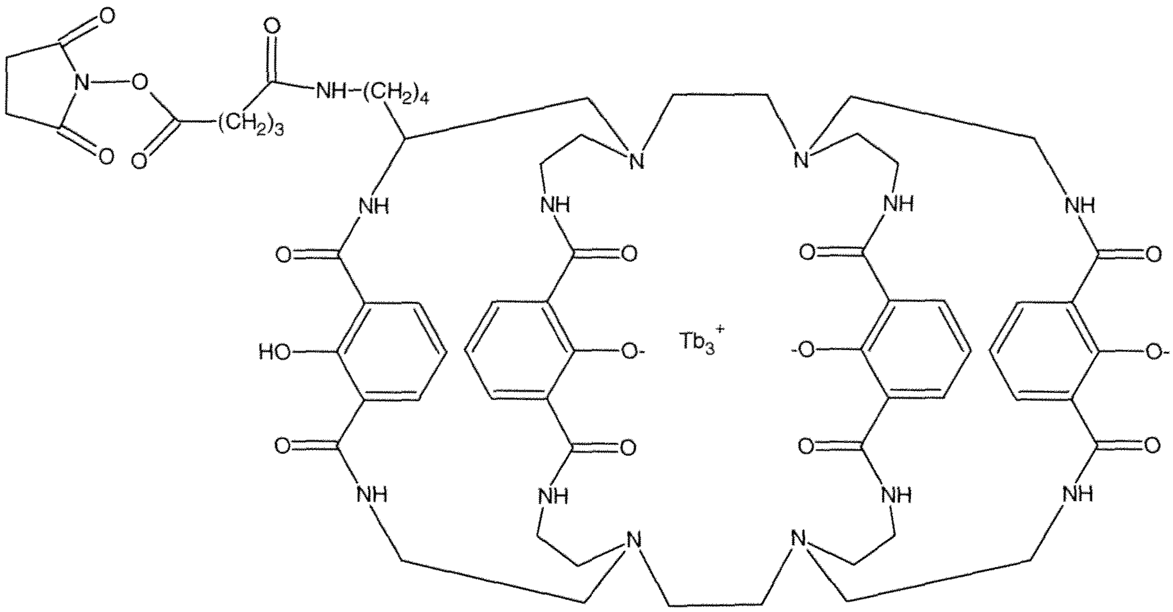

et dont la synthèse est décrite dans la demande internationale WO2008/063721 est l'un des cryptates de terbium les plus appropriés à la mise en oeuvre de l'invention.

- Le cryptate de Terbium Lumi4-Tb de la société Lumiphore, commercialisé par Cisbio Bioassay.

- Le « quantum dye » de la société Research Organics, de formule ci-dessous (qui peut être couplé au composé à marquer via un groupe réactif, ici NCS) :

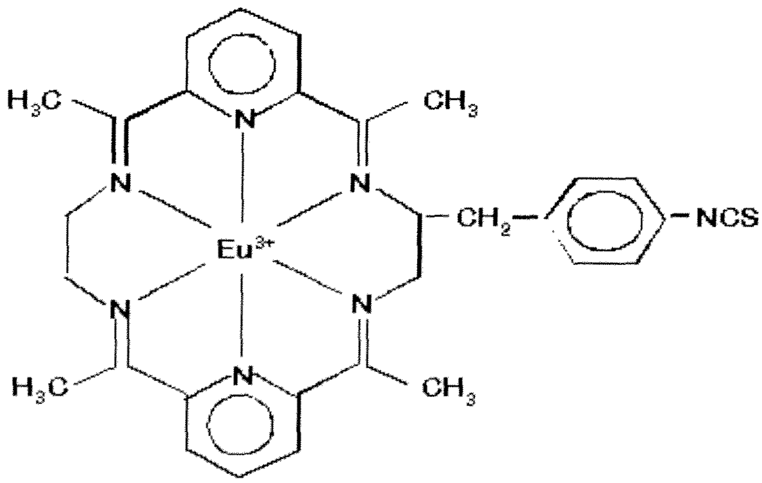

- Le chélate de terbium DTPA-cs124 Tb, commercialisé par la société Invitrogen de formule ci-dessous (qui peut être couplé au composé à marquer via un groupe réactif R) et dont la synthèse est décrite dans le brevet américain US 5 622 821.

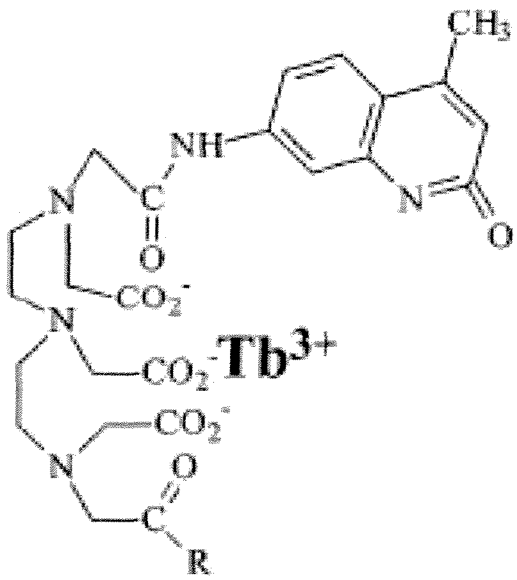

En ce qui concerne les accepteurs, les dérivés de cyanine et de fluorescéine sont préférés.

**MARQUAGE DES PROTEINES R1 ET/OU R2 :**

Selon l'invention, les récepteurs membranaires R1 et/ou R2 sont marqués par un membre d'un couple de partenaires de FRET, à savoir, un composé fluorescent donneur ou un composé fluorescent accepteur d'énergie. Plusieurs techniques connues de l'homme du métier permettent de coupler un récepteur membranaire avec des composés fluorescents :

*(a) Couplage du récepteur membranaire avec un donneur ou un accepter de manière non covalente*

Le donneur ou l'accepteur peut être couplé avec le récepteur membranaire par l'intermédiaire d'un couple de partenaires de liaison dont l'un au moins est de nature protéique. Dans cette approche, le récepteur membranaire est fusionné avec le partenaire de liaison de nature protéique par les techniques classiques de biologie moléculaire (construction d'un vecteur d'expression comprenant une séquence de nucléotides codant pour le récepteur membranaire, fusionnée avec celle codant pour le partenaire de liaison protéique, et introduction du vecteur d'expression dans la cellule).

Le donneur ou l'accepteur est conjugué de manière covalente à l'autre partenaire de liaison, que l'on appelle ici agent de couplage, qui sera ensuite ajouté au milieu extracellulaire. La reconnaissance des partenaires de liaison permet le marquage indirect du récepteur membranaire par le donneur ou l'accepteur.

A titre d'exemple non limitatif de ce type de partenaires de liaison, on peut citer :

- le couple constitué de la séquence cystéine-cystéine-X-X-cystéine-cystéine (SEQ ID N°1) dans laquelle X est un acide aminé quelconque et d'un composé bi-arsenic. Ces composés bi-arsenic peuvent être facilement marqués avec une molécule organique du type fluorescéine ou rhodamine (voir B.A.Griffin et al. (1998) Science. 1998, 281, 269-271 et S.A. Adams et al. (2002) J. Am. Chem. Soc. 2002, 124, 6063-6076 pour des détails sur la technologie).
- Le peptide BTX (bungarotoxine), composé de 13 acides aminés qui est reconnu par la bungarotoxine (BTX), peut être couplé à une molécule fluorescente (voir C. M. McCann et al. (2005), Biotechnique (2005), 38, 945-952).
- Le couple streptavidine (ou avidine) / biotine : la séquence de liaison de la Streptavidine (SBP-Tag) est une séquence formée par 38 acides aminés qui présente une haute affinité pour la biotine pouvant être préalablement marquée avec un donneur ou un accepteur (voir C. M. McCann et al. (2005), Biotechnique (2005), 38, 945-952).
- La séquence de l'enzyme dihydrofolate réductase d'E. coli (eDHFR) qui lie spécifiquement et avec une haute affinité des ligands, tels que la triméthoprime sur lesquels peuvent être greffés le donneur ou l'accepteur selon la technologie dénommée « Ligand link Universal labelling technology » de la Société Active Motif.
- Les couples tags/antitags sont des partenaires de liaison fréquemment utilisés pour marquer des protéines. Le terme « tag » désigne une petite « étiquette » protéique constituée d'un séquences d'acides aminés, généralement mais pas obligatoirement assez courte (moins de 15 acides aminés), qui est fusionnée au récepteur membranaire ou bien est naturellement présente dans cette protéine. Le terme « antitag » désigne un anticorps se liant de manière spécifique audit « tag ». Dans cette mise en oeuvre, l'anticorps « antitag » est lié de manière covalente au donneur

ou à l'accepteur. Lorsque l'anticorps ainsi marqué est ajouté au milieu extracellulaire, il se lie au « tag » conjugué au récepteur membranaire et l'interaction « tag/antitag » permet le marquage indirect de cette protéine par le donneur ou l'accepteur.

A titre d'exemple non limitatif de couples « tags/antitags », on peut citer les couples suivants dont les membres sont disponibles commercialement : GST/anticorps anti-GST dans lequel GST représente la glutathione S-transférase ou un de ses fragments ; 6HIS/anticorps anti-6HIS dans lequel 6HIS est un peptide constitué de 6 histidines ; Myc/anticorps anti-Myc dans lequel Myc est un peptide constitué des acides aminés 410-419 de la protéine Myc humaine ; FLAG/anticorps anti-FLAG dans lequel FLAG est un peptide ayant les 8 acides aminés DYKDDDDK (SEQ ID N°2) ; HA/anticorps anti HA dans lequel HA est un épitope de l'hémaglutinine d'Influenza, constitué des 9 acides aminés YPYDVPFYA (SEQ ID N°3). Il est clair que la nature exacte du tag n'est pas critique pour la mise en oeuvre de l'invention.

*(b) Couplage du récepteur membranaire avec un donneur ou un accepteur de manière covalente*

Dans cette approche, le donneur ou l'accepteur est couplé au récepteur membranaire par une liaison covalente ; plusieurs techniques ont été décrites et les réactifs nécessaires à leurs mises en oeuvre sont disponibles commercialement. Pour ce couplage, on pourra utiliser l'une des techniques ci-après :

- formation d'une liaison covalente au niveau d'un groupe réactif présent sur le récepteur membranaire, en particulier au niveau d'un des groupes suivants : le groupe amino terminal, les groupes carboxylates des acides aspartiques et glutamiques, les groupes amines des lysines, les groupes guanidines des arginines, les groupes thiols des cystéines, les groupes phénols des tyrosines, les cycles indoles des tryptophanes, les groupes thioéthers des méthionines, les groupes imidazoles des histidines.
  Ces groupes présents sur le récepteur membranaire peuvent former une liaison covalente avec un groupe réactif porté par le donneur ou l'accepteur. Les groupes réactifs appropriés sont connus de l'homme du métier : un donneur ou l'accepteur fonctionnalisé par un groupe maléimide sera par exemple capable de se lier de manière covalente avec les groupes thiols portés par les cystéines de la protéine. De même, un donneur/accepteur portant un ester de N-hydroxysuccinimide sera capable de se fixer de manière covalente à une amine du récepteur membranaire.
- Utilisation d'une enzyme suicide
  Par enzyme suicide on entend des protéines qui ont une activité enzymatique modifiée par des mutations spécifiques qui leur confèrent la capacité de lier rapidement et de façon covalente un substrat. Ces enzymes sont dites « suicides » car chacune ne peut lier qu'une seule molécule fluorescente, l'activité de l'enzyme étant bloquée par la fixation du substrat. Ces enzymes constituent par conséquent un outil de choix pour marquer spécifiquement des récepteurs d'intérêt avec un rapport d'une molécule fluorescente pour une protéine. Dans cette approche, une enzyme suicide est fusionnée, par les techniques classiques de biologie moléculaire, avec le récepteur membranaire -de préférence dans sa partie N-terminale- et le substrat de l'enzyme lié de manière covalente à un donneur/accepteur est introduit dans le milieu extracellulaire. La réaction enzymatique a pour conséquence la liaison covalente du substrat marqué à l'enzyme, et donc le marquage du récepteur membranaire par le donneur ou l'accepteur.
  On peut citer à titre d'exemple non limitatif les enzymes suivantes :

  - les mutants de l'O6-alkylguanine DNA alkyltransférase (AGT). Les enzymes SNAP-tag (Juillerat et al, Chemistry & biology, Vol.10, 313-317 Avril 2003) et CLIP-tag (Gautier et al, Chemistry et Biology, 15, 128-136, février 2008) commercialisées par la société NEB sont des mutants de l'AGT humaine dont les substrats sont respectivement, l'$O^6$-benzylguanine (ci-après abrégée BG) et l'$O^2$-benzylcytosine (ci-après abrégée BC). L'enzyme N-AGT (Gronemeyer et al (Protein engineering, design & selection, vol. 19, no 7, pp 309-3016, 2006) est un autre mutant de cette enzyme dont la réactivité avec l'$O^6$-benzylguanine est meilleure que celle de l'enzyme SNAP-tag.
  - les mutant d'une déhalogénase (telle que l'enzyme HaloTag commercialisée par Promega) qui génère également une réaction enzymatique du type suicide (voir WO2004/072232), dont certains des substrats sont des composés de la famille des chloroalcanes, en particulier les chloroalcanes comportant le motif $-NH-CH_2CH_2-O-CH_2CH_2-O-(CH_2)_6-Cl$. Dans ce cas, le donneur/accepteur sera conjugué à ce type de motif.
  - La protéine ACP (« Acyl Carrier Protein »), protéine transporteur d'acyles, sur laquelle est transféré, en présence de phosphopantéthéine transférase, le résidu 4'-phosphopantéthéine du coenzyme A sur une sérine de l'ACP (N.George et al, Journal of the American Chemical society 126 (2004) p 8896-8897). Lorsque cette approche est utilisée pour marquer le récepteur membranaire par le donneur ou l'accepteur, il est nécessaire de rajouter au milieu réactionnel de la phosphopantéthéine transférase. La société NEB commercialise un fragment de l'ACP sous le nom commercial « ACP-Tag » pour le marquage de protéines.

Lorsque cette approche est utilisée pour marquer le récepteur d'intérêt, les cellules sont transfectées avec un plasmide d'expression comportant l'ADN codant pour une protéine de fusion comprenant l'enzyme suicide et le récepteur d'intérêt. Ce plasmide peut également comprendre, en amont de l'ADN codant pour ces protéines, l'ADN codant pour une étiquette telle que par exemple l'épitope FLAG, l'épitope myc, ou encore celui de l'hémaglutinine de l'influenza (HA). Ces étiquettes ne sont pas essentielles mais facilitent la manipulation de la protéine de fusion à des fins de contrôle ou de purification. La transfection est opérée par les techniques classiques, telle que l'électropartition.

Pour s'assurer que la protéine de fusion sera exprimée dans la membrane cellulaire, il peut être utile d'inclure dans le plasmide d'expression, en amont de la séquence codant pour le récepteur d'intérêt et de l'enzyme suicide, celle codant pour un peptide d'adressage membranaire, tels que le peptide signal T8 ou le peptide signal du récepteur mGluR5, dont l'utilisation à cet effet est connue de l'homme du métier. Enfin, il peut également être souhaitable de s'assurer que la séquence codant pour le récepteur d'intérêt ne comporte pas de séquence d'adressage membranaire native qui pourrait faire l'objet d'un clivage post-traductionnel de la liaison entre le récepteur d'intérêt et l'enzyme suicide : si c'est le cas, il est préférable de ne pas introduire ce domaine dans le plasmide d'expression.

Dans le cas ou l'on travaille sur des cellules intactes, et pour que la réaction enzymatique ait lieu avec le substrat de l'enzyme présent dans le milieu extracellulaire (tel qu'un conjugué BG-partenaire de FRET), il est nécessaire que l'enzyme suicide soit exposée au milieu extracellulaire : lorsque la partie N-terminale du récepteur d'intérêt naturelle est exposée au milieu extra-cellulaire, ce qui est le cas pour les GPCR et les RTK, la protéine de fusion sera construite de telle sorte que l'enzyme suicide soit exprimée dans la partie N-terminale de la protéine de fusion, mais toujours en aval du peptide d'adressage membranaire s'il est présent.

Finalement, lorsque une enzyme suicide est utilisée pour marquer le récepteur d'intérêt avec le partenaire de FRET, et que le récepteur d'intérêt est un GPCR ou un RTK, l'invention comprend une étape préliminaire de transfection des cellules par un vecteur d'expression comprenant la séquence d'ADN codant pour une protéine de fusion dont la partie N-terminale comprend une enzyme suicide et la partie C-terminale comprend le récepteur d'intérêt (R1 ou R2).

L'introduction dans le milieu extracellulaire du substrat de l'enzyme conjugué à un partenaire de FRET aura pour conséquence le marquage du récepteur d'intérêt par ce partenaire de FRET.

Avantageusement, chacun des récepteurs R1 et R2 est exprimé sous forme de protéine de fusion avec une enzyme suicide, leurs marquages étant effectués par addition dans le milieu de mesure des membres des couples de partenaires de FRET, l'un et l'autre étant chacun lié de manière covalente au substrat de ladite enzyme suicide. Dans ce cas les enzymes suicides utilisées pour chaque récepteur peuvent être différentes ou identiques.

Dans ce mode de réalisation, les vecteurs d'expression codant pour une protéine de fusion choisie parmi les protéines de fusion suivantes peuvent être utilisés :

enzyme suicide - récepteur d'intérêt, ou
étiquette - enzyme suicide - récepteur d'intérêt, ou
peptide d'adressage membranaire - enzyme suicide - récepteur d'intérêt, ou
peptide d'adressage membranaire - étiquette - enzyme suicide - récepteur d'intérêt.

A titre d'illustration de l'utilisation de cette approche, on peut citer les travaux de Maurel et al qui ont décrit la préparation de plasmides codant pour une protéine de fusion comprenant une enzyme suicide (Snaptag) dans la partie N-terminale du récepteur membranaire (GABAB B1, GABAB B2, mGlu1) et leur transfection dans des cellules (Nature Methods, 2008, Supplementary methods).

## LIGAND MARQUE

La méthode selon l'invention nécessite l'utilisation de ligands des récepteurs membranaires étudiés, marqués par un membre d'un couple de partenaires de FRET, c'est-à-dire un composé donneur ou un composé accepteur d'énergie. Le terme ligand désigne ici une molécule organique, éventuellement protéique, capable de se lier au récepteur et d'en moduler l'activité. Le ligand est de préférence différent d'un anticorps, et de manière encore plus préférée est un agoniste (y compris un agoniste partiel ou un agoniste inverse) ou un antagoniste des récepteurs membranaires étudiés. Il est également possible d'utiliser un ligand qui soit un modulateur allostérique de ces récepteurs.

De très nombreux ligands de GPCR notamment ont été décrits : une base de donnée a été mise à la disposition du public et fournit des informations sur les GPCR et leurs ligands (Okuno, Y. et al., 2008. GLIDA: GPCR ligand database for chemical genomics drug discovery database and tools update. Nucl. Acids Res., 36(suppl_1), D907-912). L'homme du métier a donc accès à un nombre très important de composés se liant à des GPCR et susceptibles d'être utilisés dans la méthode selon l'invention.

Le marquage d'un ligand par un composé donneur ou accepteur fluorescent est effectué par les techniques classiques de conjugaison faisant appel à l'utilisation de groupes réactifs. Les composés donneurs ou accepteurs fluorescents sont généralement commercialisés sous forme « fonctionnalisée », c'est-à-dire qu'ils portent un groupe

réactif susceptible de réagir avec un groupe fonctionnel présent sur le composé à marquer, en l'occurrence, le ligand.

Typiquement, le groupe réactif présent sur le composé fluorescent donneur ou accepteur est un groupe électrophile ou nucléophile qui peut former une liaison covalente lorsqu'il est mis en présence d'un groupe nucléophile ou électrophile approprié, respectivement. A titre d'exemples, les couples de groupes électrophiles/nucléophiles et le type de liaison covalente formée lorsqu'ils sont mis en présence sont listés ci-dessous :

| Groupe électrophile | Groupe nucléophile | Type de liaison |
|---|---|---|
| acrylamides | Thiols | thioéthers |
| halogénures d'acyle | amines/anilines | carboxamides |
| aldéhydes | amines/anilines | imines |
| aldéhydes ou cétones | hydrazines | hydrazones |
| aldéhydes ou cétones | hydroxylamines | oximes |
| alkyl sulfonates | thiols | thioéthers |
| anhydrides | amines/anilines | carboxamides |
| halogénure d'aryle | thiols | thiophénols |
| halogénure d'aryle | amines | aryl amines |
| aziridines | thiols | thioéthers |
| carbodiimides | acides carboxyliques | N-acylurées ou anhydrides |
| esters activés* | amines/anilines | carboxamides |
| haloacétamides | Thiols | thioéthers |
| halotriazines | amines/anilines | aminotriazines |
| imido esters | amines/anilines | amidines |
| isocyanates | amines/anilines | urées |
| isothiocyanates | amines/anilines | thiourées |
| maléimides | thiols | thioéthers |
| sulfonate esters | amines/anilines | alkyl amines |
| halogénures de sulfonyle | amines/anilines | sulfonamides |
| * : par ester activé, on entend des groupes de formule COY, ou Y est : • un groupe partant, choisi parmi les groupes succinimidyloxy ($-OC_4H_4NO_2$), sulfo succinimidyloxy ($-OC_4H_3NO_2-SO_3H$) ; • un groupe aryloxy substitué ou non par au moins un substituant électrophile tel que les groupes nitro, fluoro, chloro, cyano, trifluorométhyle, formant ainsi un aryle ester activé ; • un acide carboxylique activé par un groupe carbodiimide, formant un anhydride -OCORa ou -OCNRaNHRb, dans lesquels Ra et Rb sont identiques ou différents et sont choisis parmi les groupes alkyles en C1-C6, perfluoroalkyles en C1-C6, alkoxy en C1-C6, cyclohexyle ; • le 3-diméthylaminopropyle, ou le N-morpholinoéthyle. | | |

Les composés fluorescents donneurs et accepteurs disponibles dans le commerce comportent généralement une fonction maléimide ou un ester activé, le plus souvent par un groupe NHS (N-hydroxysuccinimidyle), qui réagissent avec les groupes thiol et amines respectivement ; il est donc particulièrement avantageux d'utiliser des ligands contenant une fonction amine ou thiol.

Lorsque le ligand est de nature protéique, il peut être avantageux d'utiliser l'un des groupes fonctionnels naturellement présent dans les protéines: le groupe amino terminal, le groupe carboxylate terminal, les groupes carboxylates des acides aspartiques et glutamiques, les groupes amines des lysines, les groupes guanidines des arginines, les groupes thiols des cystéines, les groupes phénols des tyrosines, les cycles indoles des tryptophanes, les groupes thioéthers des méthionines, les groupes imidazoles des histidines.

Si le ligand ne comporte pas de groupe fonctionnel à l'état naturel, de tels groupes peuvent être introduits. Des méthodes d'introduction de groupes fonctionnels sont notamment décrites dans C. Kessler, Nonisotopic probing, Blotting and Sequencing, 2nd édition, L.J. Kricka (1995), Ed. Academic press Ltd., Londres, p. 66-72.

Une autre approche pour le marquage d'un ligand par un composé fluorescent consiste à introduire un groupe réactif dans le ligand, par exemple un groupe NHS ou un groupe maléimide, et de le mettre en présence d'un fluorophore portant un groupe fonctionnel qui réagira avec le groupe réactif pour former une liaison covalente.

Il est important de vérifier que le ligand marqué conserve une affinité suffisante pour son récepteur ; cela peut être contrôlé de manière simple par des expériences de liaison classiques, permettant de calculer la constante d'affinité du ligand marqué pour le récepteur.

Plusieurs auteurs ont décrit le marquage de ligands connus de GPCR par des composés fluorescents et l'homme du métier est à même de choisir les ligands appropriés selon les GPCR étudiés. On peut citer en particulier les publications suivantes:

- Middleton, R.J. & Kellam, B., ont publié un article de revue décrivant de nombreux ligands fluorescents (2005. Fluorophore-tagged GPCR ligands. Current Opinion in Chemical Biology, 9(5), 517-525).
- Beaudet, A. et al. ont présenté les principes essentiels à la préparation de ligands fluorescents (1998. Fluorescent ligands for studying neuropeptide receptors by confocal microscopy. Brazilian Journal of Médical and Biological Research, 31(11), 1479-1489).
- Thierry Durroux et al. ("Fluorescent Pseudo-Peptide Linear Vasopressine Antagonists: Design, Synthesis, and Applications " Journal of Médicinal Chemistry 42, no. 7 Avril 1, 1999: 1312-1319), ont décrit des ligands du récepteur V1a, marqués à la fluorescéine.
- La demande internationale de brevet WO00/12544 décrit un procédé pour marquer diverses chimiokines, qui sont les ligands des récepteurs aux chimiokines.
- La demande internationale de brevet WO2004/088312 décrit la synthèse d'agonistes et d'antagonistes des récepteurs β2-adrénergiques, marqués par un dérivé du boron-dipyrrométhène (« Bodipy »).
- L Hein et al. ("Intracellular trafficking of angiotensine II and its AT1 and AT2 receptors: evidence for selective sorting of receptor and ligand," Molecular Endocrinology (Baltimore, Md.) 11, no. 9 (Août 1997): 1266-1277), ont synthétisé un conjugué angiotensine-fluoréscéine.
- Le brevet US4767718 décrit la synthèse de ligands fluorescent des récepteurs opioïdes, en particulier des antagonistes de ces récepteurs dérivés de la naloxone et de la naltrexone.
- Heather L Handl et al. ("Lanthanide-based time-resolved fluorescence of in cyto ligand-receptor interactions," Analytical Biochemistry 330, no. 2 (Juillet 15, 2004): 242-250) présentent la fabrication d'un analogue de l'α-MSH, qui est un ligand des récepteurs de la mélacortine, marqué par un chélate d'europium.
- Bakthavachalam, V. et al. ont décrit le marquage de ligands des récepteurs dopaminergiques D1 et D2 par de la fluorescéine ou du NBD, en particulier du NAPS (dérivé de la spipérone, antagoniste D2), du PPHT (agoniste D1), de SKF83566 (antagoniste D1), et du SKF38393 (agoniste D1) (1991. Fluorescent probes for dopamine receptors: synthesis and characterization of fluorescein and 7-nitrobenz-2-oxa-1,3-diazol-4-yl conjugates of D-1 and D-2 receptor ligands. Journal of Medicinal Chemistry, 34(11), 3235-3241).

A titre d'exemple non limitatif de ligand utilisable dans les méthodes selon l'invention, on peut ajouter les ligands suivants :

EDA9 (antagoniste du récepteur V2, dont la synthèse est décrit par Manning, M. et al., 1992. Potent V2/V1a vasopressin antagonists with C-terminal ethylenediamine-linked retro-amino acids. Journal of Medicinal Chemistry, 35(21), 3895-3904), NAPS (antagoniste du récepteur D2), RS544 (antagoniste du récepteur V1a), SDF1 (agoniste de CXCR4 et CXCR7), Propanolol (antagoniste des récepteurs beta2-adrénergiques), VIP (agoniste de VPAC1 et de VPAC2), NDPalphaMSH ( agoniste des récepteurs MC3 et MC4), MIP1alpha (protéine inflammatoire des macrophages = CCL3 , agoniste de CCR1 et CCR5), RANTES (= CCL5, agoniste de CCR5), MDC (= CCL22, agoniste de CCR4), Angiotensine II (agoniste du récepteur de l'AT1), Substance P (agoniste du récepteur NK1), Neurokinine A (agoniste du récepteur NK2).

Par ailleurs, certains ligands fluorescents sont disponibles dans le commerce : c'est le cas par exemple de la naloxone-fluorescéine (antagoniste opioïde) qui est commercialisée par Molecular Probe. Cisbio bioassays commercialise également une large gamme de ligands fluorescents de récepteurs membranaires appropriés à la mise en oeuvre de l'invention, en particulier des dérivés fluorescents de la prazosine (antagoniste du récepteur alpha adrénergique), du propanolol (antagoniste du récepteur beta 2 adrénergique), de l'angiotensine II (agoniste des récepteur AT1 et AT2), du HOE140 (antagoniste du récepteur bradykinine B2), du SDF1alpha (agoniste du CXCR4), de la cholecystokinine (agoniste du récepteur CCK1, CCK2), de la spiperone et du NAPS (antagonistes du récepteur dopaminergique D2), du CGP 54626 (antagoniste du récepteur GABAB), de la ghréline (antagoniste du récepteur GHSR1A), du GIP (« Gastric inhibitory polypeptide », agoniste du récepteur GIPR), de la mepyramine (agoniste inverse du récepteur de l'histamine H1), de la MSH (« melanocyte stimulating hormone », agoniste des récepteurs de la melanocortine MC3, MC4, MC5),

de la naltrexone (antagoniste des récepteurs mu, delta et kappa opioïdes), de la substance P (agoniste des récepteurs de la neurokinine NK1), de la neurokinine A (agoniste du récepteur de la neurokinine NK2), d'un agoniste spécifique de VPAC1 ne se liant pas à VPAC2, de l'arginine-vasopressine (antagoniste des récepteurs V1a et V2).

Ces ligands sont utilisables lorsque R1 et/ou R2 sont l'un des récepteurs membranaires auxquels se lient ces ligands.

Ainsi l'EDA9 conjugué à un composé fluorescent est utile pour mettre en oeuvre l'invention avec les hétérodimères V1a-V2, V2-OT, D2-V2 ou l'homodimère V2-V2.

Le NAPS ou la spiperone conjugué à un composé fluorescent sont utiles pour mettre en oeuvre l'invention avec les hétérodimères D2-A1, D2-A2A, D2-SSTR5, D2-D3, D2-SSTR1B, D2-CCR4, D2-CCR3, D2-CCR1, D2-NK1, D2-NK2, D2-AT1, D2-MC3, D2-MC4, D2-Mu Opioïde, D2-GHSR1a, D2-ETA, D2-ETB, D2-CCK1, D2-CCK2, D2-VPAC1, D2-VPAC2, D2- β2AR, D2-CXCR4, D2-CXCR7, D2-V2 et les homodimères D2-D2.

Le RS544 conjugué à un composé fluorescent est utile pour mettre en oeuvre l'invention avec les hétérodimères V1a-V2, V1a-OT, et les homodimères V1a-V1a.

La naltrexazone conjuguée à un composé fluorescent est utile pour mettre en oeuvre l'invention avec les hétérodimères μ-opioïde-SSTR1A, K-opioïde-δ-opioïde, μ-opioïde-δ-opioïde, δ-opioïde-α2aAR, δ-opioïde-SSTR2A, δ-opioïde-NK1-P, δ-opioïde-β2AR, μ-opioïde -D2 et les homodimères μ-opioïde- μ-opioïde et δ-opioïde-δ-opioïde.

La substance P conjuguée à un composé fluorescent est utile pour mettre en oeuvre l'invention avec les hétérodimères NK1- δ-opioïde, NK1-D2.

L'angiotensine II conjuguée à un composé fluorescent est utile pour mettre en oeuvre l'invention avec les hétérodimères AT2-β2AR, AT2-B2, AT2-AT1, AT1-D2.

RANTES conjugué à un composé fluorescent est utile pour mettre en oeuvre l'invention avec les hétérodimères CCR5-CCR2 et les homodimères de CCR5.

Le propanolol conjugué à un composé fluorescent est utile pour mettre en oeuvre l'invention avec les hétérodimères β2AR-AT2, β2AR-δ-opioïde, β2AR- K-opioïde, β2AR-Y1, β2AR- H2, β2AR-M3, β2AR-5HT2B, β2AR-5HT2C, β2AR-H3, β2AR-EP1, β2AR-β3AR, β2ARα2aAR, β2AR-M71, β2AR -D2 et les homodimères β2AR-β2AR.

Le SDF1 conjugué à un composé fluorescent est utile pour mettre en oeuvre l'invention avec les hétérodimères CXCR4-CCR2, CXCR4-CCRΔ32, CXCR4-CXCR2B, CXCR4-D2 et les homodimères CXCR4CXCR4.

**PROTEINE MEMBRANAIRE : MONOMERES, DIMERES, HOMODIMERES ET HETERODIMERES**

L'invention peut être mise en oeuvre avec divers récepteurs membranaires mais de manière préférée, les récepteurs membranaires R1 et R2 sont des GPCR.

Les récepteurs membranaires sont exprimés dans les membranes cellulaires de manière naturelle, ou bien sont exprimés en utilisant les techniques classiques de biologie moléculaire, en particulier des vecteurs d'expression introduits dans les cellules de manière stable ou transitoire. Les réactifs destinés à l'introduction d'ADN hétérologue dans des cellules, de manière stable ou transitoire, sont disponibles dans le commerce et les séquences d'ADN codant pour les récepteurs d'intérêts, en particulier celles codant pour les GPCR, sont disponibles dans les bases de données telles que Genbank. Lorsque les récepteurs d'intérêt sont exprimés par les cellules de manière stable, des phénomènes de cytotoxicité peuvent être observés en raison de la présence d'un trop grand nombre de GPCR; dans ces cas là, il peut être intéressant d'utiliser un système d'expression inductible pour limiter l'expression des GPCR.

Ainsi, la méthode selon l'invention peut comprendre une étape préliminaire de transfection de cellules avec un vecteur d'expression codant pour les récepteur membranaires R1 et / ou R2. Comme décrit plus bas, ce / ces vecteurs peuvent également contenir les séquences codant pour des enzymes suicides permettant le marquage covalent des récepteurs membranaires avec un partenaire de FRET. En particulier, la transfection des cellules s'effectue au moyen d'un vecteur d'expression comprenant la séquence d'ADN codant pour le récepteur R1 d'une part, et par un vecteur d'expression comprenant la séquence d'ADN codant pour le récepteur R2 d'autre part.

De manière préférée les récepteurs R1 et R2 sont chacun exprimés par un vecteur d'expression introduit dans la cellule de manière stable ou transitoire.

L'invention est particulièrement adaptée à l'étude de la pharmacologie des GPCR. Une liste des GPCR connus a été publiée et les séquences d'ADN codant pour ces récepteurs sont également accessibles (Harmar AJ et al (2009) IUPHAR-DB: the IUPHAR database of G protein-coupled receptors and ion channels. Nucl. Acids Res. 37 (Database issue): D680-D685). Cette liste, résumée dans la table 1, mentionne également les ligands naturels connus de ces récepteurs.

**Table 1 - GPCR**

| Famille | Ligand naturel | Nom officiel IUPHAR |
|---|---|---|
| Récepteurs de la 5-hydroxytryptam ine | 5-hydroxytryptamine | 5-HT1A |
| | 5-hydroxytryptamine | 5-HT1B |
| | 5-hydroxytryptamine | 5-HT1D |
| | 5-hydroxytryptamine | 5-ht1e |
| | 5-hydroxytryptamine | 5-HT1F |
| | 5-hydroxytryptamine | 5-HT2A |
| | 5-hydroxytryptamine | 5-HT2B |
| | 5-hydroxytryptamine | 5-HT2C |
| | 5-hydroxytryptamine | 5-HT4 |
| | 5-hydroxytryptamine | 5-ht5a |
| | 5-hydroxytryptamine | 5-HT6 |
| | 5-hydroxytryptamine | 5-HT7 |
| Récepteurs de l'acetylcholine (muscariniques) | acétylcholine | M1 |
| | acétylcholine | M2 |
| | acétylcholine | M3 |
| | acétylcholine | M4 |
| | acétylcholine | M5 |
| Récepteurs de l'adénosine | adénosine | A1 |
| | adénosine | A2A |
| | adénosine | A2B |
| | adénosine | A3 |
| Récepteurs adrénergiques | noradrénaline | α1A-adrenoceptor |
| | adrénaline | α1B-adrenoceptor |
| | adrénaline | α1D-adrenoceptor |
| | adrénaline | α2A-adrenoceptor |
| | adrénaline | α2B-adrenoceptor |
| | adrénaline | α2C-adrenoceptor |
| | noradrénaline | β1-adrenoceptor |
| | adrénaline | β2-adrenoceptor |
| | adrénaline | β3-adrenoceptor |
| Récepteurs de l'anaphylatoxine | anaphylatoxine C5a, C5a des Arg74 | C5L2 |
| | anaphylatoxine C5a | C5a |
| | anaphylatoxine C3a | C3a |

(suite)

| Famille | Ligand naturel | Nom officiel IUPHAR |
|---|---|---|
| Récepteurs de l'angiotensine | angiotensine | AT1 |
| | angiotensine | AT2 |
| Récepteurs de l'apéline | apéline | APJ |
| Récepteurs des acides biliaires | Acides biliaires | GPBA |
| Récepteurs de la bombésine | neuromédine B | BB1 |
| | gastrine-releasing peptide | BB2 |
| | | BB3 |
| Récepteurs de la Bradykinine | Bradykinine | B1 |
| | Bradykinine | B2 |
| Récepteurs des cannabinoïdes | cannabinoïides | CB1 |
| | cannabinoïdes | CB2 |
| Récepteurs des chimiokines | CXCL8 | CXCR1 |
| | CXCL1-3, CXCL5-8, macrophage derived lectine | CXCR2 |
| | CXCL9-11 | CXCR3 |
| | CXCL12 | CXCR4 |
| | CXCL13 | CXCR5 |
| | CCL3, CCL5, CCL7, CCL8, CCL13-16, CCL23 | CCR1 |
| | CCL2, CCL7, CCL8, CCL13 | CCR2 |
| | CCL11 (eotaxin); CCL5, CCL7, CCL8, CCL13, CCL15, CCL24, CCL26 | CCR3 |
| | CCL17, CCL22 | CCR4 |
| | CCL3, CCL4, CCL5, CCL8, CCL14 | CCR5 |
| | CCL20 | CCR6 |
| | CCL19, CCL21 | CCR7 |
| | CCL1, CCL4, CCL17 | CCR8 |
| | CCL25 | CCR9 |
| | CCL26-28 | CCR10 |
| | CX3CL1 | CX3CR1 |
| | XCL1, XCL2 | XCR1 |
| | CXCL16 | CXCR6 |
| Récepteurs de la cholécystokinine | cholécystokinine | CCK1 |
| | cholecystokinin, gastrine | CCK2 |
| Récepteurs de la dopamine | dopamine | D1 |
| | dopamine | D2 |
| | dopamine | D3 |
| | dopamine | D4 |
| | dopamine | D5 |

(suite)

| Famille | Ligand naturel | Nom officiel IUPHAR |
|---|---|---|
| Récepteurs de l'endothéline | endothéline 1, endothéline 2 | ETA |
| | endothélines 1, 2 and 3 | ETB |
| Récepteurs de l'estrogène (couplé aux protéines G) | | GPER |
| Récepteurs des formylpeptides | | FPR2/ALX |
| | | FPR3 |
| | | FPR1 |
| Récepteurs des acides gras libres | Acides carboxyliques à longue chaine | FFA1 |
| | | FFA3 |
| | | FFA2 |
| | | GPR42 |
| Récepteurs de la galanine | galanine | GAL1 |
| | galanine | GAL2 |
| | galanine | GAL3 |
| Récepteurs de la ghréline | ghréline | ghrelin |
| Récepteurs des hormones glycoprotéiques | follicle-stimulating hormone | FSH |
| | luteinizing hormone, chorionic gonadotropin | LH |
| | thyroid-stimulating hormone | TSH |
| Récepteurs de la gonadolibérine | gonadolibérine | GnRH |
| | gonadolibérine | GnRH2 |
| Récepteurs de l'histamine | histamine | H1 |
| | histamine | H2 |
| | histamine | H3 |
| | histamine | H4 |
| Récepteurs du peptide dérivé de KiSS1 | | KiSS1 |
| Récepteurs des leucotriènes | leukotriène B4 | BLT2 |
| | | FPR2/ALX |
| | 5-oxo-ETE | OXE |
| | leucotriène B4 | BLT1 |
| | leucotriene D4 | CysLT1 |
| | leucotriene C4 | CysLT2 |
| Récepteurs des lysophospholipides | acide lysophosphatidique | LPA1 |
| | acide lysophosphatidique | LPA2 |
| | acide lysophosphatidique | LPA3 |
| | sphingosine 1-phosphate | S1P1 |
| | sphingosine 1-phosphate | S1P2 |
| | sphingosine 1-phosphate | S1P3 |

(suite)

| Famille | Ligand naturel | Nom officiel IUPHAR |
|---|---|---|
| | sphingosine 1-phosphate | S1P4 |
| | sphingosine 1-phosphate | S1P5 |
| Récepteurs de l'hormone de mélano-concentration | | MCH1 |
| | | MCH2 |
| Récepteurs de la mélanocortine | melanocyte stimulating hormone | MC1 |
| | adrenocorticotropic hormone | MC2 |
| | γ melanocyte stimulating hormone | MC3 |
| | β-melanocyte stimulating hormone | MC4 |
| | α-melanocyte stimulating hormone | MC5 |
| Récepteurs de la mélatonine | mélatonine | MT1 |
| | mélatonine | MT2 |
| Récepteur de la motiline | motiline | motiline |
| Récepteurs de la neuromédine U | neuromédine U | NMU1 |
| | neuromédine U | NMU2 |
| Récepteurs du neuropeptide FF/neuropeptide AF | | NPFF1 |
| | | NPFF2 |
| Récepteur du neuropeptide S | | NPS |
| Récepteurs du neuropeptide W/neuropeptide B | | NPBW1 |
| | | NPBW2 |
| Récepteurs du neuropeptide Y | neuropeptide Y | Y1 |
| | neuropeptide Y | Y2 |
| | pancreatic polypeptide | Y4 |
| | neuropeptide Y | Y5 |
| Récepteurs de la neurotensine | neurotensine | NTS1 |
| | neurotensine | NTS2 |
| Récepteurs de l'acide nicotinique | nicotinic acid (low affinitv) | GPR109B (temporary name) |
| | nicotinic acid (high affinity) | GPR109A (temporary name) |
| | lactate | GPR81 (temporary name) |
| Non-signalling 7TM chimiokine-binding proteins | | FY |
| | | CCPB2 |
| | | CCRL1 |
| Récepteurs des opioïdes | β-endorphine | μ |
| | β-endorphine | δ |
| | dynorphine A | K |
| | nociceptine/orphanine FQ | NOP |

(suite)

| Famille | Ligand naturel | Nom officiel IUPHAR |
|---|---|---|
| Récepteurs des orexines | orexine A, orexine B | OX1 |
| | orexine A, orexine B | OX2 |
| Récepteurs P2Y | ADP | P2Y1 |
| | UTP, ATP | P2Y2 |
| | UTP | P2Y4 |
| | UDP | P2Y6 |
| | ATP | P2Y11 |
| | ADP | P2Y12 |
| | UDP-glucose | P2Y14 |
| | ADP | P2Y13 |
| Récepteur du peptide P518 | RF-amide P518 gene product | QRFP |
| Récepteur du facteur activateur des plaquettes | platelet-activating factor | PAF |
| Récepteurs de la prokinéticine | | PKR1 |
| | | PKR2 |
| Récepteurs de la prolactolibérine | | PRRP |
| Récepteurs des prostanoïdes | prostaglandine D2 | DP1 |
| | prostaglandine E2 | EP1 |
| | prostaglandine E2 | EP2 |
| | prostaglandine E2 | EP3 |
| | prostaglandine E2 | EP4 |
| | prostaglandine F2a | FP |
| | prostacycline | IP1 |
| | thromboxane A2 | TP |
| | 11-déshydrothromboxane B2 | DP2 |
| Récepteurs activés par les protéases | thrombine | PAR1 |
| | serine protéase | PAR2 |
| | thrombine | PAR3 |
| | serine protéase | PAR4 |
| Récepteurs des peptides de la famille de la relaxine | | RXFP1 |
| | | RXFP2 |
| | | RXFP3 |
| | | RXFP4 |

(suite)

| Famille | Ligand naturel | Nom officiel IUPHAR |
|---|---|---|
| Récepteurs de la Somatostatine | somatostatine | sst2 |
| | somatostatine | sst5 |
| | somatostatine | sst3 |
| | somatostatine | sst1 |
| | somatostatine | sst4 |
| Récepteurs de la tachykinine | substance P | NK1 |
| | neurokinine A | NK2 |
| | neurokinine B | NK3 |
| Récepteur de la thyréolibérine | thyrotropine-releasing hormone | TRH1 |
| Récepteur des amines à l'état de trace (« Trace Amines ») | | TA1 |
| Récepteur de l'urotensine | urotensine II | UT |
| Récepteurs de la Vasopressine et de l'ocytocine | Vasopressine | V1A |
| | Vasopressine | V2 |
| | Vasopressine | V1B |
| | ocytocine | OT |
| Récepteurs Olfactifs | | |
| Récepteurs de la calcitonine | amylin, CGRP | CT |
| | | AMY1 |
| | | AMY2 |
| | | AMY3 |
| | adrenomedulline, CGRP | CALCRL |
| | | CGRP |
| | | AM1 |
| | | AM2 |
| Récepteurs de la corticolibérine | | CRF1 |
| | | CRF2 |
| Récepteurs du glucagon | glucagon | glucagon |
| | | GLP-1 |
| | | GLP-2 |
| | | GIP |
| | sécrétine | sécrétine |
| | | GHRH |
| Récepteurs de l'hormone parathyroïdienne | hormone parathyroïdienne | PTH1 |
| | TIP-39 | PTH2 |

(suite)

| Famille | Ligand naturel | Nom officiel IUPHAR |
|---|---|---|
| Récepteurs du VIP et du PACAP | PACAP | PAC1 |
| | VIP, PACAP | VPAC1 |
| | VIP, PACAP | VPAC2 |
| Récepteurs sensibles au calcium | Calcium | CaS |
| | | GPRC6 |
| Récepteurs du GABAB | GABAB | GABAB1 |
| | | GABAB2 |
| | | GABAB |
| Récepteurs GPRC5 | | RAIG1 |
| | | RAIG2 |
| | | RAIG3 |
| | | RAIG4 |
| Récepteurs métabotropiques du glutamate | Glutamate | mGlu1 |
| | Glutamate | mGlu2 |
| | Glutamate | mGlu3 |
| | Glutamate | mGlu4 |
| | Glutamate | mGlu5 |
| | Glutamate | mGlu6 |
| | Glutamate | mGlu7 |
| | Glutamate | mGlu8 |
| Récepteurs du goût | | T1R1 |
| | | T1R2 |
| | | T1R3 |
| Récepteurs "Frizzled" | Wnt3A, Wnt3, Wnt1, Wnt2 | FZD1 |
| | Wnt | FZD2 |
| | Wnt | FZD3 |
| | Wnt | FZD4 |
| | Wnt | FZD5 |
| | Wnt | FZD6 |
| | Wnt | FZD7 |
| | Wnt | FZD8 |
| | Wnt | FZD9 |
| | Wnt | FZD10 |
| | constitutif | SMO |

La table 2 donne des exemples d'hétérodimères de GPCR constitués d'un premier récepteur R1 et d'un second récepteur R2. Ces hétérodimères peuvent être utilisés dans les méthodes selon l'invention.

**Table 2**

| R1 est un GPCR | | | R2 est un GPCR | | |
|---|---|---|---|---|---|
| | classe | type | | classe | type |
| | | | | | |
| MgluR2 | C | metabo Glu | MgluR3 | C | metabo Glu |
| MgluR2 | C | metabo Glu | MgluR4 | C | metabo Glu |
| MgluR2 | C | metabo Glu | MgluR8 | C | metabo Glu |
| MgluR3 | C | metabo Glu | MgluR4 | C | metabo Glu |
| MgluR3 | C | metabo Glu | MgluR8 | C | metabo Glu |
| MgluR4 | C | metabo Glu | MgluR8 | C | metabo Glu |
| MgluR1 | C | metabo Glu | CaS | C | calcium sensing |
| MgluR1 | C | metabo Glu | A1 | A | adenosine |
| MgluR2 | C | metabo Glu | 5-HT2A | A | serotonine |
| MgluR5 | C | metabo Glu | A2A | A | adenosine |
| GABAB1 | C | GABAB | GABAB2 | C | GABAB |
| 5-HT1B | A | sérotonine | 5-HT1D | A | sérotonine |
| A1 | A | adénosine | D1 | A | Dopamine |
| A1 | A | adénosine | D2 | A | Dopamine |
| A1 | A | adénosine | P2Y1 | A | Purinergique |
| A2A | A | adénosine | D2 | A | Dopamine |
| A2A | A | adénosine | D3 | A | Dopamine |
| AT1 | A | angiotensine | AT2 | A | angiotensine |
| AT1 | A | angiotensine | B2 | A | Bradykinine |
| AT2 | A | angiotensine | β2AR | A | B2-adrenoceptor |
| AT2 | A | angiotensine | B2 | A | Bradykinine |
| M2 | A | muscaranic Achl | M3 | A | muscaranic Achl |
| MT1 | A | mélatonine | MT2 | A | mélatonine |
| SSTR2A | A | somatostatine | SSTR1B | A | somatostatine |
| D2 | A | dopamine | SSTR5 | A | somatostatine |
| D2 | A | dopamine | D3 | A | dopamine |
| D1 | A | dopamine | D2 | A | Dopamine |
| CCR2 | A | chimiokine | CCR5 | A | chimiokine |
| SSTR1A | A | somatostatine | μ-opioïde | A | opioïde |
| SSTR1A | A | somatostatine | SSTR1C | A | somatostatine |
| SSTR1 | A | somatostatine | SSTR5 | A | somatostatine |
| SSTR1B | A | somatostatine | D2 | A | dopamine |
| SSTR2 | A | somatostatine | SSTR3 | A | somatostatine |
| T1R1 | C | goût | T1R3 | C | goût |
| T1R2 | C | goût | T1R3 | C | goût |

(suite)

| R1 est un GPCR | | | R2 est un GPCR | | |
|---|---|---|---|---|---|
| | classe | type | | classe | type |
| δ-opioïde | A | opioïde | K -opioïde | A | opioïde |
| μ-opioïde | A | opioïde | δ-opioïde | A | opioïde |
| δ-opioïde | A | opioïde | α2aAR | A | A2-adrenoceptor |
| δ-opioïde | A | opioïde | SSTR2A | A | somatostatine |
| δ-opioïde | A | opioïde | NK1-P | A | tachykinine |
| δ-opioïde | A | opioïde | β2AR | A | B2-adrenoceptor |
| δ-opioïde | A | opioïde | SNSR-4 | A | |
| K-opioïde | A | opioïde | β2AR | A | B2-adrenoceptor |
| Orexin1 | A | Orexin | CB1 | A | Cannabinoid |
| MT1 | A | mélatonine | GPR50 | ? | Orph |
| MrgE | ? | Orph | MrgD | ? | Orph |
| ETA | A | endothéline | ETB | A | endothéline |
| V1a | A | Vasopressine | V2 | A | Vasopressine |
| V1a | A | Vasopressine | OT | A | Ocytocine |
| V2 | A | Vasopressine | OT | A | Ocytocine |
| α1aAR | A | A1-adrenoceptor | α1bAR | A | A1-adrenoceptor |
| α1bAR | A | A1-adrenoceptor | H1 | A | Histamine |
| α1dAR | A | A1-adrenoceptor | α1bAR | A | A1-adrenoceptor |
| β1AR | A | B1-adrenoceptor | α2aAR | A | A2-adrenoceptor |
| β1AR | A | B1-adrenoceptor | β2AR | A | B2-adrenoceptor |
| β2AR | A | B2-adrenoceptor | M71 | A | olfactory receptor |
| β2AR | A | B2-adrenoceptor | α2aAR | A | A2-adrenoceptor |
| β2AR | A | B2-adrenoceptor | B3AR | A | B3-adrenoceptor |
| β2AR | A | B2-adrenoceptor | EP1 | A | |
| β2AR | A | B2-adrenoceptor | H3 | A | Histamine |
| β2AR | A | B2-adrenoceptor | 5HT2C | A | sérotonine |
| β2AR | A | B2-adrenoceptor | 5HT2B | A | sérotonine |
| β2AR | A | B2-adrenoceptor | M3 | A | muscaranic Achl |
| β2AR | A | B2-adrenoceptor | H2 | A | Histamine |
| β2AR | A | B2-adrenoceptor | Y1 | A | neuropeptide Y |
| 5HT2C | A | sérotonine | β3AR | A | B3-adrenoceptor |
| 5HT2C | A | sérotonine | Y5 | A | neuropeptide Y |
| 5HT2C | A | sérotonine | M1 | A | muscaranic Achl |
| CXCR1 | A | chimiokine | CXCR2 | A | chimiokine |
| CXCR4 | A | chimiokine | CXCR2B | A | chimiokine |
| CXCR4 | A | chimiokine | CCRΔ32 | A | chimiokine |
| CXCR4 | A | chimiokine | CCR2 | A | chimiokine |

(suite)

| R1 est un GPCR | | | R2 est un GPCR | | |
|---|---|---|---|---|---|
| | **classe** | **type** | | **classe** | **type** |
| CCR2 | A | chimiokine | CCR5 | A | chimiokine |
| D2 | A | Dopamine | CCR1 | A | chimiokine |
| D2 | A | Dopamine | CCR3 | A | chimiokine |
| D2 | A | Dopamine | CCR4 | A | chimiokine |
| D2 | A | Dopamine | NK1 | A | tachykinine |
| D2 | A | Dopamine | NK2 | A | tachykinine |
| D2 | A | Dopamine | AT1 | A | angiotensine |
| D2 | A | Dopamine | MC3 | A | mélanocortine |
| D2 | A | Dopamine | MC4 | A | mélanocortine |
| D2 | A | Dopamine | μ-opioïde | A | opioïde |
| D2 | A | Dopamine | GHSR1a | A | ghréline |
| D2 | A | Dopamine | ETA | A | endothéline |
| D2 | A | Dopamine | ETB | A | endothéline |
| D2 | A | Dopamine | CCK1 | A | cholécystokinine |
| D2 | A | Dopamine | CCK2 | A | cholécystokinine |
| D2 | A | Dopamine | VPAC1 | B | VIP, PACAP |
| D2 | A | Dopamine | VPAC2 | B | VIP, PACAP |
| D2 | A | Dopamine | β2AR | A | B2-adrenoceptor |
| D2 | A | Dopamine | CXCR4 | A | chimiokine |
| D2 | A | Dopamine | CXCR7 | A | chimiokine |
| D2 | A | Dopamine | V2 | A | Vasopressine |

Les GPCR de la table 3 peuvent former des homodimères.

**Table 3**

| GPCR | classe | type |
|---|---|---|
| CaSR | C | calciumSensing |
| MgluR1 | C | metabo Glu |
| MgluR2 | C | metabo Glu |
| MgluR3 | C | metabo Glu |
| MgluR4 | C | metabo Glu |
| MgluR5 | C | metabo Glu |
| MgluR6 | C | metabo Glu |
| MgluR7 | C | metabo Glu |
| MgluR8 | C | metabo Glu |
| 5-HT2C | A | serotonine |
| 5-HT1B | A | serotonine |
| 5-HT1C | A | serotonine |
| β1AR | A | B1-adrenoceptor |
| β2AR | A | B2-adrenoceptor |
| V1a | A | Vasopressine |
| V2 | A | Vasopressine |

(suite)

| GPCR | classe | type |
|---|---|---|
| δ-opioïde | A | opioïde |
| μ-opioïde | A | opioïde |
| k-opioïde | A | opioïde |
| D1 | A | dopamine |
| D2 | A | dopamine |
| D3 | A | dopamine |
| H2 | A | Histamine |
| H4 | A | Histamine |
| α2aAR | A | A2-adrenoceptor |
| B4 | A | Bradykinine |
| B2 | A | Bradykinine |
| CCR2 | A | chimiokine |
| CCR5 | A | chimiokine |
| CXCR4 | A | chimiokine |
| CXCR2 | A | chimiokine |
| CXCR1 | A | chimiokine |
| CCK | A | cholecystokinin |
| LTB1 | A | leukotriene |
| MT1 | A | mélatonine |
| MT2 | A | mélatonine |
| M2 | A | muscarinic Achl |
| M3 | A | muscarinic Achl |
| OT | A | Ocytocine |
| SSTR5 | A | somatostatine |
| SSTR1A | A | somatostatine |
| SSTR1B | A | somatostatine |
| SSTR1C | A | somatostatine |
| SSTR2A | A | somatostatine |
| GnRH | A | gonadotrophin |
| TRH | B | thyrotropin |
| GHSR1 | A | ghréline |
| IgGhepta | B | |
| A1 | A | adénosine |
| PACAP | B | |
| PAR1 | A | thrombine |
| AT1 | A | angiotensine |
| Rhodopsin | A | |
| Frizzled4 | | |

(suite)

| GPCR | classe | type |
|---|---|---|
| MC1 | A | mélacortine |

## EXEMPLES

**Exemple 1 :** Préparation de cellules exprimant des monomères et des homodimères de récepteurs V2, fusionnés avec une enzyme suicide SNAPtag (ST) ou CLIPtag (CT)

Réactifs et matériels utilisés :

OptiMEM (Invitrogen (51985-026))

Krebs-glucose : Tampon Krebs + glucose 0,5g/l
Plasmide **FLAG-ST-V2** : plasmide comportant la séquence codant pour une protéine de fusion comprenant un peptide signal d'adressage membranaire T8, l'épitope FLAG, l'enzyme SNAPTAG et le récepteur V2. La séquence de ce plasmide est la SEQ ID n°4.
Plasmide **FLAG-CT-V2** : plasmide comportant la séquence codant pour une protéine de fusion comprenant un peptide signal d'adressage membranaire T8, l'épitope FLAG, l'enzyme CLIPTAG et le récepteur V2. La séquence de ce plasmide est identique à celle du plasmide FLAG-ST-V2 à l'exception de la séquence codant pour SNAPtag qui a été remplacée par celle codant pour CLIPtag. Un vecteur comportant l'enzyme CLIP-tag est commercialisé par la société New England biolabbs (NEB), qui fournit également sa séquence.

Traitement des plaques

50 µl de solution de poly-L-Ornithine (solution 0,01%, poids moléculaire 30,000-70,000 (SIGMA P4957)) ont été distribués dans chaque puits d'une plaque de 96 puits (Cellstar, noire à fond noir) afin de favoriser l'adhérence des cellules au fond du puits, et les plaques ont été mises à incuber pendant 30 min à 37° C.

Transfection

Le mélange de transfection suivant a ensuite été distribué dans chaque puits :

- 0,16µg de plasmide FLAG-ST-V2 + 0,16µg de plasmide FLAG-CT-V2
- 0,8 µl de Lipofectamine 2000
- 50 µl de milieu OptiMEM

Après 30 minutes d'incubation, 100 µl d'une suspension contenant 100 000 cellules COS7 ont été ajoutées dans chaque puits, puis incubées pendant 24 heures à 37°C en présence de 5% de $CO_2$.

**Exemple 2 :** Préparation de cellules exprimant des monomères de récepteurs mGlu3, mGlu2, et des hétérodimères de récepteurs mGlu2-mGlu3

Le même protocole que celui de l'exemple 1 a été utilisé pour l'expression d'hétérodimères mGluR2-mGluR3, en utilisant les même plasmides que dans cet exemple mais en remplaçant la séquence du récepteur V2 par celle du récepteur mGlu2 ou mGlu3. Ainsi, les plasmide FLAG-ST-mGluR2 et FLAG-ST-mGluR3 ont été utilisés pour l'expression des dimères mGluR2-mGluR3, dans lesquels mGluR2 est fusionné à SNAPtag et mGluR3 est fusionné à CLIPtag.

**Exemple 3 :** Cellules exprimant le récepteur de la dopamine D2 et le récepteur Delta Opioïde

3.1 Réactifs :

- *Milieu dit complet* : DMEM Glutamax™-L, 10% Sérum de Veau Foetal, 1% MEM NEAA (Acides Aminés Non Essentiels), 1% Pen-Strep, 2mM HEPES. Fournisseur : Gibco (Invitrogen).

- *Opti MEM Glutamax™-L* commercialisé par Gibco.
- *tampon Tag-Lite®* : commercialisé par Cisbio bioassays.
- *Tag-Lite® SNAP-Lumi4Tb :* Substrat de l'enzyme SNAP-Tag, commercialisé par Cisbio Bioassays. Ce réactif permet ici de marquer le récepteur SNAPTag-Delta Opioïde.
- *Tag-Lite® Halo-Lumi4Tb :* Substrat de l'enzyme Halo-Tag, commercialisé par Cisbio Bioassays. Ce réactif permet ici de marquer le récepteur HaloTag-Dopamine D2.
- *Antagoniste rouge du récepteur de la dopamine D2*: Ligand du récepteur Dopamine D2 (dérivé de la Spiperone), couplé à un fluorophore accepteur rouge. Commercialisé par Cisbio (« Opioid receptor red antagonist », Ref. L0002RED).
- *Antagoniste rouge du récepteur des opioïdes* : Ligand du récepteur Delta Opioïde (dérivé de la naltrexone), couplé à un fluorophore accepteur rouge. Commercialisé par Cisbio (« Opioid receptor red antagonist », Ref. L0005RED).
- *Antagonistes, Agonistes, Agonistes inverses spécifiques du récepteur Dopamine D2* : NAPS (antagoniste) fournit par Columbia University, New-York; Bromocriptine (agoniste partiel), Spiperone (antagoniste), Halopéridol (agoniste inverse), (S)-(-)-Sulpiride (agoniste inverse) commercialisés par Tocris ; PPHT (agoniste) de Sigma. Ces six composés sont repris chacun dans une solution contenant 100% de Diméthylsulfoxyde (DMSO, Sigma), exception faite du NAPS qui est resolubilisé dans 10% de DMSO et 90% d'eau.
- *Antagonistes, Agonistes, Agonistes inverses spécifiques du récepteur Delta Opioïde* : Endomorphine I (agoniste) resolubilisé dans 100% $H_2O$, Naltrindole (antagoniste) repris dans 100% de DMSO, SNC-162 (agoniste) resolubilisé dans $H_2O$ + 1 équivalent HCl. Naloxone (Antagoniste) resolubilisé dans 90% $H_2O$ et 10% de DMSO. Ces composés sont commercialisés par Tocris.
- *Plasmide Flag-SNAP-Delta Opioïde* : plasmide comportant la séquence codant pour une protéine de fusion comprenant un peptide signal d'adressage membranaire T8, l'épitope FLAG, l'enzyme SNAPTAG et le récepteur Delta Opioïde. La séquence de ce plasmide est la SEQ ID n°5.
- *Plasmide Flag-Halo-Dopamine D2* : plasmide comportant la séquence codant pour une protéine de fusion comprenant un peptide signal d'adressage membranaire T8, l'épitope FLAG, l'enzyme Halotag et le récepteur de la dopamine D2. La séquence de ce plasmide est la SEQ ID n°6.

3.2 Cultures cellulaires :

Des cellules HEK293T/17 adhérentes ont été mise en culture avec 25 ml de milieu de culture dit complet, dans des flasque Easy Flask T175 (Nunc), à 37 °C sous atmosphère humide à 5 % de $CO_2$. Les cellules ont été décollées de la flasque dont le milieu de culture a été préalablement éliminé, grâce à 5 ml de tampon de dissociation cellulaire (Gibco). La densité et la viabilité des cellules ont été évaluées grâce à un compteur de cellules Vi-Cell™-XR (Beckman Coulter).

3.3. Transfection transitoire et marquage :

50 µl de solution de poly-L-Ornithine (solution 0,01%, poids moléculaire 30,000-70,000 (SIGMA P4957)) ont été distribués dans chaque puits d'une plaque de 96 puits (Cellstar, noire à fond noir) afin de favoriser l'adhérence des cellules au fond du puits, et les plaques ont été mises à incuber pendant 20 min à 37° C.
Le mélange de transfection suivant a été préparé, pour chaque puits :

- 48,5 µl de milieu OptiMEM préalablement chauffé à 37°C
- 0,35 µg de plasmide Flag-SNAP-Delta Opioïde + 0,35 µg de plasmide Flag-Halo-Dopamine D2
- 0,8 µl de Lipofectamine 2000

Après mélange au vortex et 30 minutes d'incubation à température ambiante, ce mélange a été déposé dans chaque puits de la plaque, après aspiration de la Poly-L-Ornithine, et à raison de 50 µl par puits.
100 µL d'une suspension de cellules HEK293T à une concentration de 1 million/ml (préparée en 3.2) ont ensuite été ajoutés dans chaque puits, soit 100 000 cellules par puits. La plaque a alors été incubée 24 h à 37 °C sous atmosphère humide à 5% de $CO_2$. Au terme des 24 h d'incubation, le milieu dans chaque puits a été aspiré. 100 µL d'une solution de SNAP-Lumi4Tb ou HALO-Lumi4Tb à 100 nM ont été ensuite ajoutés dans chaque puits, pour marquer les récepteurs avec les substrats fluorescents, par réaction avec les domaines Snaptag ou Halotag des protéines de fusion. La plaque a ensuite été incubée à 37°C pendant 1h, puis rincée par quatre lavages successifs avec 100 µl par puits de tampon Tag-Lite®. Enfin, 50 µl de ce tampon ont été ajoutés dans chaque puits.

3.4. Détermination de l'affinité des ligands de référence fluorescents sur l'hétérodimère delta opioïde - D2

L'affinité des ligands de référence fluorescents pour les hétérodimères a été déterminée en incubant les cellules à température ambiante avec des concentrations croissantes de ligand fluorescent. Pour chaque concentration de ligand fluorescent, le signal de liaison non spécifique est déterminé en ajoutant un excès de ligand non fluorescent (10 μM de NAPS pour Dopamine D2 et 10 μM de Naltrindole pour delta Opioïde). Les ligands fluorescents et non fluorescents ont été dilués dans le tampon Tag-lite. Dans les plaques préparées à l'exemple 3.3, 25 μl de ligand non fluorescent ou de tampon Tag-lite ont été ajoutés, suivis de l'ajout de 25 μl de ligand fluorescent. Les plaques ont ensuite été incubées à température ambiante pendant 3h avant détection du signal.

Le signal a été mesuré à 665 nm et 620 nm sur un lecteur Rubystar (BMG Labtech). Le ratio HTRF a ensuite été calculé en divisant le signal de l'accepteur (665 nm) par celui du donneur (620 nm) et en multipliant cette valeur par 10 000. Les résultats ont ensuite été analysés sur GraphPad Prism (GraphPad Software, Inc., San Diego, CA). Les constantes de dissociation (Kd) des ligands fluorescents ont été obtenues à partir des courbes de saturation du signal de liaison spécifique. Le signal spécifique de liaison a été obtenu en soustrayant le ratio HTRF non spécifique au ratio HTRF total.

La figure 8 correspond à la courbe dose réponse obtenue en mesurant le signal émis par des cellules dont le récepteur D2 a été marqué par HALO-Lumi4Tb, en présence de concentration croissante d'antagoniste rouge du récepteur des opioïdes. Le Kd de l'antagoniste rouge du récepteur des opioïdes pour le récepteur Delta Opioïde impliqué dans un hétérodimère est de 4,57 nM.

La figure 9 correspond à la courbe dose réponse obtenue en mesurant le signal émis par des cellules dont le récepteur Delta Opioïde a été marqué par SNAP-Lumi4Tb, en présence de concentration croissante d'antagoniste rouge du récepteur de la dopamine D2. Le Kd de l'antagoniste rouge du récepteur D2 pour le récepteur D2 impliqué dans un hétérodimère est de 1,44 nM.

3.5. Etude de la pharmacologie de différents composés sur les hétérodimères de récepteurs delta opioïde - D2

Des essais de compétitions entre les ligands fluorescents à des concentrations fixes (3 nM de l'antagoniste rouge du récepteur de la dopamine D2 ou du récepteur des opioïdes) et des composés à tester à des concentrations croissantes, ont permis d'évaluer l'affinité de ces composés sur les hétérodimères.

Les ligands fluorescents et les composés à tester ont été dilués dans le tampon Tag-lite. Dans les plaques préparées à l'exemple 3.3, 25 μl de composé à tester ou de tampon Tag-lite ont été ajoutés, suivis de l'ajout de 25 μl de ligand fluorescent. Les plaques ont ensuite été incubées à température ambiante pendant 3h avant détection du signal.

Le signal a été mesuré à 665 nm et 620 nm sur un lecteur Rubystar (BMG Labtech). Le ratio HTRF a ensuite été calculé en divisant le signal de l'accepteur (665 nm) par celui du donneur (620 nm) et en multipliant cette valeur par 10 000. Les résultats ont ensuite été analysés sur GraphPad Prism (GraphPad Software, Inc., San Diego, CA). Les valeurs des constantes d'inhibition (Ki) des composés ont été obtenues à partir des expériences de compétitions grâce à l'équation de Cheng et Prusoff.

La figure 10 représente l'évolution du signal émis par des cellules dont le récepteur D2 a été marqué par HALO-Lumi4Tb, en présence d'une concentration fixe d'antagoniste rouge du récepteur des opioïdes, et de concentration croissante de différents composés à tester (Endomorphine 1, Naloxone, SNC-162 et Naltrindole). Les courbes obtenues montrent que les composés tests rentrent en compétition avec le ligand fluorescent de référence et se lient aux hétérodimères. Cette figure montre également les Ki calculées pour chacun des composés testés.

La figure 11 représente l'évolution du signal émis par des cellules dont le récepteur Delta opioïde a été marqué par SNAP-Lumi4Tb, en présence d'une concentration fixe d'antagoniste rouge du récepteur de la dopamine D2, et de concentration croissante de différents composés à tester (NAPS, PPHT, Bromocriptine, Spiperone, Haloperidol et Sulpiride). Les courbes obtenues montrent que les composés tests rentrent en compétition avec le ligand fluorescent de référence et se lient aux hétérodimères. Cette figure montre également les Ki calculées pour chacun des composés testés.

## Revendications

1. Méthode de détermination de la liaison d'un composé à tester avec des récepteurs membranaires R1 et R2 exprimés dans les membranes cellulaires, ces récepteurs étant connus pour être présents sous forme hétérodimérique, **caractérisée en ce qu'**elle comprend les étapes suivantes :

   (a) Marquage du récepteur R1 de manière covalente ou non-covalente par le premier membre d'un couple de partenaires de FRET (D,A1), dans lequel D est le composé donneur et A1 est le composé accepteur d'énergie ;
   (b) Ajout au milieu de mesure d'un ligand connu de R2 capable de se lier à l'hétérodimère R1R2 mais ni au récepteur R1 ni à l'homodimère R1R1, ledit ligand étant marqué par le second membre dudit couple de partenaires de FRET, et ledit ligand étant choisi parmi : un composé agoniste connu de R2, un composé antagoniste

connu de R2 ou un modulateur allostérique connu de R2 ;
(c) Mesure de la luminescence $L_1$ émise à la longueur d'onde d'émission du composé accepteur $A_1$, en présence et en l'absence du composé à tester.

**2.** Méthode selon la revendication 1, **caractérisée en ce que** le premier membre dudit couple de partenaires de FRET, utilisé pour marquer le récepteur R1, est le composé donneur d'énergie D, et **en ce que** le second membre de ce couple, lié au ligand, est le composé accepteur A1.

**3.** Méthode selon la revendication 1, **caractérisée en ce que** le premier membre dudit couple de partenaires de FRET, utilisé pour marquer le récepteur R1, est le composé accepteur d'énergie A1, et **en ce que** le second membre de ce couple, lié au ligand, est le composé donneur D.

**4.** Méthode selon la revendication, 3 **caractérisée en ce que**

- l'étape (a) comprend également le marquage du récepteur R2 de manière covalente ou non-covalente par un second composé accepteur d'énergie A2, A1 et A2 ayant des longueurs d'onde d'émission différentes et (D,A2) formant un couple de partenaires de FRET, et
- l'étape (c) comprend également la mesure de la luminescence $L_2$ émise à la longueur d'onde d'émission du composé accepteur A2, en présence et en l'absence du composé à tester.

**5.** Méthode de détermination de la liaison d'un composé à tester avec des récepteurs R1 et R2 exprimés dans les membranes cellulaires, ces récepteurs étant connus pour être présents sous forme homodimérique mais pas hétérodimérique, **caractérisée en ce qu'**elle comprend les étapes suivantes :

(a) Marquage du récepteur R1 de manière covalente ou non-covalente par un premier composé donneur d'énergie D1 et du récepteur R2 de manière covalente ou non-covalente par un second composé donneur d'énergie D2 ;
(b) Ajout au milieu de mesure d'un ligand connu capable de se lier aux récepteurs R1, R2 et aux homodimères R1R1 et R2R2, ce ligand étant choisi parmi : un composé agoniste connu de R1 et R2, un composé antagoniste connu de R1 et R2 ou un modulateur allostérique connu de R1 et R2, et ce ligand étant marqué par un composé accepteur d'énergie A, les couples (D1,A) et (D2,A) formant chacun des couples de partenaires de FRET ;
(c) Mesure de la luminescence totale $L_{tot}$ émise à la longueur d'onde d'émission du composé accepteur A ;
(d) Ajout d'un composé extincteur du signal de FRET du couple (D1,A) ;
(e) Mesure de la luminescence $L_2$ émise à la longueur d'onde d'émission du composé accepteur A ;
(f) Détermination de la luminescence $L_1$ due au couple (D1,A) par la formule $L_1=L_{tot}-L_2$,

la séquence des étapes (c) à (f) étant mise en oeuvre en présence et en l'absence du composé à tester.

**6.** Méthode selon la revendication 5, **caractérisée en ce que** le composé donneur d'énergie D1 est un complexe de lanthanide et le composé extincteur du FRET du couple (D1,A) est un composé comportant un domaine de liaison avec D1.

**7.** Méthode selon la revendication 6, **caractérisée en ce que** D1 est un cryptate de lanthanide et **en ce que** le composé extincteur du FRET du couple (D1,A) est un anticorps spécifique de D1.

**8.** Méthode de détermination de la liaison d'un composé à tester avec des récepteurs membranaires R1 et R2 exprimés dans les membranes cellulaires, ces récepteurs étant connus pour être présents sous forme homodimérique mais pas hétérodimérique, **caractérisée en ce qu'**elle comprend les étapes suivantes :

(a) Marquage du récepteur R1 de manière covalente ou non-covalente par un premier composé accepteur d'énergie A1 et du récepteur R2 de manière covalente ou non-covalente par un second composé accepteur d'énergie A2, A1 et A2 ayant des longueurs d'onde d'émission différentes ;
(b) Ajout au milieu de mesure d'un ligand connu capable de se lier aux récepteurs R1, R2 et aux homodimères R1R1 et R2R2, ce ligand étant choisi parmi : un composé agoniste connu de R1 et R2, un composé antagoniste connu de R1 et R2 ou un modulateur allostérique connu de R1 et R2, et ce ligand étant marqué par un composé donneur d'énergie D, et les couples (D,A1) et (D,A2) formant chacun des couples de partenaires de FRET ;
(c) Mesure de la luminescence $L_1$ émise à la longueur d'onde d'émission du composé accepteur A1 d'une part, et de la luminescence $L_2$ émise à la longueur d'onde d'émission du composé accepteur A2 d'autre part, chacune

en présence et en l'absence du composé à tester.

**9.** Méthode selon l'une quelconque des revendications 1 à 8, **caractérisée en ce que** R1 et R2 sont des récepteurs couplés aux protéines G.

**10.** Méthode selon l'une quelconque des revendications 1 à 9, **caractérisée en ce que** les composés donneurs d'énergie sont des complexes de lanthanides fluorescents.

**11.** Méthode selon la revendication 10, **caractérisée en ce que** les complexes de lanthanides sont des complexes de terbium fluorescents ou des complexes d'europium fluorescents.

**12.** Méthode selon l'une quelconque des revendications 1 à 11, **caractérisée en ce que** le marquage des récepteurs R1 et R2 par les membres d'un couple de partenaires de FRET est un marquage direct par liaison covalente.

**13.** Méthode selon la revendication 12, **caractérisée en ce que** les récepteurs R1 et R2 sont chacun exprimés sous forme d'une protéine de fusion avec une enzyme suicide, et **en ce que** leurs marquages sont effectués par addition dans le milieu de mesure des membres des couples de partenaires de FRET, l'un et l'autre étant chacun lié de manière covalente au substrat de ladite enzyme suicide.

**14.** Méthode selon la revendication 13, **caractérisée en ce que** les récepteurs R1 et R2 sont chacun exprimés sous forme d'une protéine de fusion avec une enzyme suicide différente.

**15.** Méthode selon la revendication 13, caractérisée en ce les récepteurs R1 et R2 sont chacun exprimés sous forme d'une protéine de fusion avec la même enzyme suicide.

**16.** Méthode selon l'une quelconque des revendications 1 à 15, **caractérisée en ce qu'**elle comprend une étape préliminaire de transfection des cellules par un vecteur d'expression comprenant la séquence d'ADN codant pour le récepteur R1 d'une part, et par un vecteur d'expression comprenant la séquence d'ADN codant pour le récepteur R2 d'autre part.

**17.** Méthode selon la revendication 16, **caractérisée en ce que** les vecteur d'expression comprennent chacun la séquence d'ADN codant pour une protéine de fusion dont la partie N-terminale comprend une enzyme suicide et la partie C-terminale comprend le récepteur R1 ou R2.

**18.** Méthode selon l'une quelconque des revendications 1 à 17, **caractérisée en ce qu'**elle comprend en outre une étape de comparaison des valeurs de luminescence L1 et/ou L2 mesurées en l'absence et en présence du composé à tester.

## Patentansprüche

**1.** Verfahren zur Bestimmung der Bindung einer zu testenden Verbindung mit Membranrezeptoren R1 und R2, die in den Zellmembranen exprimiert sind, wobei diese Rezeptoren dafür bekannt sind, in heterodimerer Form vorzuliegen, **dadurch gekennzeichnet, dass** es die folgenden Schritte umfasst:

(a) Markieren des Rezeptors R1 auf kovalente oder nicht kovalente Weise mit dem ersten Glied eines Paares von FRET-Partnern (D, A1), wobei D die Donor-Verbindung und A1 die Energie-Akzeptor-Verbindung ist,
(b) Zugeben, zu dem Messmedium, eines bekannten Liganden von R2, der geeignet ist, an das Heterodimer R1 R2, aber weder an den Rezeptor R1 noch an das Homodimer R1 R1 zu binden, wobei der Ligand mit dem zweiten Glied des Paares von FRET-Partnern markiert ist und wobei der Ligand ausgewählt ist aus: einer bekannten Agonist-Verbindung von R2, einer bekannten Antagonist-Verbindung von R2 oder einem bekannten allosterischen Modulator von R2,
(c) Messen der Lumineszenz $L_1$, die bei der Emissionswellenlänge der Akzeptor-Verbindung $A_1$ emittiert wird, in Gegenwart und in Abwesenheit der zu testenden Verbindung.

**2.** Verfahren nach Anspruch 1, **dadurch gekennzeichnet, dass** das erste Glied des Paares von FRET-Partnern, das zum Markieren des Rezeptors R1 verwendet wird, die Energie-Donor-Verbindung D ist und dass das zweite Glied dieses Paares, das an den Liganden gebunden ist, die Akzeptor-Verbindung $A_1$ ist.

3. Verfahren nach Anspruch 1, **dadurch gekennzeichnet, dass** das erste Glied des Paares von FRET-Partnern, das zum Markieren des Rezeptors R1 verwendet wird, die Energie-Akzeptor-Verbindung $A_1$ ist und dass das zweite Glied dieses Paares, das an den Liganden gebunden ist, die Donor-Verbindung D ist.

4. Verfahren nach Anspruch 3, **dadurch gekennzeichnet, dass**

- der Schritt (a) auch die Markierung des Rezeptors R2 auf kovalente oder nicht kovalente Weise mit einer zweiten Energie-Akzeptor-Verbindung A2 umfasst, wobei A1 und A2 unterschiedliche Emissionswellenlängen aufweisen, und wobei (D, A2) ein Paar von FRET-Partnern bilden, und
- der Schritt (c) auch das Messen der Lumineszenz $L_2$, die bei der Emissionswellenlänge der Akzeptor-Verbindung $A_2$ emittiert wird, in Gegenwart und in Abwesenheit der zu testenden Verbindung umfasst.

5. Verfahren zur Bestimmung der Bindung einer zu testenden Verbindung mit Rezeptoren R1 und R2, die in den Zellmembranen exprimiert sind, wobei diese Rezeptoren dafür bekannt sind, in homodimerer, aber nicht heterodimerer Form vorzuliegen, **dadurch gekennzeichnet, dass** es die folgenden Schritte umfasst:

(a) Markieren des Rezeptors R1 auf kovalente oder nicht kovalente Weise mit einer ersten Energie-Donor-Verbindung D1 und des Rezeptors R2 auf kovalente oder nicht kovalente Weise mit einer zweiten Energie-Donor-Verbindung D2,
(b) Zugeben, zu dem Messmedium, eines bekannten Liganden, der geeignet ist, an die Rezeptoren R1, R2 und an die Homodimere R1 R1 und R2R2 zu binden, wobei dieser Ligand ausgewählt ist aus: einer bekannten Agonist-Verbindung von R1 und R2, einer bekannten Antagonist-Verbindung von R1 und R2 oder einem bekannten allosterischen Modulator von R1 und R2, und wobei dieser Ligand mit einer Energie-Akzeptor-Verbindung A markiert ist, wobei die Paare (D1, A) und (D2, A) jeweils Paare von FRET-Partnern bilden,
(c) Messen der Gesamtlumineszenz $L_{tot}$, die bei der Emissionswellenlänge der Akzeptor-Verbindung A emittiert wird,
(d) Zugeben einer Quencher-Verbindung des FRET-Signals des Paares (D1, A),
(e) Messen der Lumineszenz $L_2$, die bei der Emissionswellenlänge der Akzeptor-Verbindung A emittiert wird,
(f) Bestimmen der durch das Paar (D1, A) bedingten Lumineszenz $L_1$ mittels der Formel

$$\text{Formel } L_1 = L_{tot} - L_2,$$

wobei die Folge der Schritte (c) bis (f) in Gegenwart und in Abwesenheit der zu testenden Verbindung durchgeführt wird.

6. Verfahren nach Anspruch 5, **dadurch gekennzeichnet, dass** die Energie-Donor-Verbindung D1 ein Lanthanoid-Komplex ist und die Quencher-Verbindung des FRET des Paares (D1, A) eine Verbindung, die eine Bindungsdomäne mit D1 umfasst, ist.

7. Verfahren nach Anspruch 6, **dadurch gekennzeichnet, dass** D1 ein Lanthanoid-Kryptat ist und dass die Quencher-Verbindung des FRET des Paares (D1, A) ein für D1 spezifischer Antikörper ist.

8. Verfahren zur Bestimmung der Bindung einer zu testenden Verbindung mit Membranrezeptoren R1 und R2, die in den Zellmembranen exprimiert sind, wobei diese Rezeptoren dafür bekannt sind, in homodimerer, aber nicht heterodimerer Form vorzuliegen, **dadurch gekennzeichnet, dass** es die folgenden Schritte umfasst:

(a) Markieren des Rezeptors R1 auf kovalente oder nicht kovalente Weise mit einer ersten Energie-Akzeptor-Verbindung A1 und des Rezeptors R2 auf kovalente oder nicht kovalente Weise mit einer zweiten Energie-Akzeptor-Verbindung A2, wobei A1 und A2 unterschiedliche Emissionswellenlängen aufweisen,
(b) Zugeben, zu dem Messmedium, eines bekannten Liganden, der geeignet ist, an die Rezeptoren R1, R2 und an die Homodimere R1 R1 und R2R2 zu binden, wobei dieser Ligand ausgewählt ist aus: einer bekannten Agonist-Verbindung von R1 und R2, einer bekannten Antagonist-Verbindung von R1 und R2 oder einem bekannten allosterischen Modulator von R1 und R2, und wobei dieser Ligand mit einer Energie-Donor-Verbindung D markiert ist und wobei die Paare (D, A1) und (D, A2) jeweils Paare von FRET-Partnern bilden,
(c) Messen einerseits der Lumineszenz $L_1$, die bei der Emissionswellenlänge der Akzeptor-Verbindung A1 emittiert wird, und andererseits der Lumineszenz $L_2$, die bei der Emissionswellenlänge der Akzeptor-Verbindung

A2 emittiert wird, jeweils in Gegenwart und in Abwesenheit der zu testenden Verbindung.

**9.** Verfahren nach einem der Ansprüche 1 bis 8, **dadurch gekennzeichnet, dass** R1 und R2 an die G-Proteine gekoppelte Rezeptoren sind.

**10.** Verfahren nach einem der Ansprüche 1 bis 9, **dadurch gekennzeichnet, dass** die Energie-Donor-Verbindungen Komplexe von fluoreszierenden Lanthanoiden sind.

**11.** Verfahren nach Anspruch 10, **dadurch gekennzeichnet, dass** die Lanthanoid-Komplexe fluoreszierende Terbium-Komplexe oder fluoreszierende Europium-Komplexe sind.

**12.** Verfahren nach einem der Ansprüche 1 bis 11, **dadurch gekennzeichnet, dass** die Markierung der Rezeptoren R1 und R2 mit den Gliedern eines Paares von FRET-Partnern eine direkte Markierung durch kovalente Bindung ist.

**13.** Verfahren nach Anspruch 12, **dadurch gekennzeichnet, dass** die Rezeptoren R1 und R2 jeweils in Form eines Fusionsproteins mit einem Suizidenzym exprimiert sind und dass ihre Markierungen durch Zugabe der Glieder der Paare von FRET-Partnern zu dem Messmedium vollzogen werden, wobei der eine und der andere jeweils an das Substrat des Suizidenzyms kovalent gebunden ist.

**14.** Verfahren nach Anspruch 13, **dadurch gekennzeichnet, dass** die Rezeptoren R1 und R2 jeweils in Form eines Fusionsproteins mit einem unterschiedlichen Suizidenzym exprimiert sind.

**15.** Verfahren nach Anspruch 13, **dadurch gekennzeichnet, dass** die Rezeptoren R1 und R2 jeweils in Form eines Fusionsproteins mit dem gleichen Suizidenzym exprimiert sind.

**16.** Verfahren nach einem der Ansprüche 1 bis 15, **dadurch gekennzeichnet, dass** es einen vorbereitenden Schritt einer Transfektion der Zellen einerseits mit einem Expressionsvektor, der die für den Rezeptor R1 kodierende DNA-Sequenz enthält, und andererseits mit einem Expressionsvektor, der die für den Rezeptor R2 kodierende DNA-Sequenz enthält, umfasst.

**17.** Verfahren nach Anspruch 16, **dadurch gekennzeichnet, dass** die Expressionsvektoren jeweils die DNA-Sequenz umfassen, die für ein Fusionsprotein kodiert, dessen N-terminaler Teil ein Suizidenzym enthält und dessen C-terminaler Teil den Rezeptor R1 oder R2 enthält.

**18.** Verfahren nach einem der Ansprüche 1 bis 17, **dadurch gekennzeichnet, dass** es ferner einen Schritt des Vergleichens der in Abwesenheit und in Gegenwart der zu testenden Verbindung gemessenen Werte der Lumineszenz L1 und/oder L2 umfasst.

## Claims

**1.** A method for determining the binding of a test compound to membrane receptors R1 and R2 expressed in the cell membranes, these receptors being known to be present in heterodimeric form, **characterized in that** it comprises the following steps:

(a) labeling the receptor R1 covalently or noncovalently with the first member of a FRET partner pair (D,A1), in which D is the energy donor compound and A1 is the energy acceptor compound;
(b) adding, to the measuring medium, a known ligand of R2 capable of binding to the heterodimer R1R2 but neither to the receptor R1 nor to the homodimer R1R1, said ligand being labeled with the second member of said FRET partner pair, and said ligand being selected from: a known agonist compound of R2, a known antagonist compound of R2 or a known allosteric modulator of R2;
(c) measuring the luminescence $L_1$ emitted at the emission wavelength of the acceptor compound $A_1$, in the presence and in the absence of the test compound.

**2.** The method as claimed in claim 1, **characterized in that** the first member of said FRET partner pair, used for labeling the receptor R1, is the energy donor compound D, and **in that** the second member of this pair, bound to the ligand, is the acceptor compound A1.

3. The method as claimed in claim 1, **characterized in that** the first member of said FRET partner pair, used for labeling the receptor R1, is the energy acceptor compound A1, and **in that** the second member of this pair, bound to the ligand, is the donor compound D.

4. The method as claimed in claim 3, **characterized in that**

- step (a) also comprises labeling the receptor R2 covalently or noncovalently with a second energy acceptor compound A2, A1 and A2 having different emission wavelengths and (D,A2) forming a FRET partner pair, and
- step (c) also comprises measuring the luminescence $L_2$ emitted at the emission wavelength of the acceptor compound A2, in the presence and in the absence of the test compound.

5. A method for determining the binding of a test compound with receptors R1 and R2 expressed in the cell membranes, these receptors being known to be present in homodimeric but not heterodimeric form, **characterized in that** it comprises the following steps:

(a) labeling the receptor R1 covalently or noncovalently with a first energy donor compound D1 and receptor R2 covalently or noncovalently with a second energy donor compound D2;
(b) adding, to the measuring medium, a ligand known to be capable of binding to the receptors R1, R2 and to the homodimers R1R1 and R2R2, this ligand being selected from: a known agonist compound of R1 and R2, a known antagonist compound of R1 and R2 or a known allosteric modulator of R1 and R2, and this ligand being labeled with an energy acceptor compound A, the pairs (D1,A) and (D2,A) each forming FRET partner pairs;
(c) measuring the total luminescence $L_{tot}$ emitted at the emission wavelength of the acceptor compound A;
(d) adding a compound that extinguishes the FRET signal of the pair (D1,A);
(e) measuring the luminescence $L_2$ emitted at the emission wavelength of the acceptor compound A;
(f) determining the luminescence $L_1$ due to the pair (D1,A) from the formula $L_1=L_{tot}-L_2$,

the sequence of steps (c) to (f) being applied in the presence and in the absence of the test compound.

6. The method as claimed in claim 5, **characterized in that** the energy donor compound D1 is a lanthanide complex and the FRET extinguishing compound of the pair (D1,A) is a compound having a binding domain with D1.

7. The method as claimed in claim 6, **characterized in that** D1 is a lanthanide cryptate and **in that** the FRET extinguishing compound of the pair (D1,A) is a specific antibody of D1.

8. A method for determining the binding of a test compound to membrane receptors R1 and R2 expressed in the cell membranes, these receptors being known to be present in homodimeric but not heterodimeric form, **characterized in that** it comprises the following steps:

(a) labeling the receptor R1 covalently or noncovalently with a first energy acceptor compound A1 and receptor R2 covalently or noncovalently with a second energy acceptor compound A2, A1 and A2 having different emission wavelengths;
(b) adding, to the measuring medium, a ligand known to be capable of binding to the receptors R1, R2 and to the homodimers R1R1 and R2R2, this ligand being selected from: a known agonist compound of R1 and R2, a known antagonist compound of R1 and R2 or a known allosteric modulator of R1 and R2, and this ligand being labeled with an energy donor compound D, and the pairs (D,A1) and (D,A2) each forming FRET partner pairs;
(c) measuring the luminescence $L_1$ emitted at the emission wavelength of the acceptor compound A1 on the one hand, and the luminescence $L_2$ emitted at the emission wavelength of the acceptor compound A2 on the other hand, each in the presence and in the absence of the test compound.

9. The method as claimed in any one of claims 1 to 8, **characterized in that** R1 and R2 are G protein-coupled receptors.

10. The method as claimed in any one of claims 1 to 9, **characterized in that** the energy donor compounds are fluorescent complexes of lanthanides.

11. The method as claimed in claim 10, **characterized in that** the complexes of lanthanides are fluorescent complexes of terbium or fluorescent complexes of europium.

**12.** The method as claimed in any one of claims 1 to 11, **characterized in that** the labeling of the receptors R1 and R2 with the members of a FRET partner pair is a direct labeling by covalent binding.

**13.** The method as claimed in claim 12, **characterized in that** the receptors R1 and R2 are each expressed in the form of a fusion protein with a suicide enzyme, and **in that** they are labeled by adding, to the measuring medium, members of FRET partner pairs, the one and the other each being bound covalently to the substrate of said suicide enzyme.

**14.** The method as claimed in claim 13, **characterized in that** the receptors R1 and R2 are each expressed in the form of a fusion protein with a different suicide enzyme.

**15.** The method as claimed in claim 13, **characterized in that** the receptors R1 and R2 are each expressed in the form of a fusion protein with the same suicide enzyme.

**16.** The method as claimed in any one of claims 1 to 15, **characterized in that** it comprises a preliminary step of transfection of the cells with an expression vector comprising the DNA sequence coding for the receptor R1 on the one hand, and with an expression vector comprising the DNA sequence coding for the receptor R2 on the other hand.

**17.** The method as claimed in claim 16, **characterized in that** the expression vectors each comprise the DNA sequence coding for a fusion protein whose N-terminal portion comprises a suicide enzyme and C-terminal portion comprises the receptor R1 or R2.

**18.** The method as claimed in any one of claims 1 to 17, **characterized in that** it further comprises a step of comparing the values of luminescence L1 and/or L2 measured in the absence and in the presence of the test compound.

FIG 1.

| Protéines dans la membrane cellulaire : monomères et homodimères | 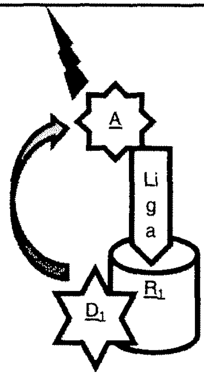 | 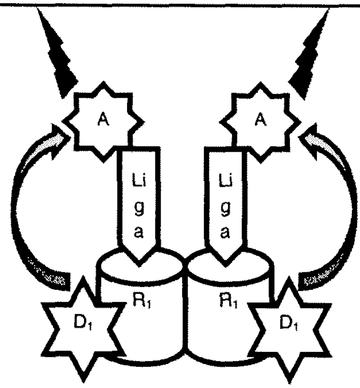 | 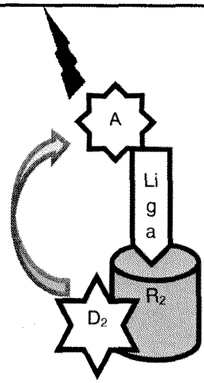 | 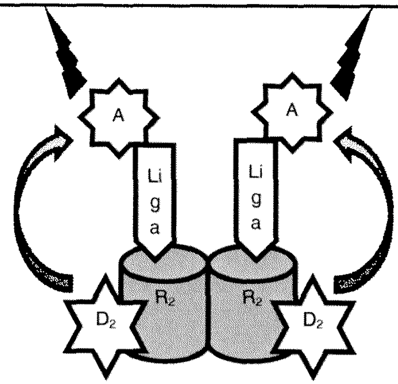 |
|---|---|---|---|---|
| *Sans composé à tester* | Signal $L_1$ | Signal $L_1$ | Signal $L_2$ | Signal $L_2$ |
| *+ composé se liant à $R_1$ et $R_1R_1$* | Signal $L_1$ supprimé | | Signal $L_2$ | Signal $L_2$ |
| *+ composé se liant à $R_2$ et $R_2R_2$* | Signal $L_1$ | Signal $L_1$ | Signal $L_2$ supprimé | |
| *+ composé se liant à la fois avec $R_1$, $R_1R_1$, $R_2$ et $R_2R_2$* | Signaux $L_1$ et $L_2$ supprimés | | | |

FIG.2

| Protéines dans la membrane cellulaire : monomères et homodimères | 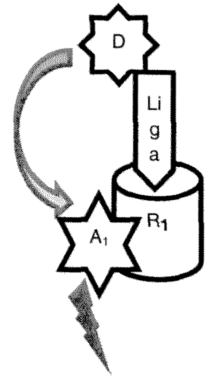 | 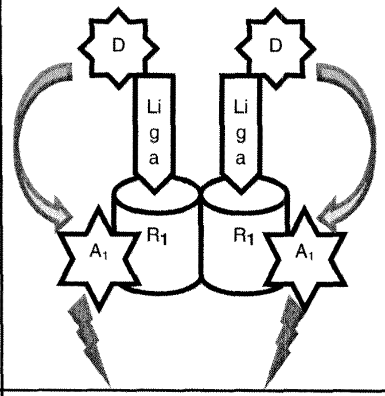 | 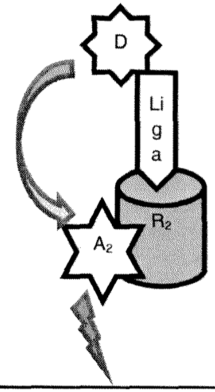 | 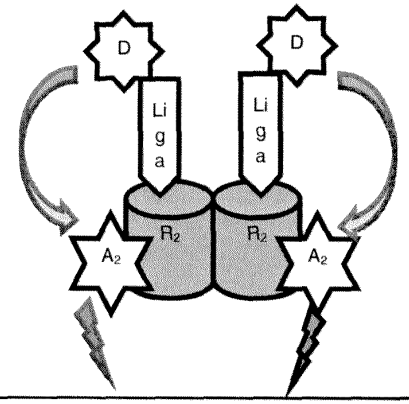 |
|---|---|---|---|---|
| *Sans composé à tester* | Signal $L_1$ | Signal $L_1$ | Signal $L_2$ | Signal $L_2$ |
| *+ composé se liant à $R_1$ et $R_1R_1$* | Signal $L_1$ supprimé | | Signal $L_2$ | Signal $L_2$ |
| *+ composé se liant à $R_2$ et $R_2R_2$* | Signal $L_1$ | Signal $L_1$ | Signal $L_2$ supprimé | |
| *+ composé se liant à la fois avec $R_1$, $R_1R_1$, $R_2$ et $R_2R_2$* | Signaux $L_1$ et $L_2$ supprimés | | | |

FIG.3

| | | | | | |
|---|---|---|---|---|---|
| *Protéines dans la membrane cellulaire : monomères, homodimères et hétérodimères* | 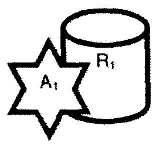 | 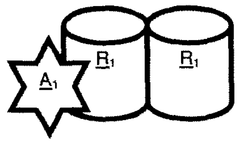 | 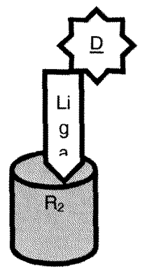 | 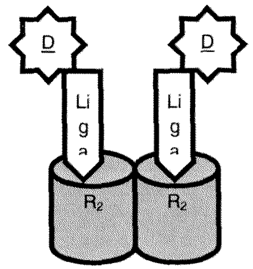 | 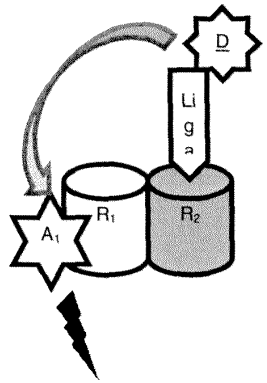 |
| *Sans composé à tester* | Pas de signal | Pas de signal | Pas de signal | Pas de signal | Signal $L_1$ |
| *+ composé se liant à $R_1R_2$* | Pas de signal | Pas de signal | Pas de signal | Pas de signal | Signal $L_1$ supprimé |

FIG.4

| *Protéines dans la membrane cellulaire : monomères, homodimères et hétérodimères* | 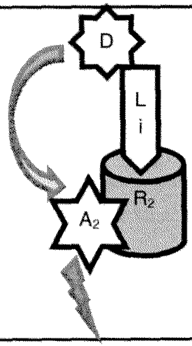 | 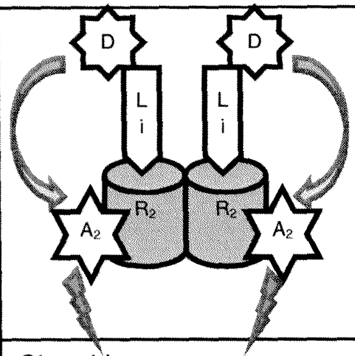 | 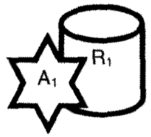 | 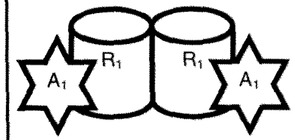 | 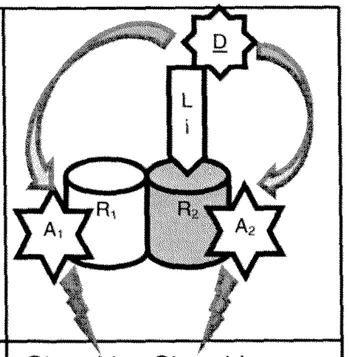 |
|---|---|---|---|---|---|
| *sans composé à tester* | Signal $L_2$ | Signal $L_2$ | Pas de signal | Pas de signal | Signal $L_1$+Signal $L_2$ |
| *+ composé se liant à $R_2$, $R_2R_2$, mais pas à $R_1R_2$* | Signal $L_2$ supprimé | | Pas de signal | Pas de signal | Signal $L_1$ +Signal $L_2$ |
| *+ composé se liant seulement à $R_1R_2$* | Signal $L_2$ | Signal $L_2$ | Pas de signal | Pas de signal | Signal $L_1$ et $L_2$ supprimés |
| *+ composé se liant à la fois à $R_2$, $R_2R_2$ et* | Signal $L_2$ supprimé | | Pas de signal | Pas de signal | Signal $L_1$ et $L_2$ supprimés |

FIG. 5

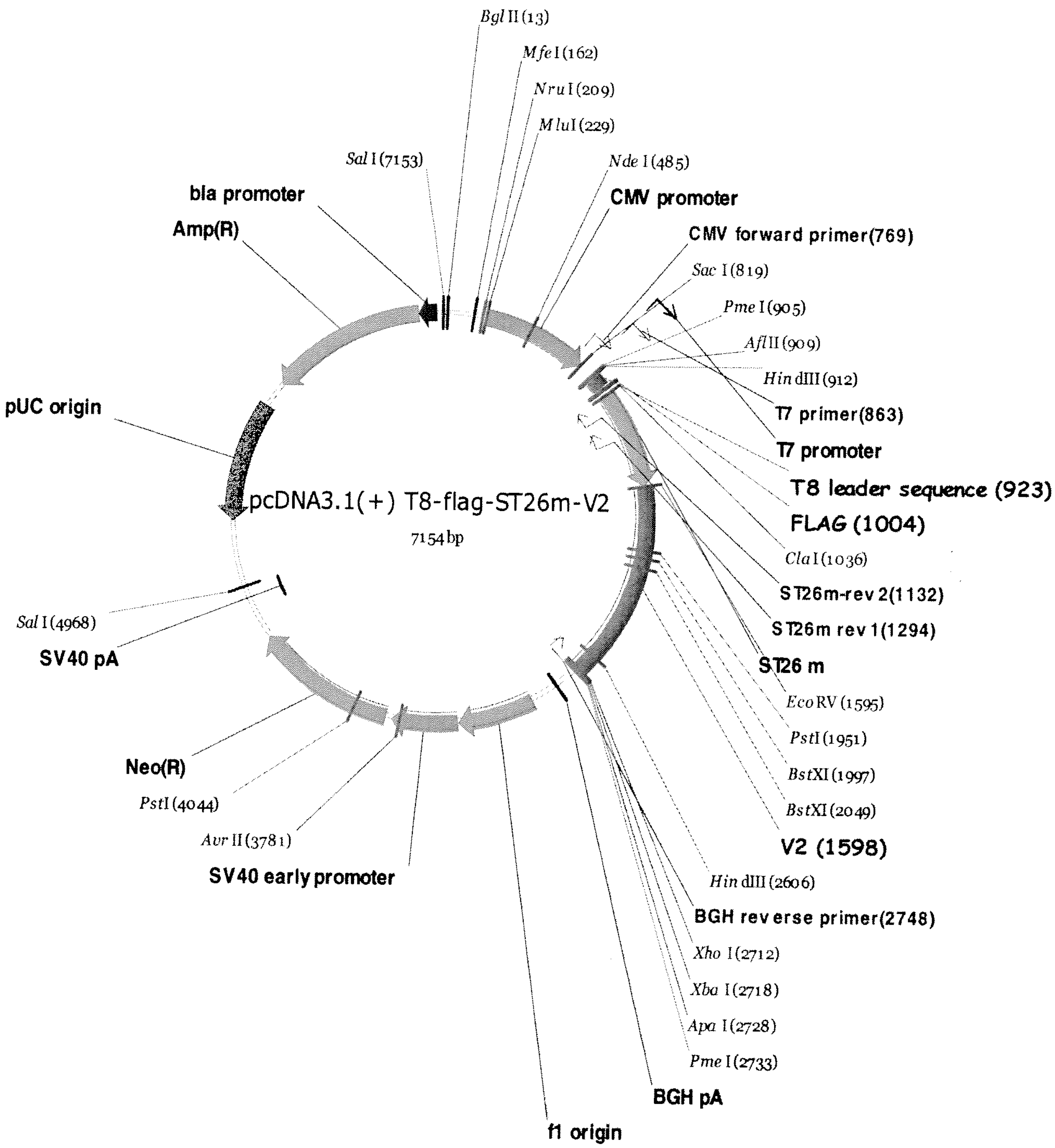
BglII(13)
MfeI(162)
NruI(209)
MluI(229)
SalI(7153)
NdeI(485)
bla promoter
CMV promoter
Amp(R)
CMV forward primer(769)
SacI(819)
PmeI(905)
AflII(909)
HindIII(912)
pUC origin
T7 primer(863)
T7 promoter
T8 leader sequence (923)
pcDNA3.1(+) T8-flag-ST26m-V2
7154bp
FLAG (1004)
ClaI(1036)
ST26m-rev 2(1132)
SalI(4968)
ST26m rev 1(1294)
SV40 pA
ST26 m
EcoRV(1595)
PstI(1951)
Neo(R)
BstXI(1997)
PstI(4044)
BstXI(2049)
AvrII(3781)
V2 (1598)
SV40 early promoter
HindIII(2606)
BGH reverse primer(2748)
XhoI(2712)
XbaI(2718)
ApaI(2728)
PmeI(2733)
BGH pA
f1 origin

FIG. 6

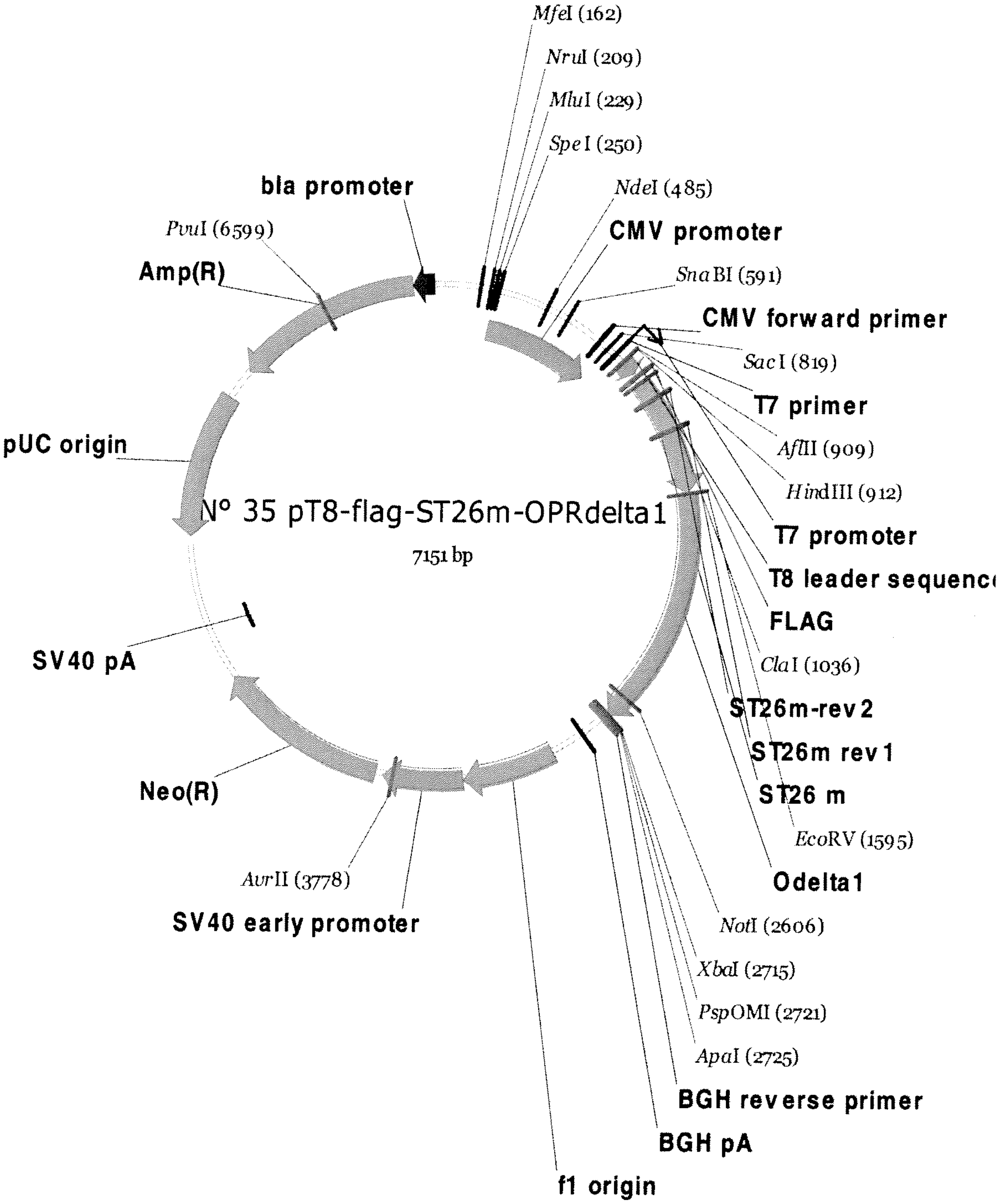
MfeI (162)
NruI (209)
MluI (229)
SpeI (250)
NdeI (485)
CMV promoter
SnaBI (591)
CMV forward primer
SacI (819)
T7 primer
AflII (909)
HindIII (912)
T7 promoter
T8 leader sequence
FLAG
ClaI (1036)
ST26m-rev2
ST26m rev1
ST26 m
EcoRV (1595)
Odelta1
NotI (2606)
XbaI (2715)
PspOMI (2721)
ApaI (2725)
BGH reverse primer
BGH pA
f1 origin
SV40 early promoter
AvrII (3778)
Neo(R)
SV40 pA
pUC origin
Amp(R)
PvuI (6599)
bla promoter
N° 35 pT8-flag-ST26m-OPRdelta1
7151 bp

FIG. 7

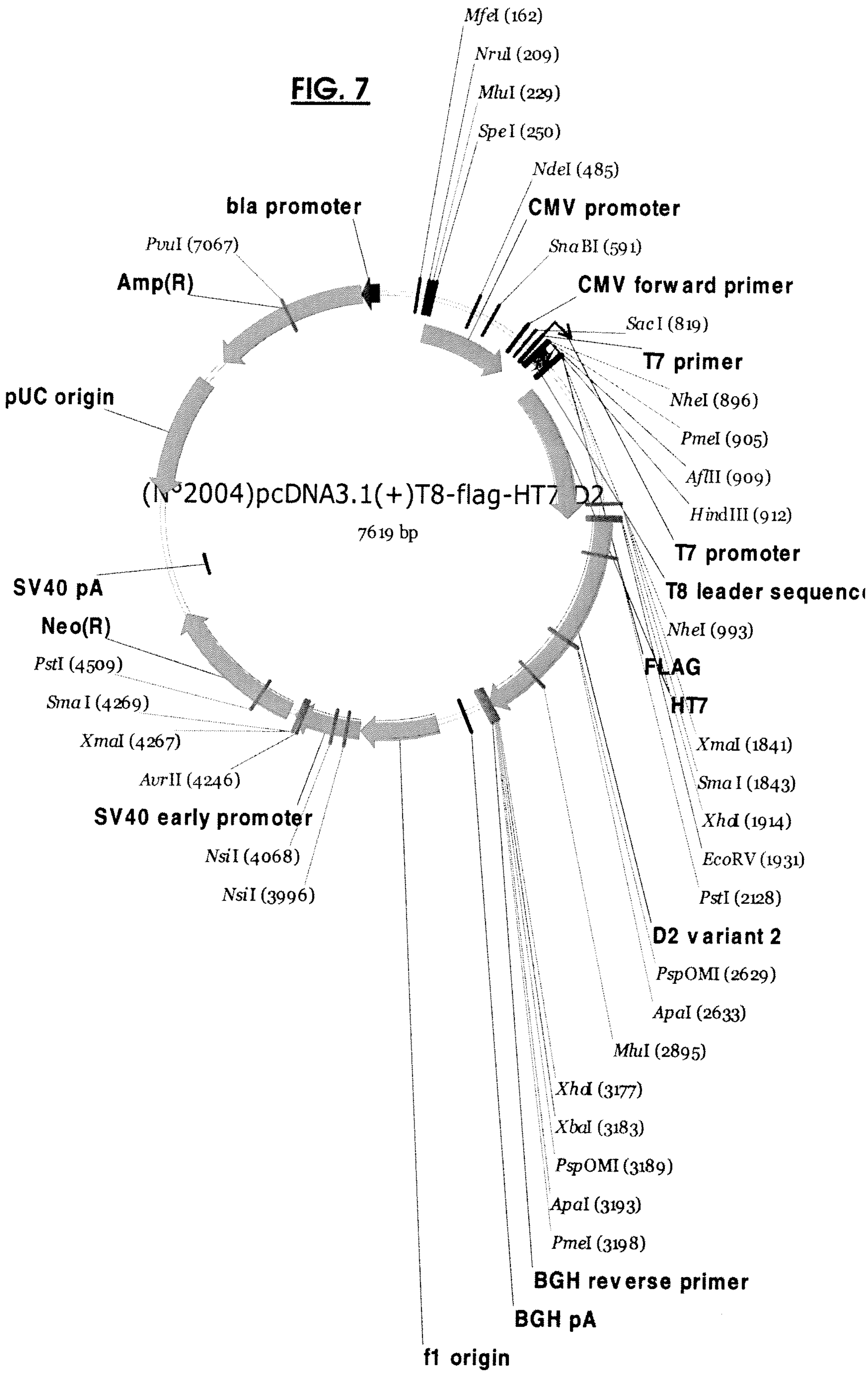
MfeI (162)
NruI (209)
MluI (229)
SpeI (250)
NdeI (485)
bla promoter
CMV promoter
PvuI (7067)
SnaBI (591)
Amp(R)
CMV forward primer
SacI (819)
T7 primer
pUC origin
NheI (896)
PmeI (905)
AflII (909)
(N°2004)pcDNA3.1(+)T8-flag-HT7_D2
7619 bp
HindIII (912)
T7 promoter
SV40 pA
T8 leader sequence
NheI (993)
Neo(R)
FLAG
PstI (4509)
HT7
SmaI (4269)
XmaI (4267)
XmaI (1841)
SmaI (1843)
AvrII (4246)
XhoI (1914)
SV40 early promoter
EcoRV (1931)
NsiI (4068)
NsiI (3996)
PstI (2128)
D2 variant 2
PspOMI (2629)
ApaI (2633)
MluI (2895)
XhoI (3177)
XbaI (3183)
PspOMI (3189)
ApaI (3193)
PmeI (3198)
BGH reverse primer
BGH pA
f1 origin

FIG. 8

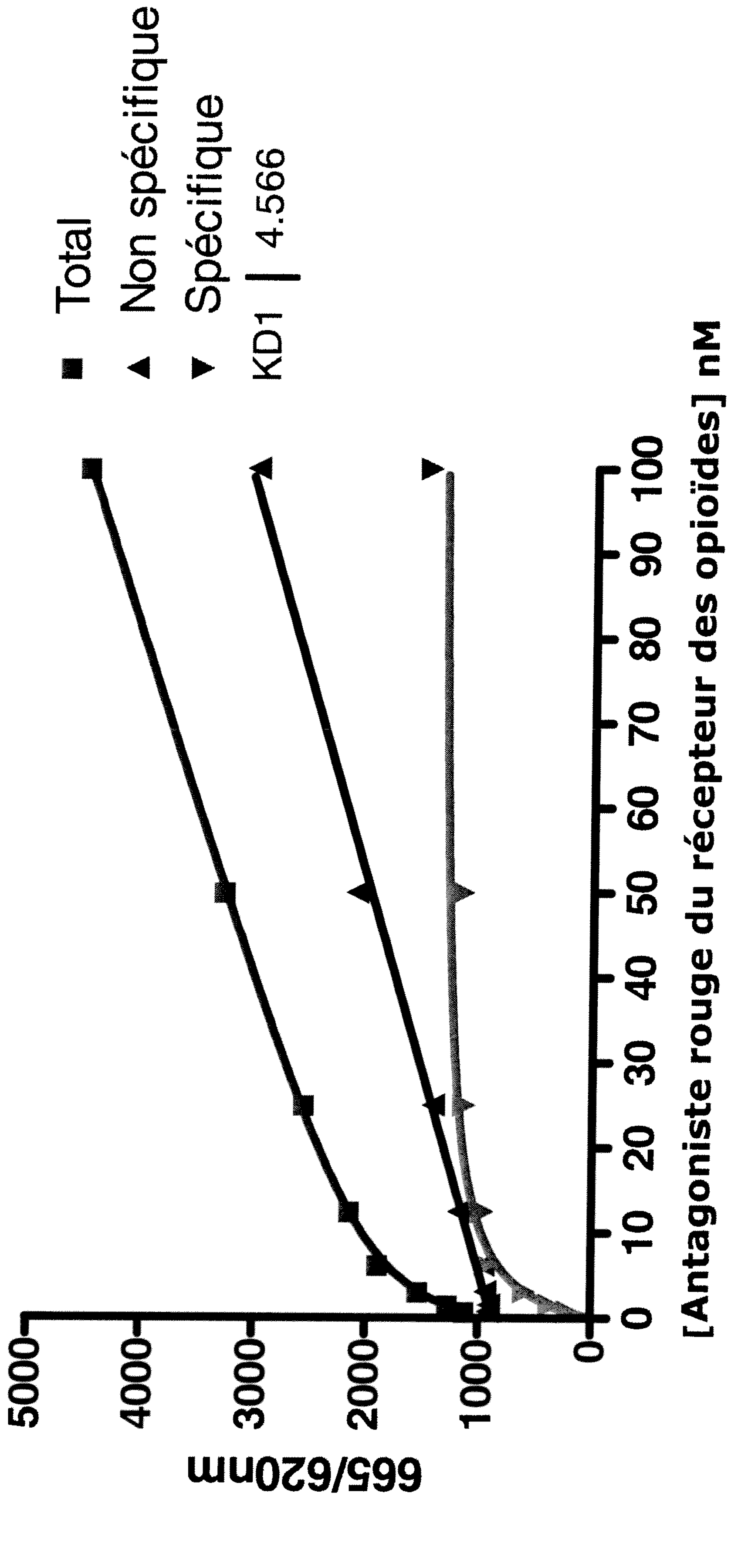

665/620nm
5000
4000
3000
2000
1000
0
0
10
20
30
40
50
60
70
80
90
100
[Antagoniste rouge du récepteur des opioïdes] nM
Total
Non spécifique
Spécifique
KD1 | 4.566

FIG. 9

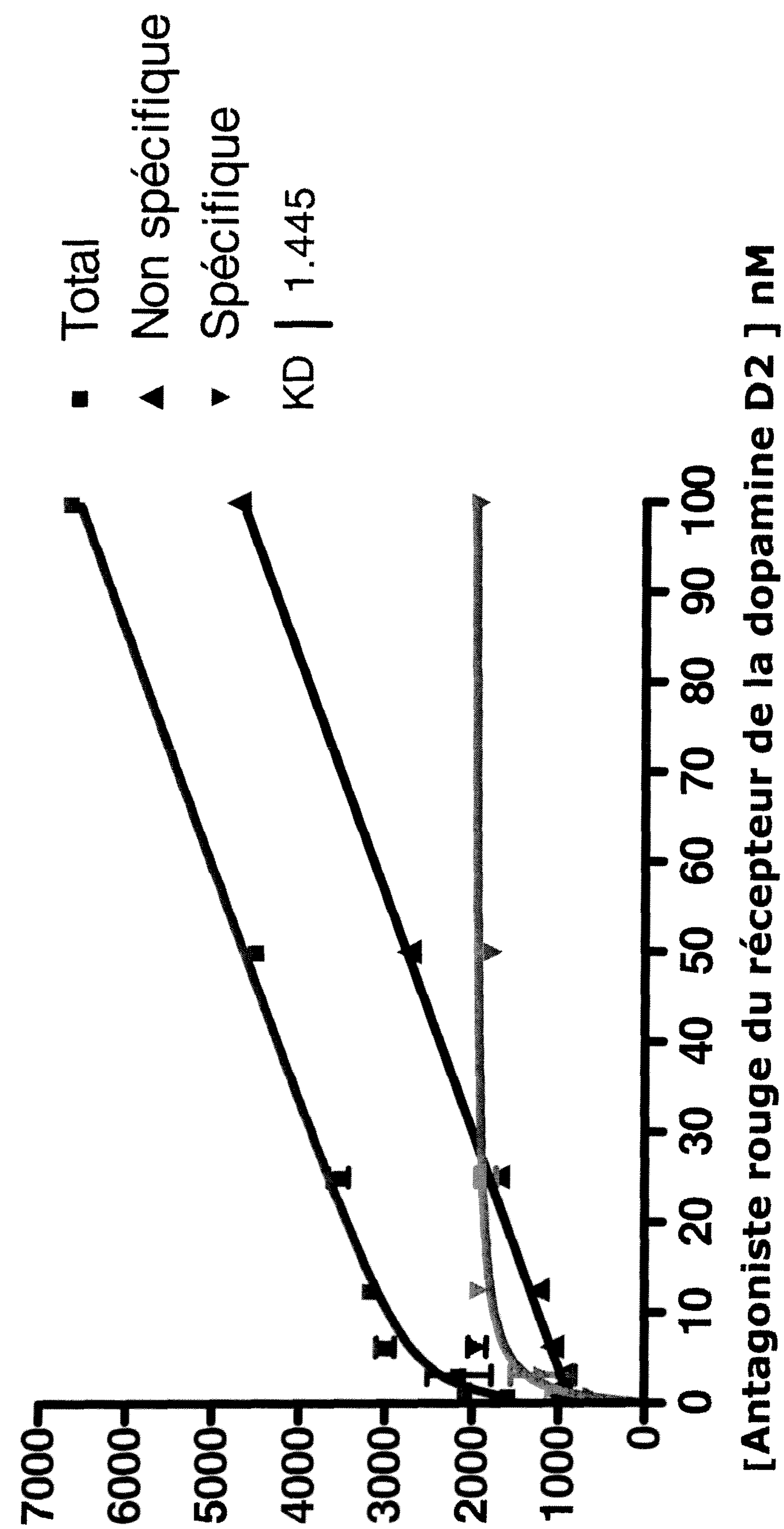
665/620nm
7000
6000
5000
4000
3000
2000
1000
0
0
10
20
30
40
50
60
70
80
90
100
[Antagoniste rouge du récepteur de la dopamine D2 ] nM
Total
Non spécifique
Spécifique
KD | 1.445

FIG. 10

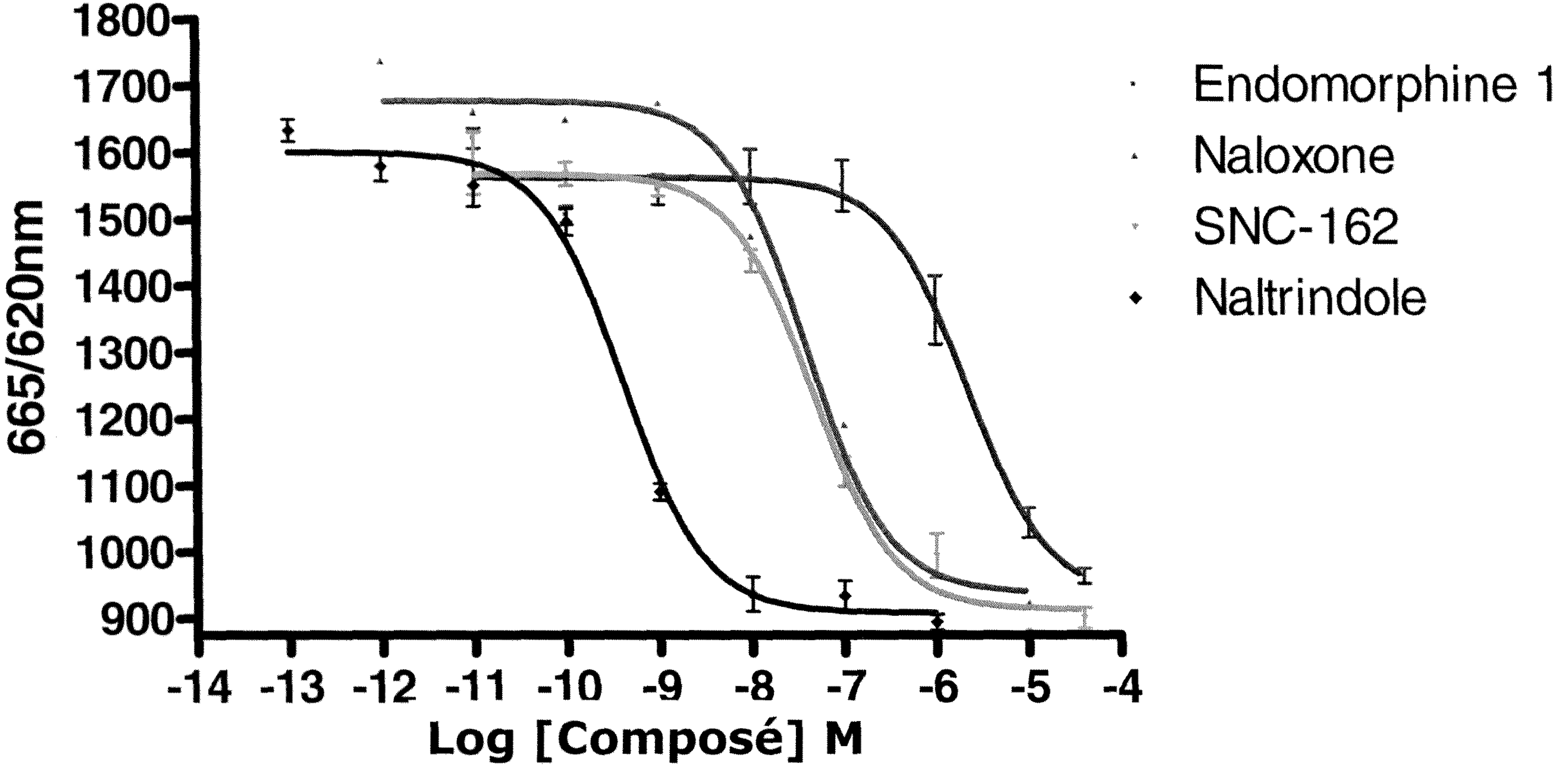


| | Endomorphine 1 | Naloxone | SNC-162 | Naltrindole |
|---|---|---|---|---|
| EC50 | 2.221e-006 | 3.982e-008 | 4.630e-008 | 4.112e-010 |
| KI | 1.088e-006 | 1.950e-008 | 2.268e-008 | 2.014e-010 |

FIG. 11

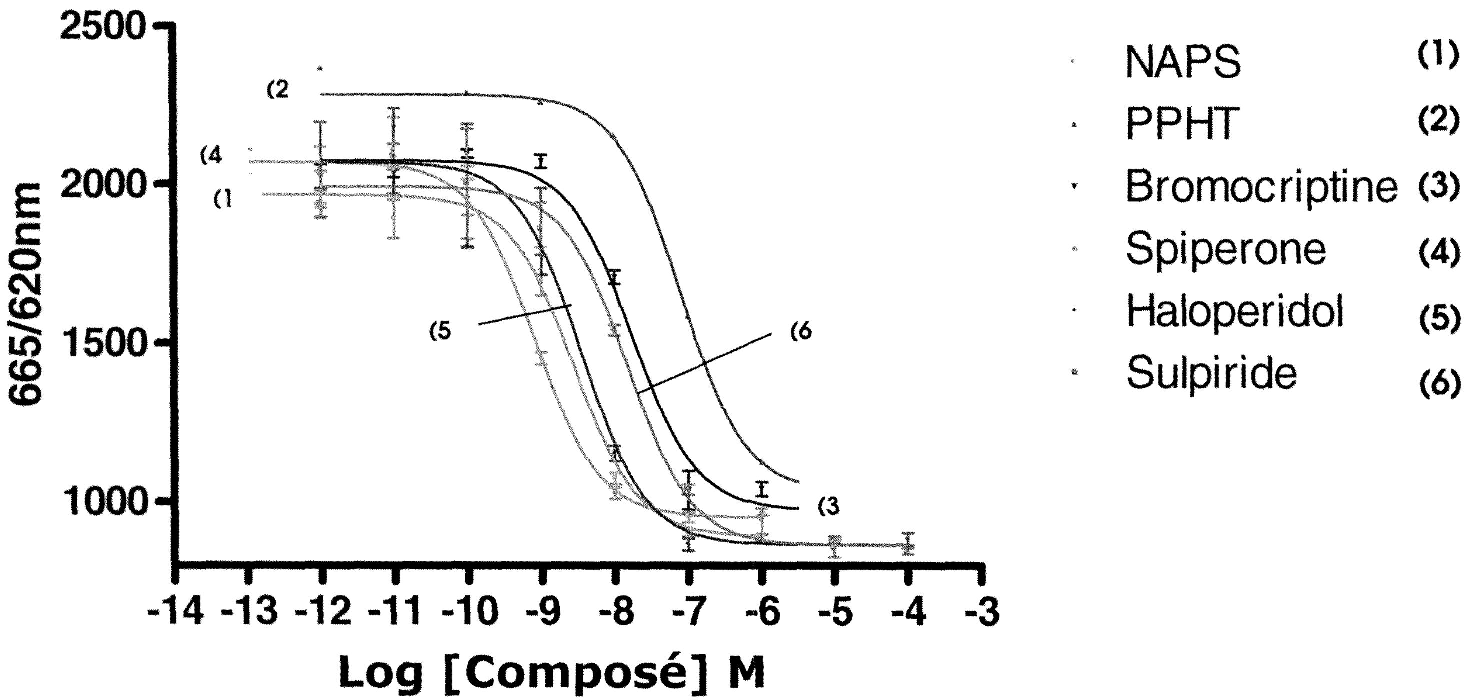


| | NAPS | PPHT | Bromocriptine | Spiperone | Haloperidol | Sulpiride |
|---|---|---|---|---|---|---|
| EC50 | 2.753e-009 | 8.035e-008 | 1.725e-008 | 8.198e-010 | 3.558e-009 | 1.500e-008 |
| KI | 8.930e-010 | 2.606e-008 | 5.594e-009 | 2.659e-010 | 1.154e-009 | 4.863e-009 |

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2007116069 A
- EP 0180492 A
- EP 0321353 A
- EP 0601113 A
- WO 0196877 A
- US 4761481 A
- US 5032677 A
- US 5055578 A
- US 5106957 A
- US 5116989 A
- US 4801722 A
- US 4794191 A
- US 4637988 A
- US 4670572 A
- US 4837169 A
- US 4859777 A
- EP 0403593 A
- US 5324825 A
- US 5202423 A
- US 5316909 A
- WO 200910580 A
- WO 2008063721 A
- US 5622821 A
- WO 2004072232 A
- WO 0012544 A
- WO 2004088312 A
- US 4767718 A


### Littérature non-brevet citée dans la description


- **S. R. GEORGE et al.** *Nature drug discovery,* 2002, vol. 1, 808-820
- **G. MILLIGAN.** *Biochimica et Biophysica Acta,* vol. 1768, 825-835
- **PRAMANIK, A. et al.** Fluorescence correlation spectroscopy detects galanin receptor diversity on insulinoma cells. *Biochemistry,* 2001, vol. 40 (36), 10839-10845
- **HANDL, H.L. et al.** Lanthanide-based time-resolved fluorescence of in cyto ligand-receptor interactions. *Analytical Biochemistry,* 2004, vol. 330 (2), 242-250
- Use of fluorescence polarization détection for the measurement of fluopeptide binding to G protein-coupled receptors. *Journal of Receptor and Signal Transduction Research,* 2002, vol. 22 (1-4), 333-343
- **ILIEN, B. et al.** Fluorescence resonance energy transfer to probe human M1 muscarinic receptor structure and drug binding properties. *Journal of Neurochemistry,* 2003, vol. 85, 768-778
- **POOLE R. et al.** Synthesis and characterisation of highly emissive and kinetically stable lanthanide complexes suitable for usage in celluloll. *Biomol. Chem,* 2005, vol. 3, 1013-1024
- **B.A.GRIFFIN et al.** *Science,* 1998, vol. 281, 269-271
- **S.A. ADAMS et al.** *J. Am. Chem. Soc.,* 2002, vol. 124, 6063-6076
- **M. MCCANN et al.** *Biotechnique,* 2005, vol. 38, 945-952
- **C. M. MCCANN et al.** *Biotechnique,* 2005, vol. 38, 945-952
- **JUILLERAT et al.** *Chemistry & biology,* Avril 2003, vol. 10, 313-317
- **GAUTIER et al.** *Chemistry et Biology,* Février 2008, vol. 15, 128-136
- **GRONEMEYER et al.** *Protein engineering, design & selection,* 2006, vol. 19 (7), 309-3016
- **N.GEORGE et al.** *Journal of the American Chemical society,* 2004, vol. 126, 8896-8897
- *Nature Methods,* 2008
- **OKUNO, Y. et al.** GLIDA: GPCR ligand database for chemical genomics drug discovery database and tools update. *Nucl. Acids Res.,* 2008, vol. 36 (1), D907-912
- **C. KESSLER.** Nonisotopic probing, Blotting and Sequencing. Academic press Ltd, 1995, 66-72
- **MIDDLETON, R.J. ; KELLAM, B.** Fluorophore-tagged GPCR ligands. *Current Opinion in Chemical Biology,* 2005, vol. 9 (5), 517-525
- **BEAUDET, A. et al.** Fluorescent ligands for studying neuropeptide receptors by confocal microscopy. *Brazilian Journal of Médical and Biological Research,* 1998, vol. 31 (11), 1479-1489
- **THIERRY DURROUX et al.** Fluorescent Pseudo-Peptide Linear Vasopressine Antagonists: Design, Synthesis, and Applications. *Journal of Médicinal Chemistry,* 01 Avril 1999, vol. 42 (7), 1312-1319

- **L HEIN et al.** Intracellular trafficking of angiotensine II and its AT1 and AT2 receptors: evidence for selective sorting of receptor and ligand. *Molecular Endocrinology,* Août 1997, vol. 11 (9), 1266-1277
- **HEATHER L HANDL et al.** Lanthanide-based time-resolved fluorescence of in cyto ligand-receptor interactions. *Analytical Biochemistry,* 15 Juillet 2004, vol. 330 (2), 242-250
- Fluorescent probes for dopamine receptors: synthesis and characterization of fluorescein and 7-nitrobenz-2-oxa-1,3-diazol-4-yl conjugates of D-1 and D-2 receptor ligands. *Journal of Medicinal Chemistry,* 1991, vol. 34 (11), 3235-3241
- **MANNING, M. et al.** Potent V2/V1a vasopressin antagonists with C-terminal ethylenediamine-linked retro-amino acids. *Journal of Medicinal Chemistry,* 1992, vol. 35 (21), 3895-3904
- **HARMAR AJ et al.** IUPHAR-DB: the IUPHAR database of G protein-coupled receptors and ion channels. *Nucl. Acids Res.,* 2009, vol. 37, D680-D685

SEQUENCE LISTING

<110> CIS BIO INTERNATIONAL

<120> Méthode de détermination de la liaison d'un composé donné à un récepteur membranaire

<130> 1H189130 0034 WO

<160> 6

<170> PatentIn version 3.3

<210> 1
<211> 6
<212> PRT
<213> séquence artificielle

<220>
<221> MISC_FEATURE
<222> (3)..(4)
<223> Xaa est un acide aminé quelconque

<400> 1

```
Cys Cys Xaa Xaa Cys Cys
1               5
```

<210> 2
<211> 8
<212> PRT
<213> séquence artificielle

<400> 2

```
Asp Tyr Lys Asp Asp Asp Asp Lys
1               5
```

<210> 3
<211> 9
<212> PRT
<213> séquence artificielle

<400> 3

```
Tyr Pro Tyr Asp Val Pro Phe Tyr Ala
1               5
```

<210> 4
<211> 7154
<212> DNA
<213> séquence artificielle

<220>
<221> misc_feature
<222> (232)..(819)
<223> promoteur CMV

<220>
<221> misc_feature
<222> (923)..(986)
<223> séquence leader T8

<220>
<221> misc_feature
<222> (1004)..(1027)
<223> QC tag

<220>
<221> misc_feature
<222> (1046)..(1591)
<223> Snap tag

<220>
<221> misc_feature
<222> (1598)..(2710)
<223> gène d’intérêt

<220>
<221> misc_feature
<222> (2754)..(2978)
<223> BGH polyA

<220>
<221> misc_feature
<222> (3862)..(4656)
<223> Neo R

<220>
<221> misc_feature
<222> (6158)..(7018)
<223> Ampi R

<400> 4

```
gacggatcgg gagatctccc gatcccctat ggtgcactct cagtacaatc tgctctgatg      60
ccgcatagtt aagccagtat ctgctccctg cttgtgtgtt ggaggtcgct gagtagtgcg     120
cgagcaaaat ttaagctaca acaaggcaag gcttgaccga caattgcatg aagaatctgc     180
ttagggttag gcgttttgcg ctgcttcgcg atgtacgggc cagatatacg cgttgacatt     240
gattattgac tagttattaa tagtaatcaa ttacggggtc attagttcat agcccatata     300
tggagttccg cgttacataa cttacggtaa atggcccgcc tggctgaccg cccaacgacc     360
cccgcccatt gacgtcaata atgacgtatg ttcccatagt aacgccaata gggactttcc     420
attgacgtca atgggtggag tatttacggt aaactgccca cttggcagta catcaagtgt     480
atcatatgcc aagtacgccc cctattgacg tcaatgacgg taaatggccc gcctggcatt     540
atgcccagta catgacctta tgggactttc ctacttggca gtacatctac gtattagtca     600
tcgctattac catggtgatg cggttttggc agtacatcaa tgggcgtgga tagcggtttg     660
actcacgggg atttccaagt ctccacccca ttgacgtcaa tgggagtttg ttttggcacc     720
aaaatcaacg ggactttcca aaatgtcgta acaactccgc cccattgacg caaatgggcg     780
```

gtaggcgtgt acggtgggag gtctatataa gcagagctct ctggctaact agagaaccca 840
ctgcttactg gcttatcgaa attaatacga ctcactatag ggagacccaa gctggctagc 900
gtttaaactt aagctttgag acatggcctt accagtgacc gccttgctcc tgccgctggc 960
cttgctgctc cacgccgcca ggccggccgc cgctagcggc atcgactaca aggacgacga 1020
tgacaaggcc ggcatcgatg ccatcatgga caaagactgc gaaatgaagc gcaccaccct 1080
ggatagccct ctgggcaagc tggaactgtc tgggtgcgaa cagggcctgc acgagatcaa 1140
gctgctgggc aaaggaacat ctgccgccga cgccgtggaa gtgcctgccc cagccgccgt 1200
gctgggcgga ccagagccac tgatgcaggc caccgcctgg ctcaacgcct actttcacca 1260
gcctgaggcc atcgaggagt tccctgtgcc agccctgcac cacccagtgt tccagcagga 1320
gagctttacc cgccaggtgc tgtggaaact gctgaaagtg gtgaagttcg gagaggtcat 1380
cagctaccag cagctggccg ccctggccgg caatcccgcc gccaccgccg ccgtgaaaac 1440
cgccctgagc ggaaatcccg tgcccattct gatcccctgc caccgggtgg tgtctagctc 1500
tggcgccgtg gggggctacg agggcgggct cgccgtgaaa gagtggctgc tggcccacga 1560
gggccacaga ctgggcaagc ctgggctggg tgatatcctc atggcgtcca ccacttccgc 1620
tgtgcctggg catccctctc tgcccagcct gcccagcaac agcagccagg agaggccact 1680
ggacacccgg gacccgctgc tagcccgggc ggagctggcg ctgctctcca tagtctttgt 1740
ggctgtggcc ctgagcaatg gcctggtgct ggcggcccta gctcggcggg gccggcgggg 1800
ccactgggca cccatacacg tcttcattgg ccacttgtgc ctggccgacc tggccgtggc 1860
tctgttccaa gtgctgcccc agctggcctg gaaggccacc gaccgcttcc gtgggccaga 1920
tgccctgtgt cgggccgtga agtatctgca gatggtgggc atgtatgcct cctcctacat 1980
gatcctggcc atgacgctgg accgccaccg tgccatctgc cgtcccatgc tggcgtaccg 2040
ccatggaagt ggggctcact ggaaccggcc ggtgctagtg gcttgggcct tctcgctcct 2100
tctcagcctg ccccagctct tcatcttcgc ccagcgcaac gtggaaggtg gcagcggggt 2160
cactgactgc tgggcctgct ttgcggagcc ctggggccgt cgcacctatg tcacctggat 2220
tgccctgatg gtgttcgtgg cacctaccct gggtatcgcc gcctgccagg tgctcatctt 2280
ccgggagatt catgccagtc tggtgccagg gccatcagag aggcctgggg ggcgccgcag 2340
gggacgccgg acaggcagcc ccggtgaggg agcccacgtg tcagcagctg tggccaagac 2400
tgtgaggatg acgctagtga ttgtggtcgt ctatgtgctg tgctgggcac ccttcttcct 2460
ggtgcagctg tgggccgcgt gggacccgga ggcacctctg gaaggggcgc cctttgtgct 2520
actcatgttg ctggccagcc tcaacagctg caccaacccc tggatctatg catctttcag 2580
cagcagcgtg tcctcagagc tgcgaagctt gctctgctgt gcccggggac gcaccccacc 2640

cagcctgggt ccccaagatg agtcctgcac caccgccagc tcctccctgg ccaaggacac 2700
ttcatcgtga ctcgagtcta gagggcccgt ttaaacccgc tgatcagcct cgactgtgcc 2760
ttctagttgc cagccatctg ttgtttgccc ctcccccgtg ccttccttga ccctggaagg 2820
tgccactccc actgtccttt cctaataaaa tgaggaaatt gcatcgcatt gtctgagtag 2880
gtgtcattct attctggggg gtggggtggg gcaggacagc aagggggagg attgggaaga 2940
caatagcagg catgctgggg atgcggtggg ctctatggct tctgaggcgg aaagaaccag 3000
ctggggctct aggggggtatc cccacgcgcc ctgtagcggc gcattaagcg cggcgggtgt 3060
ggtggttacg cgcagcgtga ccgctacact tgccagcgcc ctagcgcccg ctcctttcgc 3120
tttcttccct tcctttctcg ccacgttcgc cggctttccc cgtcaagctc taaatcgggg 3180
gctcccttta gggttccgat ttagtgcttt acggcacctc gaccccaaaa aacttgatta 3240
gggtgatggt tcacgtagtg ggccatcgcc ctgatagacg gtttttcgcc ctttgacgtt 3300
ggagtccacg ttctttaata gtggactctt gttccaaact ggaacaacac tcaaccctat 3360
ctcggtctat tcttttgatt tataagggat tttgccgatt tcggcctatt ggttaaaaaa 3420
tgagctgatt taacaaaaat ttaacgcgaa ttaattctgt ggaatgtgtg tcagttaggg 3480
tgtggaaagt ccccaggctc cccagcaggc agaagtatgc aaagcatgca tctcaattag 3540
tcagcaacca ggtgtggaaa gtccccaggc tccccagcag gcagaagtat gcaaagcatg 3600
catctcaatt agtcagcaac catagtcccg cccctaactc cgcccatccc gcccctaact 3660
ccgcccagtt ccgcccattc tccgccccat ggctgactaa ttttttttat ttatgcagag 3720
gccgaggccg cctctgcctc tgagctattc cagaagtagt gaggaggctt ttttggaggc 3780
ctaggctttt gcaaaaagct cccgggagct tgtatatcca ttttcggatc tgatcaagag 3840
acaggatgag gatcgtttcg catgattgaa caagatggat tgcacgcagg ttctccggcc 3900
gcttgggtgg agaggctatt cggctatgac tgggcacaac agacaatcgg ctgctctgat 3960
gccgccgtgt tccggctgtc agcgcagggg cgcccggttc tttttgtcaa gaccgacctg 4020
tccggtgccc tgaatgaact gcaggacgag gcagcgcggc tatcgtggct ggccacgacg 4080
ggcgttcctt gcgcagctgt gctcgacgtt gtcactgaag cgggaaggga ctggctgcta 4140
ttgggcgaag tgccggggca ggatctcctg tcatctcacc ttgctcctgc cgagaaagta 4200
tccatcatgg ctgatgcaat gcggcggctg catacgcttg atccggctac ctgcccattc 4260
gaccaccaag cgaaacatcg catcgagcga gcacgtactc ggatggaagc cggtcttgtc 4320
gatcaggatg atctggacga agagcatcag gggctcgcgc cagccgaact gttcgccagg 4380
ctcaaggcgc gcatgcccga cggcgaggat ctcgtcgtga cccatggcga tgcctgcttg 4440
ccgaatatca tggtggaaaa tggccgcttt tctggattca tcgactgtgg ccggctgggt 4500
gtggcggacc gctatcagga catagcgttg gctacccgtg atattgctga agagcttggc 4560

ggcgaatggg ctgaccgctt cctcgtgctt tacggtatcg ccgctcccga ttcgcagcgc 4620
atcgccttct atcgccttct tgacgagttc ttctgagcgg gactctgggg ttcgaaatga 4680
ccgaccaagc gacgcccaac ctgccatcac gagatttcga ttccaccgcc gccttctatg 4740
aaaggttggg cttcggaatc gttttccggg acgccggctg gatgatcctc cagcgcgggg 4800
atctcatgct ggagttcttc gcccacccca acttgtttat tgcagcttat aatggttaca 4860
aataaagcaa tagcatcaca aatttcacaa ataaagcatt tttttcactg cattctagtt 4920
gtggtttgtc caaactcatc aatgtatctt atcatgtctg tataccgtcg acctctagct 4980
agagcttggc gtaatcatgg tcatagctgt ttcctgtgtg aaattgttat ccgctcacaa 5040
ttccacacaa catacgagcc ggaagcataa agtgtaaagc ctggggtgcc taatgagtga 5100
gctaactcac attaattgcg ttgcgctcac tgcccgcttt ccagtcggga aacctgtcgt 5160
gccagctgca ttaatgaatc ggccaacgcg cggggagagg cggtttgcgt attgggcgct 5220
cttccgcttc ctcgctcact gactcgctgc gctcggtcgt tcggctgcgg cgagcggtat 5280
cagctcactc aaaggcggta atacggttat ccacagaatc aggggataac gcaggaaaga 5340
acatgtgagc aaaaggccag caaaaggcca ggaaccgtaa aaaggccgcg ttgctggcgt 5400
ttttccatag gctccgcccc cctgacgagc atcacaaaaa tcgacgctca agtcagaggt 5460
ggcgaaaccc gacaggacta taaagatacc aggcgtttcc ccctggaagc tccctcgtgc 5520
gctctcctgt tccgaccctg ccgcttaccg gatacctgtc cgcctttctc ccttcgggaa 5580
gcgtggcgct ttctcatagc tcacgctgta ggtatctcag ttcggtgtag gtcgttcgct 5640
ccaagctggg ctgtgtgcac gaaccccccg ttcagcccga ccgctgcgcc ttatccggta 5700
actatcgtct tgagtccaac ccggtaagac acgacttatc gccactggca gcagccactg 5760
gtaacaggat tagcagagcg aggtatgtag gcggtgctac agagttcttg aagtggtggc 5820
ctaactacgg ctacactaga agaacagtat ttggtatctg cgctctgctg aagccagtta 5880
ccttcggaaa aagagttggt agctcttgat ccggcaaaca aaccaccgct ggtagcggtt 5940
tttttgtttg caagcagcag attacgcgca gaaaaaaagg atctcaagaa gatcctttga 6000
tcttttctac ggggtctgac gctcagtgga acgaaaactc acgttaaggg attttggtca 6060
tgagattatc aaaaaggatc ttcacctaga tccttttaaa ttaaaaatga agttttaaat 6120
caatctaaag tatatatgag taaacttggt ctgacagtta ccaatgctta atcagtgagg 6180
cacctatctc agcgatctgt ctatttcgtt catccatagt tgcctgactc cccgtcgtgt 6240
agataactac gatacgggag ggcttaccat ctggccccag tgctgcaatg ataccgcgag 6300
acccacgctc accggctcca gatttatcag caataaacca gccagccgga agggccgagc 6360
gcagaagtgg tcctgcaact ttatccgcct ccatccagtc tattaattgt tgccgggaag 6420

```
ctagagtaag tagttcgcca gttaatagtt tgcgcaacgt tgttgccatt gctacaggca   6480
tcgtggtgtc acgctcgtcg tttggtatgg cttcattcag ctccggttcc caacgatcaa   6540
ggcgagttac atgatccccc atgttgtgca aaaaagcggt tagctccttc ggtcctccga   6600
tcgttgtcag aagtaagttg gccgcagtgt tatcactcat ggttatggca gcactgcata   6660
attctcttac tgtcatgcca tccgtaagat gcttttctgt gactggtgag tactcaacca   6720
agtcattctg agaatagtgt atgcggcgac cgagttgctc ttgcccggcg tcaatacggg   6780
ataataccgc gccacatagc agaactttaa aagtgctcat cattggaaaa cgttcttcgg   6840
ggcgaaaact ctcaaggatc ttaccgctgt tgagatccag ttcgatgtaa cccactcgtg   6900
cacccaactg atcttcagca tcttttactt tcaccagcgt ttctgggtga gcaaaaacag   6960
gaaggcaaaa tgccgcaaaa aagggaataa gggcgacacg gaaatgttga atactcatac   7020
tcttcctttt tcaatattat tgaagcattt atcagggtta ttgtctcatg agcggataca   7080
tatttgaatg tatttagaaa aataaacaaa taggggttcc gcgcacattt ccccgaaaag   7140
tgccacctga cgtc                                                      7154
```

<210> 5
<211> 7151
<212> DNA
<213> séquence artificielle

<220>
<221> misc_feature
<222> (232)..(819)
<223> promoteur CMV

<220>
<221> misc_feature
<222> (923)..(986)
<223> séquence leader T8

<220>
<221> misc_feature
<222> (1004)..(1027)
<223> flag tag

<220>
<221> misc_feature
<222> (1046)..(1591)
<223> Snap tag

<220>
<221> misc_feature
<222> (1598)..(2713)
<223> gène d’intérêt

<220>
<221> misc_feature
<222> (2751)..(2975)

<223> BGH polyA

<220>
<221> misc_feature
<222> (3859)..(4653)
<223> Neo R

<220>
<221> misc_feature
<222> (6155)..(7015)
<223> Ampi R

<400> 5

```
gacggatcgg gagatctccc gatcccctat ggtgcactct cagtacaatc tgctctgatg     60
ccgcatagtt aagccagtat ctgctccctg cttgtgtgtt ggaggtcgct gagtagtgcg    120
cgagcaaaat ttaagctaca acaaggcaag gcttgaccga caattgcatg aagaatctgc    180
ttagggttag gcgttttgcg ctgcttcgcg atgtacgggc cagatatacg cgttgacatt    240
gattattgac tagttattaa tagtaatcaa ttacggggtc attagttcat agcccatata    300
tggagttccg cgttacataa cttacggtaa atggcccgcc tggctgaccg cccaacgacc    360
cccgcccatt gacgtcaata atgacgtatg ttcccatagt aacgccaata gggactttcc    420
attgacgtca atgggtggag tatttacggt aaactgccca cttggcagta catcaagtgt    480
atcatatgcc aagtacgccc cctattgacg tcaatgacgg taaatggccc gcctggcatt    540
atgcccagta catgacctta tgggactttc ctacttggca gtacatctac gtattagtca    600
tcgctattac catggtgatg cggttttggc agtacatcaa tgggcgtgga tagcggtttg    660
actcacgggg atttccaagt ctccacccca ttgacgtcaa tgggagtttg ttttggcacc    720
aaaatcaacg ggactttcca aaatgtcgta acaactccgc cccattgacg caaatgggcg    780
gtaggcgtgt acggtgggag gtctatataa gcagagctct ctggctaact agagaaccca    840
ctgcttactg gcttatcgaa attaatacga ctcactatag ggagacccaa gctggctagc    900
gtttaaactt aagctttgag acatggcctt accagtgacc gccttgctcc tgccgctggc    960
cttgctgctc cacgccgcca ggccggccgc cgctagcggc atcgactaca aggacgacga   1020
tgacaaggcc ggcatcgatg ccatcatgga caaagactgc gaaatgaagc gcaccaccct   1080
ggatagccct ctgggcaagc tggaactgtc tgggtgcgaa cagggcctgc acgagatcaa   1140
gctgctgggc aaaggaacat ctgccgccga cgccgtggaa gtgcctgccc cagccgccgt   1200
gctgggcgga ccagagccac tgatgcaggc caccgcctgg ctcaacgcct actttcacca   1260
gcctgaggcc atcgaggagt tccctgtgcc agccctgcac cacccagtgt tccagcagga   1320
gagctttacc cgccaggtgc tgtggaaact gctgaaagtg gtgaagttcg gagaggtcat   1380
cagctaccag cagctggccg ccctggccgg caatcccgcc gccaccgccg ccgtgaaaac   1440
cgccctgagc ggaaatcccg tgcccattct gatcccctgc caccgggtgg tgtctagctc   1500
tggcgccgtg gggggctacg agggcgggct cgccgtgaaa gagtggctgc tggcccacga   1560
```

gggccacaga ctgggcaagc ctgggctggg tgatatcgaa ccggccccct ccgccggcgc 1620
cgagctgcag cccccgctct tcgccaacgc ctcggacgcc taccctagcg cctgccccag 1680
cgctggcgcc aatgcgtcgg ggccgccagg cgcgcggagc gcctcgtccc tcgccctggc 1740
aatcgccatc accgcgctct actcggccgt gtgcgccgtg gggctgctgg gcaacgtgct 1800
tgtcatgttc ggcatcgtcc ggtacactaa gatgaagacg gccaccaaca tctacatctt 1860
caacctggcc ttagccgatg cgctggccac cagcacgctg cctttccaga gtgccaagta 1920
cctgatggag acgtggccct tcggcgagct gctctgcaag gctgtgctct ccatcgacta 1980
ctacaatatg ttcaccagca tcttcacgct caccatgatg agtgttgacc gctacatcgc 2040
tgtctgccac cctgtcaagg ccctggactt ccgcacgcct gccaaggcca agctgatcaa 2100
catctgtatc tgggtcctgg cctcaggcgt tggcgtgccc atcatggtca tggctgtgac 2160
ccgtccccgg gacggggcag tggtgtgcat gctccagttc cccagcccca gctggtactg 2220
ggacacggtg accaagatct gcgtgttcct cttcgccttc gtggtgccca tcctcatcat 2280
caccgtgtgc tatggcctca tgctgctgcg cctgcgcagt gtgcgcctgc tgtcgggctc 2340
caaggagaag gaccgcagcc tgcggcgcat cacgcgcatg gtgctggtgg ttgtgggcgc 2400
cttcgtggtg tgttgggcgc ccatccacat cttcgtcatc gtctggacgc tggtggacat 2460
cgaccggcgc gacccgctgg tggtggctgc gctgcacctg tgcatcgcgc tgggctacgc 2520
caatagcagc ctcaaccccg tgctctacgc tttcctcgac gagaacttca agcgctgctt 2580
ccgccagctc tgccgcaagc cctgcggccg cccagacccc agcagcttca gccgcgcccg 2640
cgaagccacg gcccgcgagc gtgtcaccgc ctgcaccccg tccgatggtc ccggcggtgg 2700
cgctgccgcc tgatctagag ggcccgttta aacccgctga tcagcctcga ctgtgccttc 2760
tagttgccag ccatctgttg tttgcccctc ccccgtgcct tccttgaccc tggaaggtgc 2820
cactcccact gtcctttcct aataaaatga ggaaattgca tcgcattgtc tgagtaggtg 2880
tcattctatt ctggggggtg gggtggggca ggacagcaag ggggaggatt gggaagacaa 2940
tagcaggcat gctggggatg cggtgggctc tatggcttct gaggcggaaa gaaccagctg 3000
gggctctagg gggtatcccc acgcgccctg tagcggcgca ttaagcgcgg cgggtgtggt 3060
ggttacgcgc agcgtgaccg ctacacttgc cagcgcccta gcgcccgctc ctttcgcttt 3120
cttcccttcc tttctcgcca cgttcgccgg ctttccccgt caagctctaa atcgggggct 3180
ccctttaggg ttccgattta gtgctttacg gcacctcgac cccaaaaaac ttgattaggg 3240
tgatggttca cgtagtgggc catcgccctg atagacggtt tttcgccctt tgacgttgga 3300
gtccacgttc tttaatagtg gactcttgtt ccaaactgga acaacactca accctatctc 3360
ggtctattct tttgatttat aagggatttt gccgatttcg gcctattggt taaaaaatga 3420
gctgatttaa caaaaattta acgcgaatta attctgtgga atgtgtgtca gttagggtgt 3480

ggaaagtccc caggctcccc agcaggcaga agtatgcaaa gcatgcatct caattagtca 3540
gcaaccaggt gtggaaagtc cccaggctcc ccagcaggca gaagtatgca aagcatgcat 3600
ctcaattagt cagcaaccat agtcccgccc ctaactccgc ccatcccgcc cctaactccg 3660
cccagttccg cccattctcc gccccatggc tgactaattt tttttattta tgcagaggcc 3720
gaggccgcct ctgcctctga gctattccag aagtagtgag gaggcttttt tggaggccta 3780
ggcttttgca aaaagctccc gggagcttgt atatccattt tcggatctga tcaagagaca 3840
ggatgaggat cgtttcgcat gattgaacaa gatggattgc acgcaggttc tccggccgct 3900
tgggtggaga ggctattcgg ctatgactgg gcacaacaga caatcggctg ctctgatgcc 3960
gccgtgttcc ggctgtcagc gcaggggcgc ccggttcttt ttgtcaagac cgacctgtcc 4020
ggtgccctga atgaactgca ggacgaggca gcgcggctat cgtggctggc cacgacgggc 4080
gttccttgcg cagctgtgct cgacgttgtc actgaagcgg gaagggactg gctgctattg 4140
ggcgaagtgc cggggcagga tctcctgtca tctcaccttg ctcctgccga gaaagtatcc 4200
atcatggctg atgcaatgcg gcggctgcat acgcttgatc cggctacctg cccattcgac 4260
caccaagcga aacatcgcat cgagcgagca cgtactcgga tggaagccgg tcttgtcgat 4320
caggatgatc tggacgaaga gcatcagggg ctcgcgccag ccgaactgtt cgccaggctc 4380
aaggcgcgca tgcccgacgg cgaggatctc gtcgtgaccc atggcgatgc ctgcttgccg 4440
aatatcatgg tggaaaatgg ccgcttttct ggattcatcg actgtggccg gctgggtgtg 4500
gcggaccgct atcaggacat agcgttggct acccgtgata ttgctgaaga gcttggcggc 4560
gaatgggctg accgcttcct cgtgctttac ggtatcgccg ctcccgattc gcagcgcatc 4620
gccttctatc gccttcttga cgagttcttc tgagcgggac tctggggttc gaaatgaccg 4680
accaagcgac gcccaacctg ccatcacgag atttcgattc caccgccgcc ttctatgaaa 4740
ggttgggctt cggaatcgtt ttccgggacg ccggctggat gatcctccag cgcggggatc 4800
tcatgctgga gttcttcgcc caccccaact tgtttattgc agcttataat ggttacaaat 4860
aaagcaatag catcacaaat ttcacaaata aagcattttt ttcactgcat tctagttgtg 4920
gtttgtccaa actcatcaat gtatcttatc atgtctgtat accgtcgacc tctagctaga 4980
gcttggcgta atcatggtca tagctgtttc ctgtgtgaaa ttgttatccg ctcacaattc 5040
cacacaacat acgagccgga agcataaagt gtaaagcctg gggtgcctaa tgagtgagct 5100
aactcacatt aattgcgttg cgctcactgc ccgctttcca gtcgggaaac ctgtcgtgcc 5160
agctgcatta atgaatcggc caacgcgcgg ggagaggcgg tttgcgtatt gggcgctctt 5220
ccgcttcctc gctcactgac tcgctgcgct cggtcgttcg gctgcggcga gcggtatcag 5280
ctcactcaaa ggcggtaata cggttatcca cagaatcagg ggataacgca ggaaagaaca 5340

```
tgtgagcaaa aggccagcaa aaggccagga accgtaaaaa ggccgcgttg ctggcgtttt   5400
tccataggct ccgcccccct gacgagcatc acaaaaatcg acgctcaagt cagaggtggc   5460
gaaacccgac aggactataa agataccagg cgtttccccc tggaagctcc ctcgtgcgct   5520
ctcctgttcc gaccctgccg cttaccggat acctgtccgc ctttctccct tcgggaagcg   5580
tggcgctttc tcatagctca cgctgtaggt atctcagttc ggtgtaggtc gttcgctcca   5640
agctgggctg tgtgcacgaa ccccccgttc agcccgaccg ctgcgcctta tccggtaact   5700
atcgtcttga gtccaacccg gtaagacacg acttatcgcc actggcagca gccactggta   5760
acaggattag cagagcgagg tatgtaggcg gtgctacaga gttcttgaag tggtggccta   5820
actacggcta cactagaaga acagtatttg gtatctgcgc tctgctgaag ccagttacct   5880
tcggaaaaag agttggtagc tcttgatccg gcaaacaaac caccgctggt agcggttttt   5940
ttgtttgcaa gcagcagatt acgcgcagaa aaaaaggatc tcaagaagat cctttgatct   6000
tttctacggg gtctgacgct cagtggaacg aaaactcacg ttaagggatt ttggtcatga   6060
gattatcaaa aaggatcttc acctagatcc ttttaaatta aaaatgaagt tttaaatcaa   6120
tctaaagtat atatgagtaa acttggtctg acagttacca atgcttaatc agtgaggcac   6180
ctatctcagc gatctgtcta tttcgttcat ccatagttgc ctgactcccc gtcgtgtaga   6240
taactacgat acgggagggc ttaccatctg gccccagtgc tgcaatgata ccgcgagacc   6300
cacgctcacc ggctccagat ttatcagcaa taaaccagcc agccggaagg gccgagcgca   6360
gaagtggtcc tgcaacttta tccgcctcca tccagtctat taattgttgc cgggaagcta   6420
gagtaagtag ttcgccagtt aatagtttgc gcaacgttgt tgccattgct acaggcatcg   6480
tggtgtcacg ctcgtcgttt ggtatggctt cattcagctc cggttcccaa cgatcaaggc   6540
gagttacatg atcccccatg ttgtgcaaaa aagcggttag ctccttcggt cctccgatcg   6600
ttgtcagaag taagttggcc gcagtgttat cactcatggt tatggcagca ctgcataatt   6660
ctcttactgt catgccatcc gtaagatgct tttctgtgac tggtgagtac tcaaccaagt   6720
cattctgaga atagtgtatg cggcgaccga gttgctcttg cccggcgtca atacgggata   6780
ataccgcgcc acatagcaga actttaaaag tgctcatcat tggaaaacgt tcttcggggc   6840
gaaaactctc aaggatctta ccgctgttga gatccagttc gatgtaaccc actcgtgcac   6900
ccaactgatc ttcagcatct tttactttca ccagcgtttc tgggtgagca aaaacaggaa   6960
ggcaaaatgc cgcaaaaaag ggaataaggg cgacacggaa atgttgaata ctcatactct   7020
tcctttttca atattattga agcatttatc agggttattg tctcatgagc ggatacatat   7080
ttgaatgtat ttagaaaaat aaacaaatag gggttccgcg cacatttccc cgaaaagtgc   7140
cacctgacgt c                                                         7151
```

<210> 6
<211> 7619
<212> DNA
<213> séquence artificielle

<220>
<221> misc_feature
<222> (232)..(819)
<223> promoteur CMV

<220>
<221> misc_feature
<222> (923)..(986)
<223> séquence leader T8

<220>
<221> misc_feature
<222> (1004)..(1027)
<223> flag tag

<220>
<221> misc_feature
<222> (1040)..(1927)
<223> halo tag

<220>
<221> misc_feature
<222> (1934)..(3181)
<223> gène d'intérêt

<220>
<221> misc_feature
<222> (3219)..(3443)
<223> BGH polyA

<220>
<221> misc_feature
<222> (4327)..(5121)
<223> Neo R

<220>
<221> misc_feature
<222> (6623)..(7483)
<223> Ampi R

<400> 6

```
gacggatcgg gagatctccc gatcccctat ggtgcactct cagtacaatc tgctctgatg      60
ccgcatagtt aagccagtat ctgctccctg cttgtgtgtt ggaggtcgct gagtagtgcg     120
cgagcaaaat ttaagctaca acaaggcaag gcttgaccga caattgcatg aagaatctgc     180
ttagggttag gcgttttgcg ctgcttcgcg atgtacgggc cagatatacg cgttgacatt     240
gattattgac tagttattaa tagtaatcaa ttacggggtc attagttcat agcccatata     300
tggagttccg cgttacataa cttacggtaa atggcccgcc tggctgaccg cccaacgacc     360
cccgcccatt gacgtcaata atgacgtatg ttcccatagt aacgccaata gggactttcc     420
attgacgtca atgggtggag tatttacggt aaactgccca cttggcagta catcaagtgt     480
atcatatgcc aagtacgccc cctattgacg tcaatgacgg taaatggccc gcctggcatt     540
```

atgcccagta catgacctta tgggactttc ctacttggca gtacatctac gtattagtca 600
tcgctattac catggtgatg cggttttggc agtacatcaa tgggcgtgga tagcggtttg 660
actcacgggg atttccaagt ctccacccca ttgacgtcaa tgggagtttg ttttggcacc 720
aaaatcaacg ggactttcca aaatgtcgta acaactccgc cccattgacg caaatgggcg 780
gtaggcgtgt acggtgggag gtctatataa gcagagctct ctggctaact agagaaccca 840
ctgcttactg gcttatcgaa attaatacga ctcactatag ggagacccaa gctggctagc 900
gtttaaactt aagctttgag acatggcctt accagtgacc gccttgctcc tgccgctggc 960
cttgctgctc cacgccgcca ggccggccgc cgctagcggc atcgactaca aggacgacga 1020
tgacaaggcc ggcatcgatg cagaaatcgg tactggcttt ccattcgacc cccattatgt 1080
ggaagtcctg ggcgagcgca tgcactacgt cgatgttggt ccgcgcgatg gcacccctgt 1140
gctgttcctg cacggtaacc cgacctcctc ctacgtgtgg cgcaacatca tcccgcatgt 1200
tgcaccgacc catcgctgca ttgctccaga cctgatcggt atgggcaaat ccgacaaacc 1260
agacctgggt tatttcttcg acgaccacgt ccgcttcatg gatgccttca tcgaagccct 1320
gggtctggaa gaggtcgtcc tggtcattca cgactggggc tccgctctgg gtttccactg 1380
ggccaagcgc aatccagagc gcgtcaaagg tattgcattt atggagttca tccgccctat 1440
cccgacctgg gacgaatggc cagaatttgc ccgcgagacc ttccaggcct tccgcaccac 1500
cgacgtcggc cgcaagctga tcatcgatca gaacgttttt atcgagggta cgctgccgat 1560
gggtgtcgtc cgcccgctga ctgaagtcga gatggaccat taccgcgagc cgttcctgaa 1620
tcctgttgac cgcgagccac tgtggcgctt cccaaacgag ctgccaatcg ccggtgagcc 1680
agcgaacatc gtcgcgctgg tcgaagaata catggactgg ctgcaccagt cccctgtccc 1740
gaagctgctg ttctggggca ccccaggcgt tctgatccca ccggccgaag ccgctcgcct 1800
ggccaaaagc ctgcctaact gcaaggctgt ggacatcggc ccgggtctga atctgctgca 1860
agaagacaac ccggacctga tcggcagcga gatcgcgcgc tggctgtcga cgctcgagat 1920
ttccggcgat atcgatccac tgaatctgtc ctggtatgat gatgatctgg agaggcagaa 1980
ctggagccgg cccttcaacg ggtcagacgg gaaggcggac agaccccact acaactacta 2040
tgccacactg ctcaccctgc tcatcgctgt catcgtcttc ggcaacgtgc tggtgtgcat 2100
ggctgtgtcc cgcgagaagg cgctgcagac caccaccaac tacctgatcg tcagcctcgc 2160
agtggccgac ctcctcgtcg ccacactggt catgccctgg gttgtctacc tggaggtggt 2220
aggtgagtgg aaattcagca ggattcactg tgacatcttc gtcactctgg acgtcatgat 2280
gtgcacggcg agcatcctga acttgtgtgc catcagcatc gacaggtaca cagctgtggc 2340
catgcccatg ctgtacaata cgcgctacag ctccaagcgc cgggtcaccg tcatgatctc 2400

catcgtctgg gtcctgtcct tcaccatctc ctgcccactc ctcttcggac tcaataacgc 2460
agaccagaac gagtgcatca ttgccaaccc ggccttcgtg gtctactcct ccatcgtctc 2520
cttctacgtg cccttcattg tcaccctgct ggtctacatc aagatctaca ttgtcctccg 2580
cagacgccgc aagcgagtca acaccaaacg cagcagccga gctttcaggg cccacctgag 2640
ggctccacta aaggaggctg cccggcgagc ccaggagctg gagatggaga tgctctccag 2700
caccagccca cccgagagga cccggtacag ccccatccca cccagccacc accagctgac 2760
tctccccgac ccgtcccacc atggtctcca cagcactccc gacagccccg ccaaaccaga 2820
gaagaatggg catgccaaag accaccccaa gattgccaag atctttgaga tccagaccat 2880
gcccaatggc aaaacgcgta cctccctcaa gaccatgagc cgtaggaagc tctcccagca 2940
gaaggagaag aaagccactc agatgctcgc cattgttctc ggcgtgttca tcatctgctg 3000
gctgcccttc ttcatcacac acatcctgaa catacactgt gactgcaaca tcccgcctgt 3060
cctgtacagc gccttcacgt ggctgggcta tgtcaacagc gccgtgaacc ccatcatcta 3120
caccaccttc aacattgagt tccgcaaggc cttcctgaag atcctccact gctgactcga 3180
gtctagaggg cccgtttaaa cccgctgatc agcctcgact gtgccttcta gttgccagcc 3240
atctgttgtt tgcccctccc ccgtgccttc cttgaccctg gaaggtgcca ctcccactgt 3300
cctttcctaa taaaatgagg aaattgcatc gcattgtctg agtaggtgtc attctattct 3360
ggggggtggg gtggggcagg acagcaaggg ggaggattgg gaagacaata gcaggcatgc 3420
tggggatgcg gtgggctcta tggcttctga ggcggaaaga accagctggg gctctagggg 3480
gtatccccac gcgccctgta gcggcgcatt aagcgcggcg ggtgtggtgg ttacgcgcag 3540
cgtgaccgct acacttgcca gcgccctagc gcccgctcct ttcgctttct tcccttcctt 3600
tctcgccacg ttcgccggct ttccccgtca agctctaaat cgggggctcc ctttagggtt 3660
ccgatttagt gctttacggc acctcgaccc caaaaaactt gattagggtg atggttcacg 3720
tagtgggcca tcgccctgat agacggtttt tcgccctttg acgttggagt ccacgttctt 3780
taatagtgga ctcttgttcc aaactggaac aacactcaac cctatctcgg tctattcttt 3840
tgatttataa gggattttgc cgatttcggc ctattggtta aaaaatgagc tgatttaaca 3900
aaaatttaac gcgaattaat tctgtggaat gtgtgtcagt tagggtgtgg aaagtcccca 3960
ggctccccag caggcagaag tatgcaaagc atgcatctca attagtcagc aaccaggtgt 4020
ggaaagtccc caggctcccc agcaggcaga agtatgcaaa gcatgcatct caattagtca 4080
gcaaccatag tcccgcccct aactccgccc atcccgcccc taactccgcc cagttccgcc 4140
cattctccgc cccatggctg actaattttt tttatttatg cagaggccga ggccgcctct 4200
gcctctgagc tattccagaa gtagtgagga ggcttttttg gaggcctagg cttttgcaaa 4260
aagctcccgg gagcttgtat atccattttc ggatctgatc aagagacagg atgaggatcg 4320

tttcgcatga ttgaacaaga tggattgcac gcaggttctc cggccgcttg ggtggagagg 4380
ctattcggct atgactgggc acaacagaca atcggctgct ctgatgccgc cgtgttccgg 4440
ctgtcagcgc aggggcgccc ggttcttttt gtcaagaccg acctgtccgg tgccctgaat 4500
gaactgcagg acgaggcagc gcggctatcg tggctggcca cgacgggcgt tccttgcgca 4560
gctgtgctcg acgttgtcac tgaagcggga agggactggc tgctattggg cgaagtgccg 4620
gggcaggatc tcctgtcatc tcaccttgct cctgccgaga aagtatccat catggctgat 4680
gcaatgcggc ggctgcatac gcttgatccg gctacctgcc cattcgacca ccaagcgaaa 4740
catcgcatcg agcgagcacg tactcggatg gaagccggtc ttgtcgatca ggatgatctg 4800
gacgaagagc atcaggggct cgcgccagcc gaactgttcg ccaggctcaa ggcgcgcatg 4860
cccgacggcg aggatctcgt cgtgacccat ggcgatgcct gcttgccgaa tatcatggtg 4920
gaaaatggcc gcttttctgg attcatcgac tgtggccggc tgggtgtggc ggaccgctat 4980
caggacatag cgttggctac ccgtgatatt gctgaagagc ttggcggcga atgggctgac 5040
cgcttcctcg tgctttacgg tatcgccgct cccgattcgc agcgcatcgc cttctatcgc 5100
cttcttgacg agttcttctg agcgggactc tggggttcga aatgaccgac caagcgacgc 5160
ccaacctgcc atcacgagat ttcgattcca ccgccgcctt ctatgaaagg ttgggcttcg 5220
gaatcgtttt ccgggacgcc ggctggatga tcctccagcg cggggatctc atgctggagt 5280
tcttcgccca ccccaacttg tttattgcag cttataatgg ttacaaataa agcaatagca 5340
tcacaaattt cacaaataaa gcattttttt cactgcattc tagttgtggt ttgtccaaac 5400
tcatcaatgt atcttatcat gtctgtatac cgtcgacctc tagctagagc ttggcgtaat 5460
catggtcata gctgtttcct gtgtgaaatt gttatccgct cacaattcca cacaacatac 5520
gagccggaag cataaagtgt aaagcctggg gtgcctaatg agtgagctaa ctcacattaa 5580
ttgcgttgcg ctcactgccc gctttccagt cgggaaacct gtcgtgccag ctgcattaat 5640
gaatcggcca acgcgcgggg agaggcggtt tgcgtattgg gcgctcttcc gcttcctcgc 5700
tcactgactc gctgcgctcg gtcgttcggc tgcggcgagc ggtatcagct cactcaaagg 5760
cggtaatacg gttatccaca gaatcagggg ataacgcagg aaagaacatg tgagcaaaag 5820
gccagcaaaa ggccaggaac cgtaaaaagg ccgcgttgct ggcgtttttc cataggctcc 5880
gcccccctga cgagcatcac aaaaatcgac gctcaagtca gaggtggcga aacccgacag 5940
gactataaag ataccaggcg tttccccctg gaagctccct cgtgcgctct cctgttccga 6000
ccctgccgct taccggatac ctgtccgcct ttctcccttc gggaagcgtg gcgctttctc 6060
atagctcacg ctgtaggtat ctcagttcgg tgtaggtcgt tcgctccaag ctgggctgtg 6120
tgcacgaacc ccccgttcag cccgaccgct gcgccttatc cggtaactat cgtcttgagt 6180

```
ccaacccggt aagacacgac ttatcgccac tggcagcagc cactggtaac aggattagca   6240
gagcgaggta tgtaggcggt gctacagagt tcttgaagtg gtggcctaac tacggctaca   6300
ctagaagaac agtatttggt atctgcgctc tgctgaagcc agttaccttc ggaaaaagag   6360
ttggtagctc ttgatccggc aaacaaacca ccgctggtag cggttttttt gtttgcaagc   6420
agcagattac gcgcagaaaa aaaggatctc aagaagatcc tttgatcttt tctacggggt   6480
ctgacgctca gtggaacgaa aactcacgtt aagggatttt ggtcatgaga ttatcaaaaa   6540
ggatcttcac ctagatcctt ttaaattaaa aatgaagttt taaatcaatc taaagtatat   6600
atgagtaaac ttggtctgac agttaccaat gcttaatcag tgaggcacct atctcagcga   6660
tctgtctatt tcgttcatcc atagttgcct gactccccgt cgtgtagata actacgatac   6720
gggagggctt accatctggc cccagtgctg caatgatacc gcgagaccca cgctcaccgg   6780
ctccagattt atcagcaata aaccagccag ccggaagggc cgagcgcaga agtggtcctg   6840
caactttatc cgcctccatc cagtctatta attgttgccg ggaagctaga gtaagtagtt   6900
cgccagttaa tagtttgcgc aacgttgttg ccattgctac aggcatcgtg gtgtcacgct   6960
cgtcgtttgg tatggcttca ttcagctccg gttcccaacg atcaaggcga gttacatgat   7020
cccccatgtt gtgcaaaaaa gcggttagct ccttcggtcc tccgatcgtt gtcagaagta   7080
agttggccgc agtgttatca ctcatggtta tggcagcact gcataattct cttactgtca   7140
tgccatccgt aagatgcttt tctgtgactg gtgagtactc aaccaagtca ttctgagaat   7200
agtgtatgcg gcgaccgagt tgctcttgcc cggcgtcaat acgggataat accgcgccac   7260
atagcagaac tttaaaagtg ctcatcattg gaaaacgttc ttcggggcga aaactctcaa   7320
ggatcttacc gctgttgaga tccagttcga tgtaacccac tcgtgcaccc aactgatctt   7380
cagcatcttt tactttcacc agcgtttctg ggtgagcaaa aacaggaagg caaaatgccg   7440
caaaaaaggg aataagggcg acacggaaat gttgaatact catactcttc ctttttcaat   7500
attattgaag catttatcag ggttattgtc tcatgagcgg atacatattt gaatgtattt   7560
agaaaaataa acaaataggg gttccgcgca catttccccg aaaagtgcca cctgacgtc    7619
```